# EUROPEAN PATENT SPECIFICATION

(11) **EP 4 735 036 B1**
(45) Date of publication and mention of the grant of the patent: **22.07.2026**
(21) Application number: 24837391.2
(22) Date of filing: 20.12.2024
(51) Int. Cl.: A61K 39/35, A61K 38/16, A61P 1/00, A61P 37/04, A61P 37/08

(54) **IMMUNOTHERAPEUTIC METHODS, COMPOUNDS, AND COMPOSITIONS FOR TREATING OR PREVENTING CELIAC DISEASE**
IMMUNTHERAPEUTISCHE VERFAHREN, VERBINDUNGEN UND ZUSAMMENSETZUNGEN ZUR BEHANDLUNG ODER PRÄVENTION VON ZÖLIAKIE
MÉTHODES, COMPOSÉS ET COMPOSITIONS IMMUNOTHÉRAPEUTIQUES POUR LE TRAITEMENT OU LA PRÉVENTION D'UNE MALADIE COELIAQUE

(30) Priority: 21.12.2023 EP 23307336; 24.09.2024 EP 24306565
(43) Date of publication of application: 06.05.2026
(60) Divisional application: 26186205.6
(73) Proprietor: DBV Technologies, 92320 Chatillon (FR)
(72) Inventor: DIOSZEGHY, Vincent, 92320 Chatillon (FR); MATTHEWS, Katie, Warren, New Jersey 07059 (US); HERVE, Pierre-Louis, 92320 Chatillon (FR); ROUCAIROL, Camille, 92320 Chatillon (FR); KOPPELMAN, Stef, Warren, New Jersey 07059 (US); ANDERSON, Robert Paul, Ashgrove, Queensland 4060 (AU); KONING, Frits, 2333 Leiden (NL); MAILLERE, Bernard, 78000 Versailles (FR)
(74) Representative: Cabinet Becker et Associés
(86) International application number: PCT/EP2024/088217
(87) International publication number: WO 2025/133325

(56) References cited:
- WO-A1-2019/157282
- WO-A1-2023/066846
- WO-A2-2005/105129
- SOLLID LUDVIG M ET AL: "Update 2020: nomenclature and listing of celiac disease-relevant gluten epitopes recognized by CD4T cells", IMMUNOGENETICS, SPRINGER VERLAG, BERLIN, DE, vol. 72, no. 1-2, 18 November 2019 (2019-11-18), pages 85 - 88, XP036992946, ISSN: 0093-7711, [retrieved on 20191118], DOI: 10.1007/S00251-019-01141-W
- R. CICCOCIOPPO ET AL: "The immune recognition of gluten in coeliac disease", CLINICAL AND EXPERIMENTAL IMMUNOLOGY, vol. 140, no. 3, 1 June 2005 (2005-06-01), GB, pages 408 - 416, XP055235019, ISSN: 0009-9104, DOI: 10.1111/j.1365-2249.2005.02783.x

## Description

### FIELD

The present application relates to an immunotherapeutic method for treating or preventing celiac disease.

### BACKGROUND

Celiac disease (CeD) is an immune-mediated disease caused by an abnormal intestinal T cell response to cereal gluten proteins. CeD is triggered by the intake of gluten proteins derived from wheat, barley, and rye (1). Gluten is a complex mix of proteins that can be divided into two fractions: gliadins and glutenins. Gliadins are the main trigger factors of CeD and can be subdivided into three main isoforms: α-gliadins (also referred to as alpha-gliadins), γ-gliadins (also referred to as gamma-gliadins), and ω-gliadins (also referred to as omega-gliadins).

CeD has a strong genetic component; human leukocyte antigen (HLA) is the most important genetic determinant. More specifically, a strong association between CeD development and expression of certain human leukocyte antigen (HLA)-DQ allotype variants (HLA-DQ2 and HLA-DQ8) has been reported. The prevalence of CeD in the general population is estimated to be 1%, with a seroprevalence of 1.4% and a biopsy-proven prevalence of 0.7% (2). CeD prevalence is increasing in Western countries. Between the years 1975 and 2000, CeD prevalence increased 5-fold in the US, for reasons that are currently unknown. The prevalence of CeD is higher in first-degree CeD relatives (10-15%) and in other at-risk groups, particularly patients with Down syndrome, type 1 diabetes, or IgA deficiency.

Symptoms of CeD can appear at any age from infancy well into senior adulthood. Symptoms usually occur in children after ingestion of gluten-containing grains between 4 and 24 months of age. There may be a delay or latent period between gluten intake and the onset of symptoms. Gastrointestinal system and extra-intestinal manifestations are common in CeD (3). The main symptoms of CeD are chronic diarrhea, recurrent abdominal pain, nausea, vomiting, and abdominal distension. Common extra-intestinal manifestations are failure to thrive, short stature, chronic anemia, osteopenia, osteoporosis, delayed puberty, dental enamel defect, irritability, chronic fatigue, neuropathy, arthritis, arthralgia, amenorrhea, and increased liver enzymes.

CeD affects the proximal small intestine, leading to villus atrophy and crypt hyperplasia, together with an increased number of intraepithelial lymphocytes (IELs) and lamina propria lymphocytes in the mucosa. The histological changes are caused by an immune response to dietary gluten in the small intestine mucosa (4). Since gluten is highly resistant to proteolysis by gastrointestinal enzymes, long gluten peptides remain in the lumen of the intestine. After crossing the epithelial layer, some of these peptides are partially and/or fully deamidated by the enzymatic activity of tissue transglutaminase (tTG) which convert some glutamine residues (Q) to glutamates/glutamic acid (E) in the motifs Q-X-proline (P) where X is not a glycine (G) or a P, and in the motif Q-X-X where X is, for instance, a phenylalanine (F), a tyrosine (Y), a tryptophan (W), an isoleucine (I) or a leucine (L). Deamidation generates negatively charged immunogenic peptides with increased affinity for the major histocompatibility complex (MHC)-II molecules HLA-DQ2 and HLA-DQ8. This leads to the presentation of immunogenic peptides by dendritic cells (DCs). Based on studies conducted in mouse models, it is believed that DCs, having captured gliadin fragments, migrate from the lamina propria to the draining mesenteric lymph nodes where they encounter naïve CD4⁺ T cells and induce their differentiation into gluten-specific Th1 CD4⁺ T cells, which migrate back to the lamina propria. The contact of CD4⁺ T cells in the lamina propria with gluten fragments induces their activation and proliferation, resulting in the production of proinflammatory cytokines (especially IFN-γ), metalloproteases, and keratinocyte growth factor by stromal cells. This in turn induces cryptal hyperplasia and villous blunting secondary to intestinal epithelial cell death induced by IELs (5). Th1 cells can also locally stimulate CD8⁺ T cells and natural killer (NK) cells, which causes apoptosis of the enterocytes. In addition to the CD4⁺ T cell response, an increased number of gluten-specific CD8⁺ cytotoxic T lymphocytes (CTLs) has been reported in the lamina propria of CeD patients. These CTLs express IFN-γ, CD95, and granzyme B when stimulated with gliadin (6).

Several HLA-DQ-restricted gluten T cell epitopes recognized by CD4⁺ T cells of CeD patients have been described to date (7-9). More specifically, various T cell epitopes from wheat gluten proteins (mainly from the alpha-, gamma-, and omega-isoforms of gliadins) have been reported. T cell epitopes from hordein (barley) and secalin (rye) have also been described and showed a high homology with those found in wheat.
At present, the only available treatment for CeD is a strict, lifelong adherence to a gluten-free diet (GFD), which often presents a challenging task. GFD alone is frequently insufficient to control symptoms and prevent mucosal damage that can result from gluten exposure. Moreover, GFD is difficult, socially isolating, extremely expensive, and can be nutritionally inferior to non-restrictive diet (deficient in some vitamins and micronutrients, with higher fat content). Therefore, most patients with CeD desire non-dietary therapies for the management of their condition (10).

Loss of tolerance to gluten epitopes is believed to be the trigger of CeD pathogenesis. Therefore, restoring specific immune tolerance to gluten is believed to be an approach to treat the root of CeD and to allow CeD patients to consume dietary gluten without negative effects. In that context, two immunotherapeutic strategies have been developed and tested so far. The first vaccine (Nexvax2) was developed by ImmusanT (Cambridge, MA, USA). This vaccine comprised three HLA-DQ2.5 restricted small soluble immunodominant peptides, one each from α-gliadin, ω-gliadin, and B-hordein. This vaccine failed in phase II trial (RESET) in 2019, following an interim analysis showing no statistically significant protection to gluten exposure compared to placebo (11,12).

A second vaccine (TAK-101, formerly known as CNP-101 or TIMP-GLIA) was developed by COUR Pharmaceuticals before being out-licensed to Takeda in 2019. This vaccine consists of acetic acid-treated gliadin isolated from wheat, encapsulated in PLGA nanoparticles for intravenous delivery. A Phase 2 clinical study aiming at assessing the efficacy and safety of the TAK-101 for prevention of gluten-specific T cell activation in participants with CeD on a gluten-free diet (NCT04530123) is ongoing.

International application WO 2005/105129 describes epitopes derived from gluten that may be used in methods of diagnosing, treating and preventing celiac disease.

Therefore, there is a need for methods for treating or preventing CeD, in particular for increasing tolerance towards wheat, barley, and/or rye gliadins in subjects suffering from CeD or in those individuals susceptible to suffering from CeD.

### SUMMARY

The invention is as defined in the appended set of claims.

The application provides an immunogenic polypeptide construct which is a non-naturally occurring molecule comprising at least two epitopes selected from (i) at least one epitope from alpha-gliadin, (ii) at least one epitope from gamma-gliadin, and (iii) at least one epitope from omega-gliadin, each epitope being independently in non-deamidated form or in at least partially deamidated form. The immunogenic polypeptide construct provided herein may be used to increase tolerance to gliadin in a subject sensitive to gliadin, e.g., in a subject suffering from, or at risk of developing, celiac disease.

The immunogenic polypeptide construct of the invention is of formula (Ic):

[BB1]-[CCw]-[BB2a]-[CCx]-[BB3a]-[CCy]-[BB4a] (Ic)

wherein:
- BB1 is a polypeptide of SEQ ID NO:15, BB2a is a polypeptide of SEQ ID NO:16, BB3a is a polypeptide of SEQ ID NO:20, and BB4a is a polypeptide of SEQ ID NO:18, wherein all Z residues are E residues in SEQ ID NOS: 15, 16, 20, and 18, and
- each CCw, CCx, and CCy are independently selected from the group consisting of KKKK (SEQ ID NO:32), KK, GPRRKK (SEQ ID NO:33), GPKK (SEQ ID NO:34), GARRKK (SEQ ID NO:35), AYY, RR, and GCRRKK (SEQ ID NO:36)

It is disclosed an immunogenic polypeptide construct which is a non-naturally occurring molecule which comprises (i) at least one epitope from alpha-gliadin, (ii) at least one epitope from gamma-gliadin, and (iii) at least one epitope from omega-gliadin, each epitope being independently in non-deamidated form or in at least partially deamidated form wherein:
- the at least one epitope from alpha-gliadin is selected from the group consisting of: SEQ ID NO:1; SEQ ID NO:2; SEQ ID NO:3; SEQ ID NO:4, and combinations thereof,
- the at least one epitope from gamma-gliadin is selected from the group consisting of: SEQ ID NO:5; SEQ ID NO:6; SEQ ID NO:7, and combinations thereof, and
- the at least one epitope from omega-gliadin is selected from the group consisting of SEQ ID NO:8; SEQ ID NO:9, and combinations thereof,
- the immunogenic polypeptide construct is for use in increasing tolerance to gliadin in a subject sensitive to gliadin by repeated administrations, preferably by epicutaneous route.

Preferably, the immunogenic polypeptide construct as described herein is used for treating or preventing celiac disease.

In some aspects , the immunogenic polypeptide construct comprises:
- a polypeptide of SEQ ID NO:14,
- at least one epitope from alpha-gliadin specific for the HLA-DQ8, wherein the at least one epitope from alpha-gliadin consists of a peptide of SEQ ID NO:4,
- at least one epitope from gamma-gliadin specific for the HLA-DQ8, wherein the at least one epitope from gamma-gliadin consists of a peptide of SEQ ID NO:6, and
- at least one epitope from omega-gliadin specific for the HLA-DQ2, wherein the at least one epitope from omega-gliadin consists of SEQ ID NO:28.

The immunogenic polypeptide construct may further comprise at least one QPEQPFP moiety.

In another aspect, the immunogenic polypeptide construct for use as described herein comprises:
(i) at least one building block comprising an epitope from alpha-gliadin which is selected from the group consisting of a polypeptide having at least 80%, at least 90%, at least 95%, at least 97%, or at least 99% sequence identity with SEQ ID NO:15, or a polypeptide which differs from SEQ ID NO:15 by 1, 2, or 3 amino acid changes (substitutions, insertions, and/or deletions, preferably substitutions); a polypeptide having at least 80%, at least 90%, at least 95%, at least 97%, or at least 99% sequence identity with SEQ ID NO:18, or a polypeptide which differs from SEQ ID NO:18 by 1, 2, or 3 amino acid changes (substitutions, insertions, and/or deletions, preferably substitutions); a polypeptide having at least 80%, at least 90%, at least 95%, at least 97%, or at least 99% sequence identity with SEQ ID NO:19 or a polypeptide which differs from SEQ ID NO:19 by 1, 2, or 3 amino acid changes (substitutions, insertions, and/or deletions, preferably substitutions); and combinations thereof,
(ii) at least one building block comprising an epitope of from gamma-gliadin which is selected from the group consisting of: a polypeptide having at least 80%, at least 90%, at least 95%, at least 97%, or at least 99% sequence identity with SEQ ID NO:16, or a polypeptide which differs from SEQ ID NO:16 by 1, 2, or 3 amino acid changes (substitutions, insertions, and/or deletions, preferably substitutions); a polypeptide having at least 80%, at least 90%, at least 95%, at least 97%, or at least 99% sequence identity with SEQ ID NO:17, or a polypeptide which differs from SEQ ID NO:17 by 1, 2, or 3 amino acid changes (substitutions, insertions, and/or deletions, preferably substitutions); and combinations thereof, and
(iii) at least one building block comprising an epitope from omega-gliadin which is selected from the group consisting of: a polypeptide having at least 80%, at least 90%, at least 95%, at least 97%, or at least 99% with SEQ ID NO:20, or a polypeptide which differs from SEQ ID NO:20 by 1, 2, or 3 amino acid changes (substitutions, insertions, and/or deletions, preferably substitutions); a polypeptide having at least 80%, at least 90%, at least 95%, at least 97%, or at least 99% sequence identity with SEQ ID NO:21, or a polypeptide which differs from SEQ ID NO:21 by 1, 2, or 3 amino acid changes (substitutions, insertions, and/or deletions, preferably substitutions), and combinations thereof,
the said building blocks being directly connected to each other or being connected by means of one or more linkers, in any order.

In another aspect, the immunogenic polypeptide construct for use as described herein comprises at least four building blocks BB1, BB2, BB3, and BB4, defined as follows:
- BB1 is a polypeptide of SEQ ID NO:15 or which differs from SEQ ID NO:15 by one, two, three, four, five, six, seven, eight, or nine amino acid change(s) (substitutions, insertions, and/or deletions),
- BB2 has at least about 40%, at least about 50%, at least about 60%, at least about 70%, at least about 80%, at least about 85%, at least about 90%, at least about 95%, at least about 97%, or at least about 99% sequence identity with SEQ ID NO:16 (BB2a) or at least about 40%, at least about 50%, at least about 60%, at least about 70%, at least about 80%, at least about 85%, at least about 90%, at least about 95%, at least about 97%, or at least about 99% sequence identity with SEQ ID NO:17 (BB2b),
- BB3 has at least about 40%, at least about 50%, at least about 60%, at least about 70%, at least about 80%, at least about 85%, at least about 90%, at least about 95%, at least about 97%, or at least about 99% sequence identity with SEQ ID NO:20 (BB3a) or at least about 40%, at least about 50%, at least about 60%, at least about 70%, at least about 80%, at least about 85%, at least about 90%, at least about 95%, at least about 97%, or at least about 99% sequence identity with SEQ ID NO:21 (BB3b),
- BB4 has at least about 40%, at least about 50%, at least about 60%, at least about 70%, at least about 80%, at least about 85%, at least about 90%, at least about 95%, at least about 97%, or at least about 99% sequence identity with SEQ ID NO:18 (BB4a) or at least about 40%, at least about 50%, at least about 60%, at least about 70%, at least about 80%, at least about 85%, at least about 90%, at least about 95%, at least about 97%, or at least about 99% sequence identity with SEQ ID NO:19 (BB4b), and
- BB1, BB2, BB3, and BB4 are directly connected to each other or are connected by means of one or more linkers, in any order. In some embodiments, some of the building blocks may be connected directly to each other while other building blocks are connected to those or other building by blocks by means of one or more linkers, in any order.

The immunogenic polypeptide construct of the invention for use as described herein comprises the following combination of building blocks:
- Combination 1: BB1, BB2 is BB2a, BB3 is BB3a, BB4 is BB4a.

In a particular aspect, the polypeptide construct for use as described herein comprises, consists essentially of, or consists of, a moiety of formula (I):

[BBw]-[L1]n1-[BBx]-[L2]n2-[BBy]-[L3]n3-[BBz] (I)

wherein:
- BBw, BBx, BBy and BBz are building blocks selected from BB1, BB2, BB3, and BB4 as defined herein, with the proviso that each building block is different and the polypeptide construct comprises at least one BB1, at least one BB2, at least one BB3, and at least one BB4,
- n1, n2, and n3 are independently 0 or 1,
- L1, L2, and L3 are linkers, each linker being of the following formula (II):

   [CC1]a-[SP1]b-[CC2]c (II)

   wherein:
   - each a, b, and c is independently 0 or 1,
   - each CC1 and CC2 are independently selected from peptides having from 2 to 20 amino acid residues in length, preferably from 1 to 10 amino acid residues in length, and preferably comprising a cathepsin cleavage site such as KK, AYY, RR, or GPGPG (SEQ ID NO:41),
      and
   - SP1 is a spacer group comprising up to 200 carbon atoms and which can optionally include a peptide moiety or a non-peptide moiety.

For instance, the immunogenic polypeptide construct for use as described herein comprises, consists essentially of, or consists of, a moiety of formula (Ia):

[BBw]-[CCw]a1-[BBx]-[CCx]a2-[BBy]-[CCy]a3-[BBz] (Ia)

wherein:
- BBw, BBx, BBy, and BBz are each building blocks selected from BB1, BB2, BB3, and BB4, with the proviso that each building block is different, and the polypeptide construct comprises at least one BB1, at least one BB2, at least one BB3, and at least one BB4,
- BB1 is a polypeptide of SEQ ID NO:15 or which differs from SEQ ID NO:15 by one, two, three, four, five, six, seven, eight, or nine amino acid change(s) (substitutions, insertions, and/or deletions),
- BB2 has at least about 40%, at least about 50%, at least about 60%, at least about 70%, at least about 80%, at least about 85%, at least about 90%, or at least about 95% sequence identity with SEQ ID NO:16 (BB2a) or at least about 40%, at least about 50%, at least about 60%, at least about 70%, at least about 80%, at least about 85%, at least about 90%, or at least about 95% sequence identity with SEQ ID NO:17 (BB2b), preferably wherein BB2 is BB2a,
- BB3 has at least about 40%, at least about 50%, at least about 60%, at least about 70%, at least about 80%, at least about 85%, at least about 90%, or at least about 95% sequence identity with SEQ ID NO:20 (BB3a) or at least about 40%, at least about 50%, at least about 60%, at least about 70%, at least about 80%, at least about 85%, at least about 90%, or at least about 95% identity with SEQ ID NO:21 (BB3b), preferably wherein BB3 is BB3a,
- BB4 has at least about 40%, at least about 50%, at least about 60%, at least about 70%, at least about 80%, at least about 85%, at least about 90%, or at least about 95% sequence identity with SEQ ID NO:18 (BB4a) or at least about 40%, at least about 50%, at least about 60%, at least about 70%, at least about 80%, at least about 85%, at least about 90%, or at least about 95% sequence identity with SEQ ID NO:19 (BB4b), preferably wherein BB4 is BB4a,
- BB1, BB2, BB3, and BB4 are directly connected or are connected by means of one or more linkers, in any order,
   - a1, a2, and a3 are independently 0 or 1, preferably 1, and
   - CCw, CCx, and CCy are independently selected from peptides having from 2 to 10 amino acid residues in length and comprising a cathepsin cleavage sites such as KK, AYY, RR, or GPGPG (SEQ ID NO:41).

In the immunogenic polypeptide construct of the invention for use as described herein, CCw, CCx, and CCy are each peptides comprising a cathepsin cleavage site, independently selected from the group consisting of KKKK (SEQ ID NO:32), KK, GPRRKK (SEQ ID NO:33), GPKK (SEQ ID NO:34), GARRKK (SEQ ID NO:35), KGKK (SEQ ID NO:45), AYY, RR, and GCRRKK (SEQ ID NO:36), preferably independently selected from the group consisting of GPRRKK (SEQ ID NO:33), GPKK (SEQ ID NO:34), GARRKK (SEQ ID NO:35), and GCRRKK (SEQ ID NO:36). In some embodiments, the C in the linker of SEQ ID NO:36 can be substituted with one or more of G, V, I, L, or M.

The immunogenic polypeptide construct of the invention for use as described herein is of formula (Ic):

[BB1]-[CCw]-[BB2a]-[CCx]-[BB3a]-[CCy]-[BB4a] (Ic)

wherein
- CCw, CCx, and CCy are peptides comprising a cathepsin cleavage site, independently selected from the group consisting of KKKK (SEQ ID NO:32), KK, GPRRKK (SEQ ID NO:33), GPKK (SEQ ID NO:34), GARRKK (SEQ ID NO:35), AYY, RR, and GCRRKK (SEQ ID NO:36), in particular GPRRKK (SEQ ID NO:33), GPKK (SEQ ID NO:34), GARRKK (SEQ ID NO:35) and GCRRKK (SEQ ID NO:36), and
- BB1 is of SEQ ID NO:15, BB2a is of SEQ ID NO:16, BB3a is of SEQ ID NO:20, and BB4a is of SEQ ID NO:18.

In a particular aspect, the immunogenic polypeptide construct for use as described herein comprises, consists essentially of, or consists of, a polypeptide having at least about 40%, at least about 50%, at least about 60%, at least about 70%, or at least about 80% sequence identity, preferably at least about 85%, at least about 90%, at least about 95%, at least about 97%, or at least about 99% sequence identity with a polypeptide selected from SEQ ID NO:22 or 23.

In another aspect, the immunogenic polypeptide construct for use as described herein comprises, consists essentially of, or consists of, a polypeptide having at least about 40%, at least about 50%, at least about 60%, at least about 70%, or at least about 80% sequence identity, preferably at least about 85%, at least about 90%, at least about 95%, at least about 97%, or at least about 99% sequence identity with a polypeptide selected from SEQ ID NO:24 to 27, 42 to 44, and 46.

In some embodiments, the immunogenic polypeptide construct of the invention for use as described herein is a polypeptide and is characterized by one or several of the following features:
The C-terminal end of the polypeptide construct is amidated, and/or
The N-terminal end of the polypeptide construct is acetylated, and/or
The N-terminal amino acid residue in the polypeptide construct is selected from lysine or arginine.

In an embodiment, the immunogenic polypeptide construct of the invention is characterized by the features wherein the C-terminal end of the polypeptide construct is amidated and the N-terminal end of the polypeptide construct is acetylated.

In some embodiments, the immunogenic polypeptide construct of the invention as described herein is administered by means of a skin patch, wherein the skin patch comprises a backing having a periphery adapted to create a hermetically closed chamber when applied on the subject's skin, the polypeptide construct is present in dry form, optionally in admixture with one or several pharmaceutically acceptable excipients, on the backing of the patch, and wherein the polypeptide construct is solubilized by trans-epidermal water loss whereby the polypeptide construct can cross the stratum corneum and penetrate into the epidermis so as to reach epidermal dendritic cells.

In some embodiments, the skin patch is as described in, e.g., WO 2023/066846 (published on April 27, 2023, titled "Improved patches and methods for delivering an active substance to the skin using the same").

For instance, the immunogenic polypeptide construct of the invention for use as described herein is administered to a symptomatic subject suffering from celiac disease, preferably under a gluten-free diet.

The invention also provides a polypeptide construct as defined in claims 1-7.

It is disclosed a polypeptide construct which comprises, consists essentially of, or consists of, a polypeptide having at least about 40%, at least about 50%, at least about 60%, at least about 70%, at least about 80%, at least about 85%, at least about 90%, or at least about 95% sequence identity with a polypeptide selected from SEQ ID NO:22 or 23.

The application also provides a skin patch for increasing immune tolerance to gliadin, which comprises a polypeptide construct of the invention.

The application further provides a pharmaceutical composition which comprises a polypeptide construct of the invention in combination with at least one pharmaceutically acceptable excipient.

### BRIEF DESCRIPTION OF THE FIGURES

The following is a brief description of the figures; a more detailed description of each Figure follows at the end of the specification.

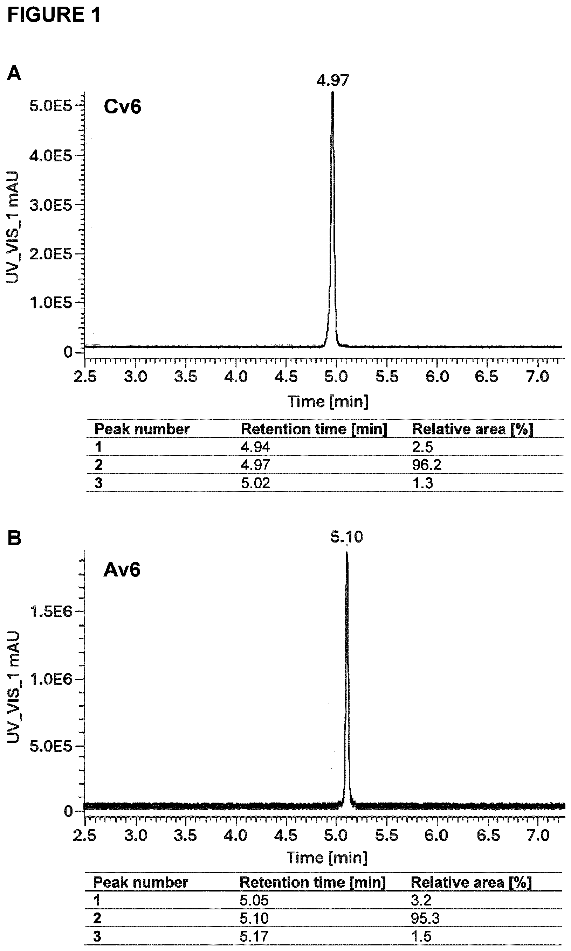

Figures 1A-1G are graphs depicting the analysis of three purified gliadin polypeptides (Cv6, Av6, and 4K) and four individual building blocks (BB1, BB2a, BB3a, and BB4a) by RP-HPLC. The three gliadin polypeptide constructs Cv6 (**A**), Av6 (**B**) and 4K (**C**), as well as the four building blocks BB1 (**D**), BB2a (**E**), BB3a (**F**), and BB4a (**G**) were produced by standard SPPS and purified by preparative HPLC. Purified materials were analyzed by RP-HPLC at 60°C on a Thermo Scientific Vanquish using a WATERS Acquity CSH C18 1.7µm 130Å (2.1x100mm) column. HPLC chromatograms are shown, and the characteristics of main peaks are described in the tables below. The relative area of the main peak number 2, expressed as percentage, is representative of the final purity of the synthetic products.

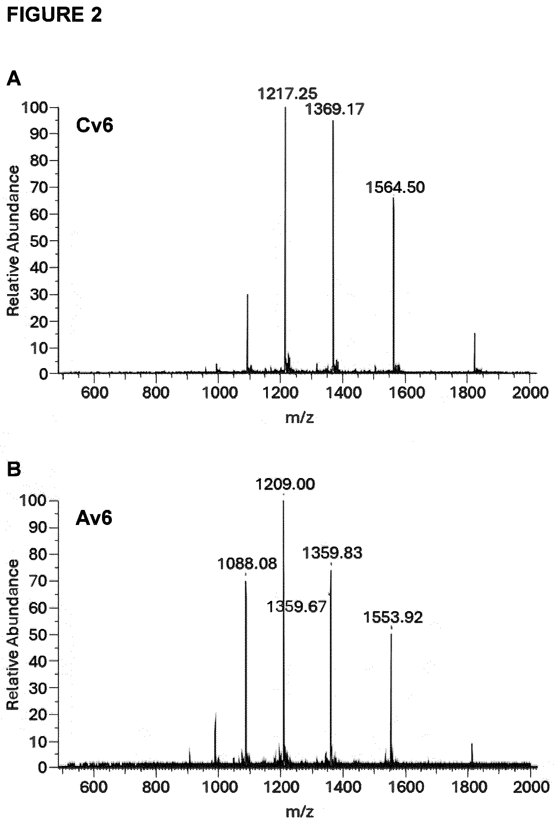

Figures 2A-2G are graphs depicting the analysis of three purified gliadin polypeptides (Cv6, Av6, and 4K) and four individual building blocks (BB1, BB2a, BB3a, and BB4a) by mass spectrometry. The three gliadin polypeptide constructs Cv6 (**A**), Av6 (**B**), and 4K (**C**), as well as the four building blocks BB1 (**D**), BB2a (**E**), BB3a (**F**), and BB4a (**G**) were produced by standard SPPS and purified by preparative HPLC. Purified materials were analyzed by mass spectrometry using heated electrospray ionization method on a Thermo Scientific LTQ XL. Mass spectra are shown.

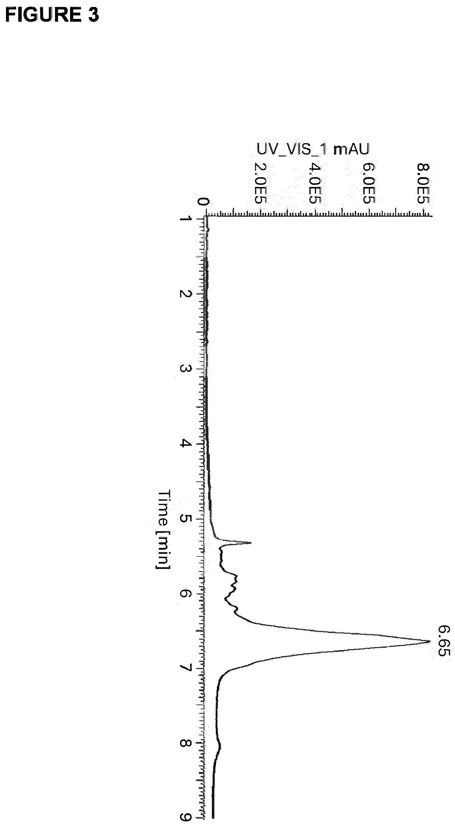

Figure 3 is a graph depicting the analysis of acetic acid-treated gliadin by RP-HPLC. Acetic acid-treated gliadin was prepared by treating commercial gliadin (Sigma Aldrich, ref. 3375) with 50 mM acetic acid for 1 hour at 40°C. Following centrifugation, the clarified supernatant was analyzed by RP-HPLC at 60°C on a Thermo Scientific Vanquish using a WATERS BEH C4 3.5µm 300Å (2.1x100mm) column. HPLC chromatogram is shown.

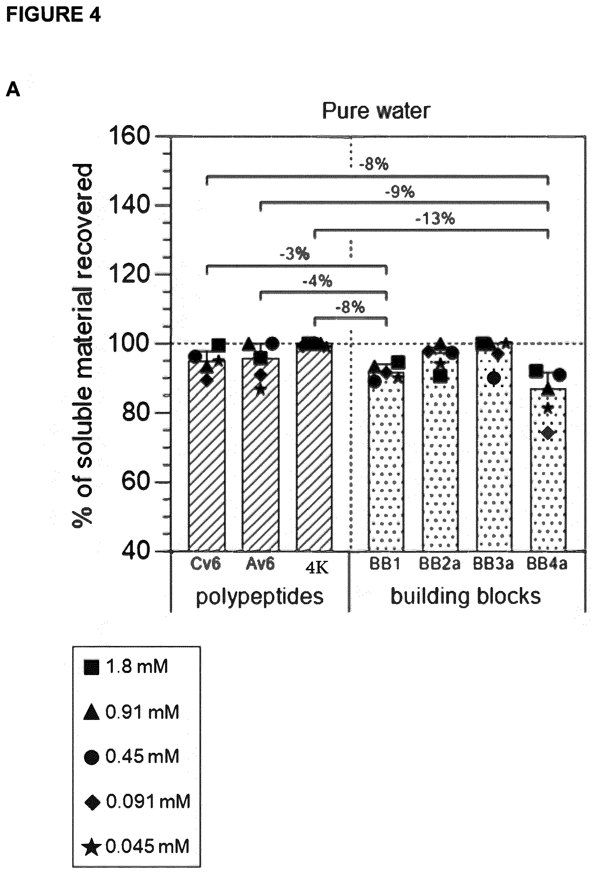

Figures 4A-4C are graphs depicting the analysis of the solubility of three gliadin polypeptides (Cv6, Av6, and 4K) and four individual building blocks (BB1, BB2a, BB3a, and BB4a) in each of three different solvents (pure water, PBS 1X, and artificial perspiration) at five different molarities each. Freeze-dried aliquots of the three gliadin polypeptide constructs Cv6, Av6 and 4K, as well as the four building blocks BB1, BB2a, BB3a, and BB4a were reconstituted in pure water (A), PBS 1X (pH7) (**B**), or artificial perspiration (pH 4.5) (**C**) at five different molarities (approximately 1.8, 0.91, 0.45, 0.091 or 0.045 mM, corresponding to respective concentrations of 20, 10, 5, 1, and 0.5 mg/mL for the polypeptide construct Cv6), as indicated. The concentration in soluble material was measured by HPLC at 215 nm. Data are median + interquartile range of the different molarities tested. Data are expressed as the percentage of soluble material recovered, based on the expected concentration (all values above 100% were set to 100).

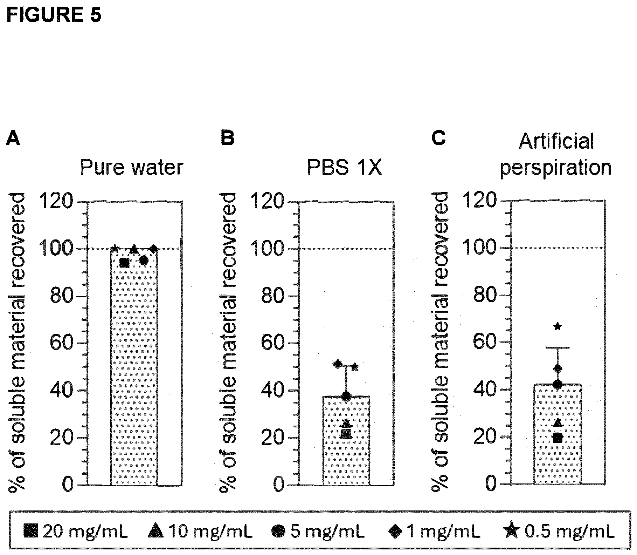

Figures 5A-5C are graphs depicting the analysis of the solubility of acetic acid-treated gliadin in each of three different solvents (pure water, PBS 1X, and artificial perspiration) at five different concentrations each. Freeze-dried aliquots of acetic acid-treated gliadin were reconstituted in pure water (**A**), PBS 1X (pH7) (**B**), or artificial perspiration (pH 4.5) (**C**) at five different concentrations (20, 10, 5, 1 and 0.5 mg/mL), as indicated. The concentration in soluble material was measured by HPLC at 215 nm. Data are median + interquartile range of the different concentrations tested. Data are expressed as the percentage of soluble material recovered, based on the expected concentration (all values above 100% were set to 100).

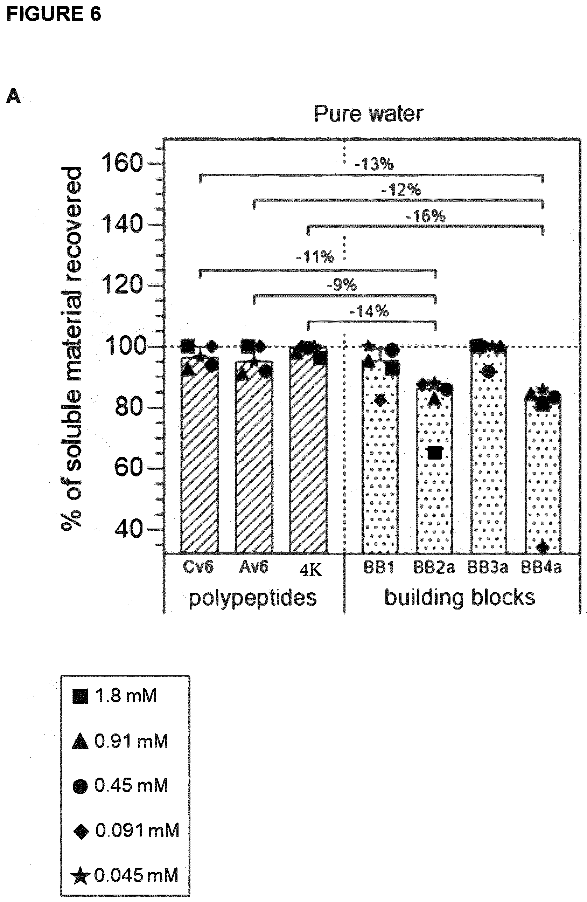

Figures 6A-6C are graphs depicting the analysis of the solubility of three gliadin polypeptides (Cv6, Av6, and 4K) and four individual building blocks (BB1, BB2a, BB3a, and BB4a) in each of three different solvents (pure water, PBS 1X, and artificial perspiration) at five different molarities each, upon drying on PET-Ti backing film. Freeze-dried aliquots of the three gliadin polypeptide constructs Cv6, Av6, and 4K, as well as the four building blocks BB1, BB2a, BB3a, and BB4a were reconstituted in pure water, loaded on a PET-Ti backing film and dried in an oven (at ~30°C). Immediately after drying, dry deposits were reconstituted at five different molarities (approximately 1.8, 0.91, 0.45, 0.091 or 0.045 mM) by pipetting up and down with either pure water (**A**), PBS 1X (**B**), or artificial perspiration (**C**). The concentration in soluble material was measured by HPLC at 215 nm from the solutions recovered from PET-Ti backing films. Data are median + interquartile range of the different molarities tested. Data are expressed as the percentage of soluble material recovered, based on the expected concentration (all values above 100% were set to 100).

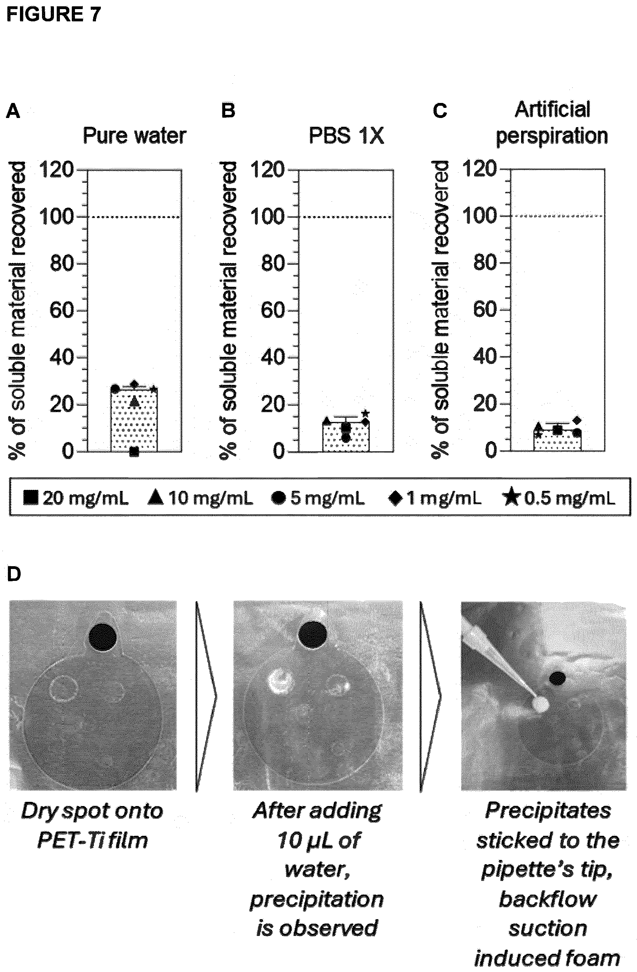

Figures 7A-7C are graphs depicting the analysis of the solubility of acetic acid-treated gliadin in each of three different solvents (pure water, PBS 1X, and artificial perspiration) at five different concentrations each, upon drying on PET-Ti backing film. Figure 7D are images illustrating the formation of insoluble precipitates when reconstituting dry deposits of acetic acid-treated gliadin with pure water. Freeze-dried aliquots of acetic acid-treated gliadin were reconstituted in pure water, loaded on a PET-Ti backing film and dried in an oven (at ~30°C). Immediately after drying, dry deposits were reconstituted at five different concentrations (20, 10, 5, 1 and 0.5 mg/mL) by pipetting up and down with either pure water (**A**), PBS 1X (**B**), or artificial perspiration (**C**). The concentration in soluble material was measured by HPLC at 215 nm from the solutions recovered from PET-Ti backing films. Data are presented as median + interquartile range of the different molarities tested. Data are expressed as the percentage of soluble material recovered, based on the expected concentration (all values above 100% were set to 100). (**D**) As an illustration, photographs of the reconstitution of dry deposits with pure water are shown and annotated.

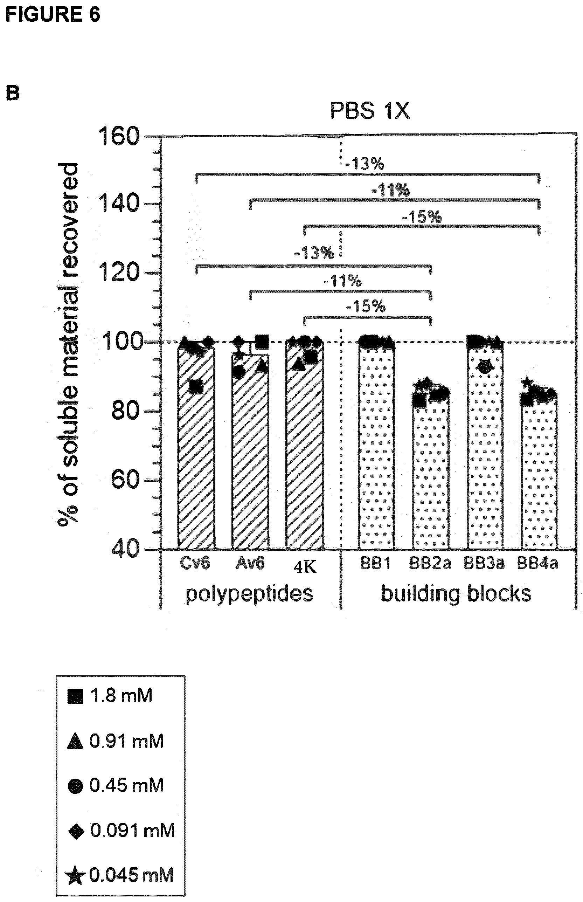

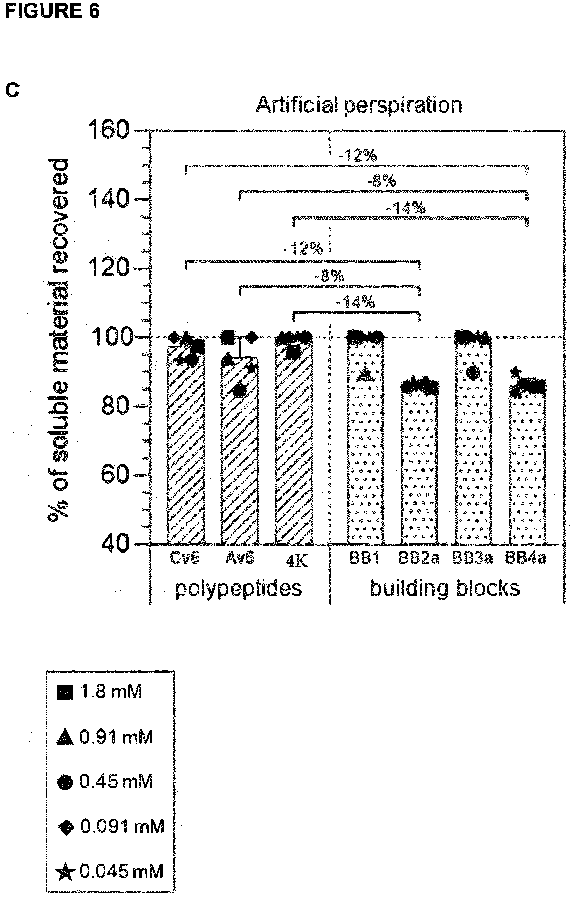

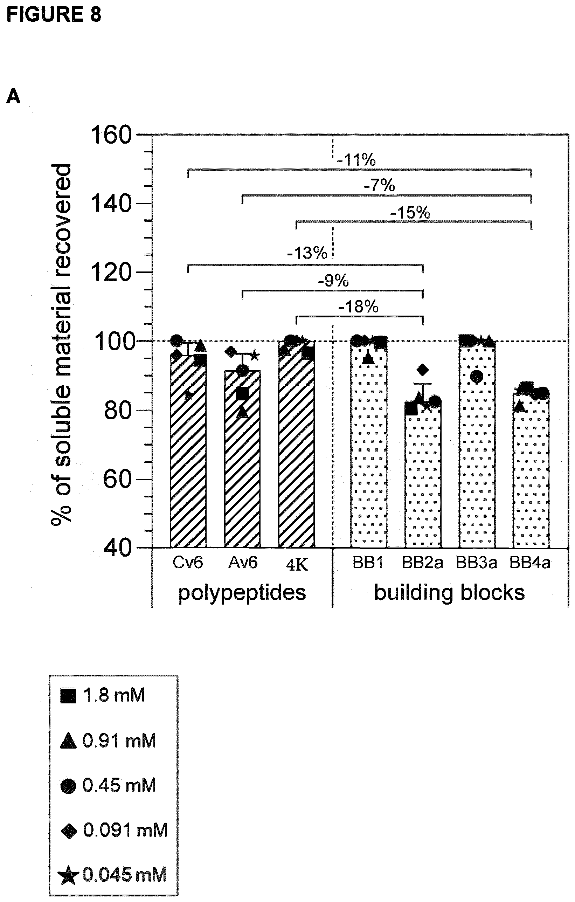

Figures 8A-8F are graphs depicting the analysis of the solubility of three gliadin polypeptides (Cv6, Av6, and 4K) and four individual building blocks (BB1, BB2a, BB3a, and BB4a) in each of three solvents (pure water, PBS 1X, and artificial perspiration) at five different molarities, upon drying on PET-Ti backing film and storage for 1 month at room temperature or 5°C. Freeze-dried aliquots of the three gliadin polypeptide constructs Cv6, Av6, and 4K, as well as the four building blocks BB1, BB2a, BB3a, and BB4a were reconstituted in pure water and dried on a PET-Ti backing film as described in Figure 6. Backing films were stored for 1 month at room temperature (**A**, **B**, **C**) or 5°C (**D**, **E**, **F**), away from light and humidity. Dry deposits were then reconstituted at five different molarities (approximately 1.8, 0.91, 0.45, 0.091 or 0.045 mM) in water (**A**, **D**), PBS 1X (**B**, **E**), or artificial perspiration (**C**, **F**), as indicated. The concentration in soluble material was measured by HPLC at 215 nm from the solutions recovered from PET-Ti backing films. Data are median + interquartile range of the different molarities tested. Data are expressed as the percentage of soluble material recovered, based on the expected concentration (all values above 100% were set to 100).

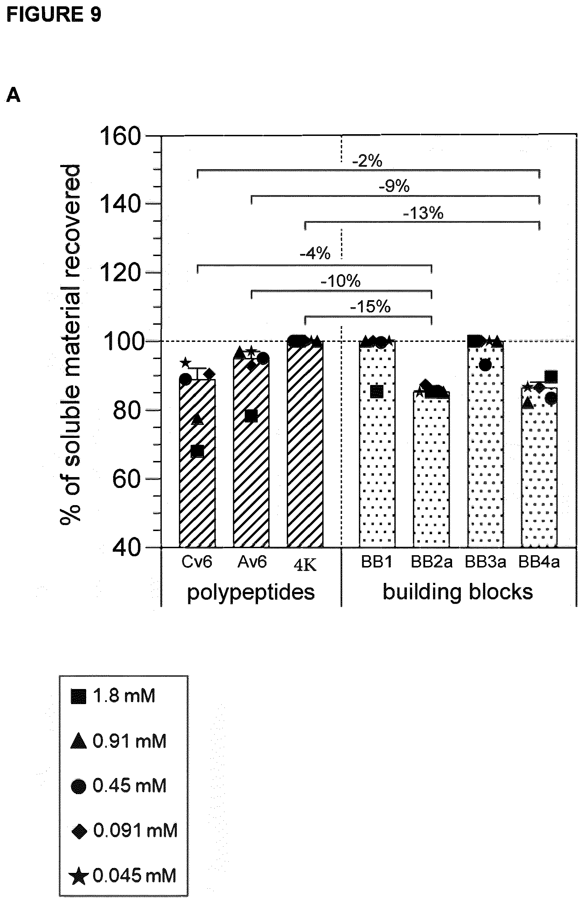

Figures 9A-9F are graphs depicting the analysis of the solubility of three gliadin polypeptides (Cv6, Av6, and 4K) and four individual building blocks (BB1, BB2a, BB3a, and BB4a) in each of three solvents (pure water, PBS 1X, and artificial perspiration) at five different molarities, upon drying on PET-Ti backing film and storage for 3 months at room temperature or 5°C. Freeze-dried aliquots of the three gliadin polypeptide constructs Cv6, Av6 and 4K, as well as the four building blocks BB1, BB2a, BB3a, and BB4a were reconstituted in pure water and dried on a PET-Ti backing film as described in Figure 6. Backing films were stored for 3 months at room temperature (**A**, **B**, **C**) or 5°C (**D**, **E**, **F**), away from light and humidity. Dry deposits were then reconstituted at five different molarities (approximately 1.8, 0.91, 0.45, 0.091 or 0.045 mM) in water (**A**, **D**), PBS 1X (**B**, **E**), or artificial perspiration (**C**, **F**), as indicated. The concentration in soluble material was measured by HPLC at 215 nm from the solutions recovered from PET-Ti backing films. Data are median + interquartile range of the different molarities tested. Data are expressed as the percentage of soluble material recovered, based on the expected concentration (all values above 100% were set to 100).

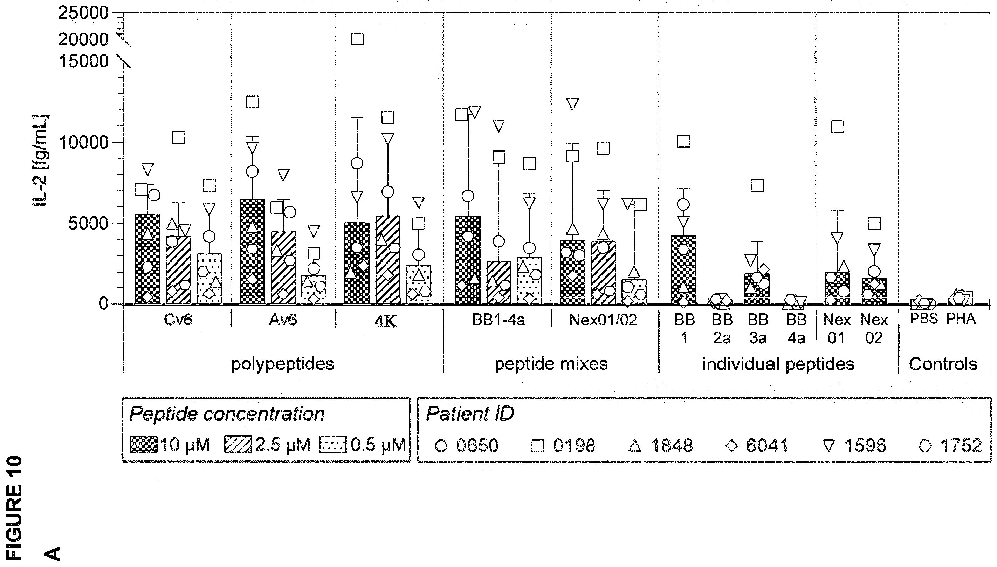

Figures 10A-10B are graphs depicting the evaluation of the ability of various molarities of three gliadin polypeptides (Cv6, Av6, and 4K), two peptide mixes containing either the four individual building blocks BB1, BB2a, BB3a and BB4a (BB1-4a) or two Nexvax2 peptides (Nex01/02), and six individual building blocks or peptides (BB1, BB2a, BB3a, BB4a, Nex01, and Nex02) to restimulate gluten-specific T cell collected from HLA-DQ2+ CeD patients using a whole blood IL-2 release assay. The whole blood cytokine release assay was performed in six HLA-DQ2+ participants with biopsy-confirmed celiac disease. Whole blood was stimulated with the three gliadin polypeptide constructs Cv6, Av6, and 4K at 10, 2.5, and 0.5 µM final molarities, as indicated. Alternatively, whole blood was stimulated with a mix of peptides containing either the four building blocks BB1, BB2a, BB3a, and BB4a (BB1-4a) or Nex01 and Nex 02 (Nex01/02) at 10, 2.5, and 0.5 µ M final molarities. Building blocks (BB1, BB2a, BB3a, and BB4a), Nex01, and Nex02 were also tested as individual peptides, at a single concentration of 10 µM. After 24 hours of incubation, the concentration of IL-2 was quantified from plasma using an MSD IL-2 S-plex ELISA. Data are expressed in femtogram/mL of plasma (A), or as fold change from the negative control (PBS 1X) (B). Symbols represent individual participants (patient ID); bars represent median + interquartile range of individual values.

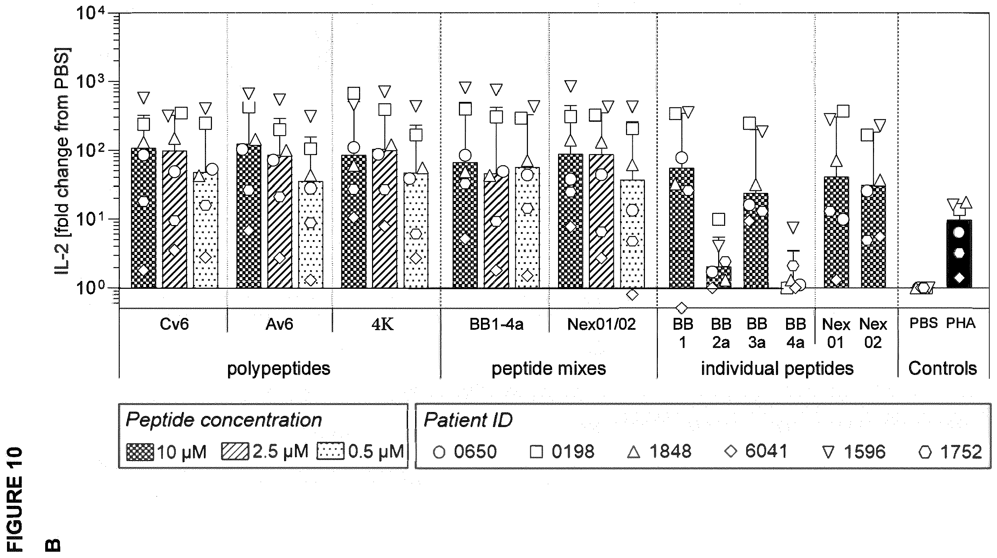

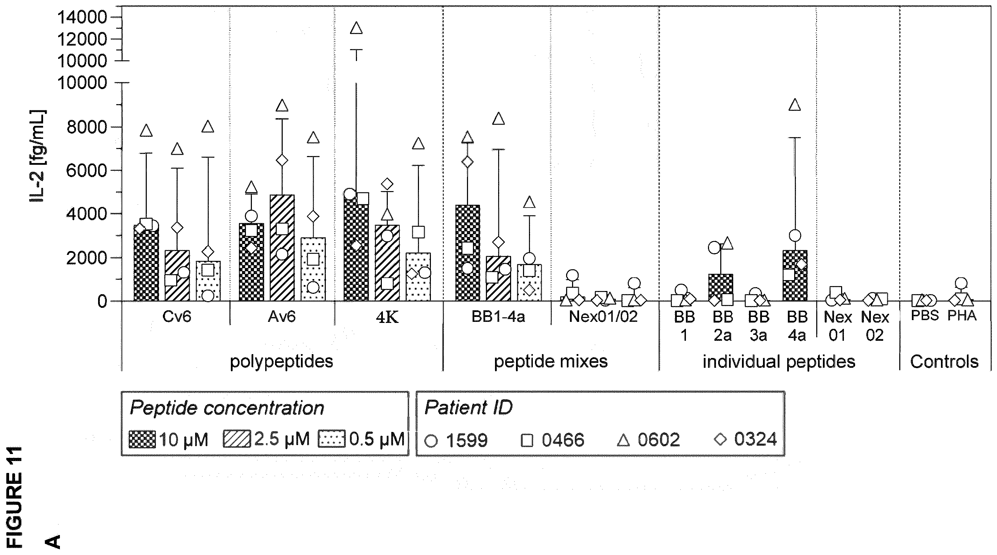

Figures 11A-11B are graphs depicting the evaluation of the ability of various molarities of three gliadin polypeptides (Cv6, Av6, and 4K), two peptide mixes containing either the four individual building blocks BB1, BB2a, BB3a and BB4a (BB1-4a) or two Nexvax2 peptides (Nex01/02), and six individual building blocks or peptides (BB1, BB2a, BB3a, BB4a, Nex01, and Nex02) to restimulate gluten-specific T cell collected from HLA-DQ8+ CeD patients using a whole blood IL-2 release assay. The whole blood cytokine release assay was performed in four HLA-DQ8+ participants with biopsy-confirmed celiac disease as described in Figure 10. Data are expressed in femtogram/mL of plasma (**A**), or as fold change from the negative control (PBS 1X) (**B**). Symbols represent individual participants (patient ID); bars represent the median + interquartile range of individual values.

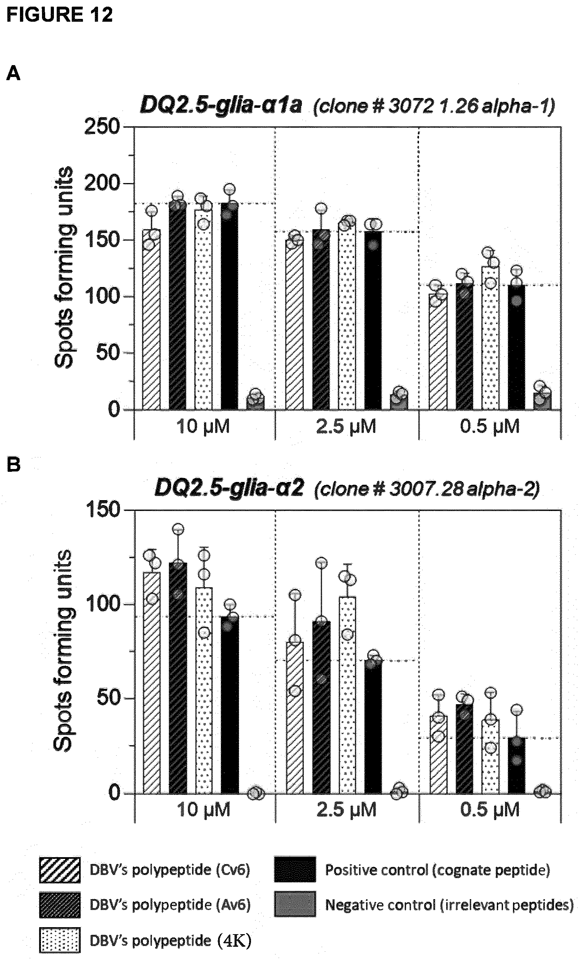

Figures 12A-12E are graphs depicting the evaluation of the ability of gliadin polypeptides (Cv6, Av6, and 4K) to reactivate gluten-specific T cell clones isolated from CeD patients and specific for a selection of key gliadin epitopes (i.e., DQ2.5-glia-α1a (**A**), DQ2.5-glia-α2 (**B**), DQ2.5-glia-ω1 (**C**), DQ2.5-glia-ω2 (**D**), and DQ8-glia-α1 (**E**)), by IFN-γ ELISpot assay. The three gliadin polypeptide constructs Cv6, Av6, and 4K were incubated overnight at three different molarities (10, 2.5, 0.5 mM) with irradiated PBMCs, and five T-cell clones originally isolated from HLA-DQ2+ or HLA-DQ8+ CeD patients. T cells clones were specific for either DQ2.5-glia-α1a (**A**), DQ2.5-glia-a2 (**B**), DQ2.5-glia-ω1 (**C**), DQ2.5-glia-ω2 (**D**) or DQ8-glia-α1 (**E**) epitopes. Spots were detected with IFN-γ-biotinylated antibody and streptavidin-ALP complex and counted using an AID ELISpot reader. Data are expressed as mean Spot Forming Units (SFU) per well of 3 replicate wells + SD.

### DETAILED DESCRIPTION

The references to methods of treatment in this description are to be interpreted as references to the compounds, pharmaceutical compositions and medicaments of the present invention for use in a method for treating a subject by therapy.

### General Definitions

As described herein, "*immunotherapy*" or "*immunotherapeutic treatment*" refers to a treatment wherein a desired immune reaction is induced in a patient to improve the patient's condition. Immunotherapeutic treatment encompasses the treatment of, vaccination against, and/or the prevention of a disease and/or disorder in the subject. In the context of the present application, a disease and/or disorder encompasses celiac disease (CeD) as well as any symptom, or manifestation associated with, or caused by, CeD in the subject.

As used herein, the terms "subject" and "patient" may at times be used interchangeably.

As used herein, the terms "disease" and "disorder" may at times be used interchangeably.

Celiac disease (also designated herein as CeD and also called celiac sprue or gluten-sensitive enteropathy) is a chronic small intestinal immune-mediated enteropathy triggered by ingestion of a complex protein, gluten, present in cereals such as wheat, barley, and rye, in genetically predisposed individuals. The most important genetic determinant of susceptibility to CeD is the presence of human leukocyte antigen-DQ (HLA-DQ) heterodimers DQ2 (encoded by alleles *DQA1***0501* and *DQB1***0201*) and HLA-DQ8 (encoded by alleles *DQA1***0301* and *DQB1***0302*). HLA-DQ2 is present in more than 90% and HLA-DQ8 in about 5% of patients with CeD. The disease encompasses a spectrum of conditions characterized by varying degrees of gluten sensitivity, including a severe form characterized by a flat small intestinal mucosa (hyperplastic villous atrophy) and other forms characterized by milder symptoms including (but not limited to) fatigue, chronic diarrhea, malabsorption of nutrients, weight loss, abdominal distension, and anemia.

Symptoms of CeD encompass both gastrointestinal and non-gastrointestinal symptoms. Gastrointestinal symptoms of CeD include chronic diarrhea, recurrent abdominal pain, nausea, vomiting, malabsorption of nutrients, abdominal distension, bloating, and gas.

Non-gastrointestinal symptoms include, without being limited to, weight loss, chronic anemia, iron deficiency, osteopenia, osteoporosis, dental enamel defect, irritability, chronic fatigue, itchy, blistering skin rash (dermatitis herpetiformis), mouth ulcers, joint pain, neuropathy, arthritis, arthralgia, amenorrhea, and increased liver enzymes. In children, other symptoms such as failure to thrive, short stature, and delayed puberty can be observed.

CeD can be associated with an enhanced risk for osteoporosis, development of secondary autoimmune diseases, and even intestinal malignancies (enteropathy-associated T-cell lymphoma (EATL) and adenocarcinoma of the small intestine) as well as non-Hodgkin's lymphoma.

As used herein, "*the treatment of a disease*" or "*treating a disease*" includes curing, delaying, alleviating, mitigating and/or slowing the progression of the disease or that of one or more symptoms thereof and/or disorders as well as the prevention, the mitigation, the attenuation, the slowing, the reverse, or the elimination of one or more of the symptoms or disorders generally caused by, or associated with, the disease.

The term "*treatment of a disease*" also encompasses the improvement or the normalization of a biological parameter in the subject suffering from said disease.

The terms "*prevention of a disease" or "preventing a disease"* include stopping, blocking, slowing down, inhibiting, or delaying the onset of a disease or one or more symptoms associated with said disease, or keeping the patient from being afflicted by the disease or any one or more symptoms thereof.

The term "*prevention of a disease*" also refers to any act intended to ameliorate the health status and/or the general condition of patients such as prophylaxis, and retardation of the disease or any one of symptoms thereof and/or to prevent the patient from being afflicted by the disease or any one of symptoms thereof. In some embodiments, this term also refers to reducing or minimizing the risk (or the probability) for a patient to develop said disease or symptom, as compared to a diseased subject who has not been administered the compound provided herein.

For instance, in the context of the present disclosure, the compound (i.e., the polypeptide construct) may be used to delay the onset of, or reduce or minimize the risk of, developing celiac disease and/or a symptom thereof (e.g., a gastrointestinal symptom) in a pre-symptomatic or paucisymptomatic patient with CeD (e.g., under gluten-free diet) or in a patient at risk of developing CeD.

As used herein, "*gluten*" refers to structural proteins naturally found in certain cereal grains, in particular in wheat species belonging to *Triticum genus* (such as common wheat (*T. aestivum*), *T. durum*, spelt (*T. spelt*), Khorasan (*T. turanicum*), emmer (*Triticum turgidum subsp. Dicoccum*) and einkorn (*T. monococcum*)), barley, rye, and some oat cultivars, as well as any cross hybrids of these grains (such as triticale). Wheat gluten primarily includes two types of proteins: the glutenins and the gliadins, which in turn can be divided into high molecular weight glutenins, low molecular weight glutenins, and gliadin isoforms. There are several isoforms of gliadin such as α-gliadins, β-gliadins (also referred to as beta-gliadins), γ-gliadins, and ω-gliadins. Examples of gluten proteins derived from cereals other than wheat encompass, without being limited to, hordeins in barley and secalins in rye.

As used herein, the term "*gliadin*" refers to the aqueous alcohol-soluble fraction of gluten, particularly, but not exclusively, gluten derived from wheat, for example, *Triticum aestivum.*

As used herein, tTG refers to "*tissue transglutaminase*" which is an important factor in CeD. Tissue transglutaminase causes selective deamidation of gluten, which in turn, leads to the generation of a series of fully or partially deamidated gluten peptides that bind to HLA-DQ2 or HLA-DQ8 molecules with increased affinity. The resulting HLA-DQ2-gluten peptide interaction or HLA-DQ8-gluten peptide interaction triggers the proinflammatory CD4⁺ T cell response.

As used herein, "*deamidation*" refers particularly to the substitution or the conversion of a glutamine residue to a glutamic acid residue (also called glutamate residue), a process that increases the propensity of gluten peptides to activate T cells. Substitution refers to the fact of using a glutamate residue instead of glutamine in chemical or recombinant synthesis. Conversion refers to the fact of converting a glutamine residue into a glutamate residue e.g., enzymatically or by using an appropriate chemical reagent (e.g., an acid such as acetic acid).

As used herein, "*glutamate*" and "*glutamic acid*" are used interchangeably.

As used herein, the terms "*human leukocyte antigen*" and "*HLA*" are here defined as a genetic fingerprint on human white blood cells and platelets, composed of proteins that play an important role in activating the body's immune system to respond to foreign organisms. In humans and other animals, the HLA is also referred to as the "major histocompatibility complex" (MHC).

As used herein, "*epitope*" refers to that portion of an antigen or a peptide that is recognized by the immune system, for example, a T cell receptor or the major histocompatibility complex (MHC) class I or class II, an antibody, a B cell receptor, which portion is sufficient for high affinity binding. Generally, a linear epitope for recognition by MHC receptors is from 7 to 17 amino acids in length.

The term "*polyepitope*" refers to the presence of two or more epitopes linked in a single polypeptide chain and more generally in a single molecule. In the context of the application, the polypeptide construct provided herein is a polyepitope.

As used herein, the terms "*antigen*" and "*immunogen*" and variations thereof are generally used interchangeably and refer to the epitope-containing structure recognized by the immune system. For instance, the polypeptide construct provided herein is an immunogen comprising several epitopes derived from gliadin isoforms.

The term "*sensitive to gluten*" refers to the state in which any one or more of the symptoms of CeD or an inappropriate T cell response are exhibited by a subject exposed to gluten, or peptide fragment thereof. In a subject who is not sensitive to gluten, there is little, or no T cell response caused by the ingestion of gluten. By contrast, in a subject sensitive to gluten, there is an inappropriate CD4⁺ T cell-mediated immune response to peptides derived from gluten after ingestion thereof.

As used herein, "*Treg*" refers to a subclass of T cells whose major role is to attenuate T cell-mediated immunity during an immune reaction, and to suppress auto-reactive T cells that escaped negative selection in the thymus. A "*Treg response*", as used herein, is characterized by the differentiation and proliferation of the population of CD4⁺ Treg cells which express the forkhead family transcription factor FOXP3 (forkhead box p3) and/or the MHC Class II (MHC-II) associated protein LAG-3, and/or express high levels of the IL-2 receptor alpha chain (CD25). The presence of Treg cells in the peripheral circulation or spleen may be determined by analysis of CD4⁺ T-cells expressing high levels of CD25⁺ on their surface and intracellular FOXP3 expression. This may conveniently be achieved using flow cytometry. In addition, or alternatively, Treg cells may be quantified by determining levels of FOXP3 mRNA in peripheral blood.

In the context of the present application, the term "*tolerance*" refers to a reduction in immunological reactivity of a subject towards any gliadin and/or any fragment(s) thereof, including immunodominant epitope(s) thereof. The term "*inducing tolerance*", or "*increasing tolerance*" refers to a treatment that reduces or eliminates sensitivity/reactivity to gliadin in a gliadin-sensitive subject by reorientating the inflammatory immune response to gliadin into a tolerogenic one. An "*increase in tolerance*" or an "*improvement in tolerance*" in a subject suffering from CeD can be evidenced by any means known by the skilled artisan, in particular by measuring the plasma level of antibodies specific to gliadin and/or tissue transglutaminase (i.e., anti-gliadin and/or anti-tTG antibodies), quantifying and analyzing the secretion of cytokines by gliadin-specific T-cells and/or quantifying and analyzing Tregs in whole blood or peripheral blood mononuclear cells (PBMCs) after an oral gluten challenge, and/or evaluating lesions of the small intestine by histological analysis of biopsies after an oral challenge. Other example methods to monitor the immunotherapeutic methods provided herein are described further below.

As used herein, "*epicutaneous administration*" or "*epicutaneous application*" designates the administration or application of a compound on the surface of the skin of a subject under conditions allowing contact with the surface of the skin. Epicutaneous administration typically comprises skin application under conditions sufficient to allow penetration or diffusion of the compound in the superficial layer(s) of the skin, preferably in the epidermis layers so that the compound can reach epidermal antigen-presenting cells known as Langerhans cells (LCs). Epicutaneous administration is preferably performed on intact and/or healthy skin area. In some embodiments, vaccines can be administered via epicutaneous administration.

As used herein, "*epidermis*" refers to the outer layer of the skin, the inner layers being the dermis and hypodermis. The epidermis is composed of 4 or 5 layers depending on the region of the skin being considered. These layers, in descending order, are the *stratum corneum*, the *stratum lucidum*, the *stratum granulosum*, the *stratum spinosum*, and the *stratum basale.* The *stratum corneum* is mostly composed of corneocytes which correspond to keratinocytes in their last stage of differentiation. The *stratum corneum* has a thickness generally ranging from 10 to 40 µm depending on the location of the skin and the age of the subject.

As used herein, "*an intact skin area*" refers to a skin area which has not been pretreated before the application of the compound of interest (i.e., the polypeptide construct provided herein).

As described herein "*epicutaneous immunotherapy*" or "EPIT" refers to an immunotherapy treatment wherein compound(s) of interest able to induce a desired immune reaction is/are administered by epicutaneous route, as defined above, e.g., by means of a skin patch.

In the context of the present application, "*epicutaneous immunotherapy*" encompasses the sequential administration of one or more of the polypeptide constructs provided herein, to induce or increase the tolerance to gliadin in a subject sensitive to gliadin, and in particular in a subject suffering from CeD.

As used herein, a "*compound*" designates any molecule able to induce a desired immune response in a subject in need thereof, such as any polypeptide construct provided herein.

The terms "*polypeptide*", "*peptide*", and "*protein*" are used interchangeably herein to refer to a polymer of amino acid residues. A polypeptide comprises at least 2 amino acid residues and in the context of the present application, generally comprises less than about 500, less than about 400, or less than about 300 amino acid residues, such as less than about 250, less than about 200, less than about 180, less than about 150 or less than about 120 amino acid residues; while generally straight, the polypeptide can in some instances be branched. In the context of the present disclosure, the polypeptide has generally at least about 30, about 50, about 70, or about 80 amino acid residues in length. The terms also apply to amino acid polymers in which one or more amino acid residues may be modified or may correspond to non-naturally occurring residues, such as an artificial chemical mimetic of a corresponding naturally occurring amino acid. In other words, the polypeptide construct may comprise naturally occurring amino acids, non-naturally occurring amino acids such as pyroglutamic acid (also called herein pyroE or pE), hydroxyproline, valine, citrulline, and the like, modified amino acids, and the D and L stereoisomers of each.

Non-natural or modified amino acid residues may preferably occur at the C-terminal and/or the N-terminal end of the polypeptide construct.

For instance, the polypeptide can bear amidation at its C-terminal amino acid residue (-CONH₂ instead of -COOH).

As a further example, the polypeptide construct can comprise a pyroglutamate (pE) at its N-terminal amino acid residue or acetylation (Ac) on terminal NH₂ (namely - CH₃CONH instead of NH₂).

The polypeptide construct may be also conjugated to a moiety able to increase its solubility such as PEG moieties. Such conjugation preferably occurs at the N-terminal or C-terminal end of the polypeptide construct.

As used herein, "*amino acid changes*" herein include amino acid mutations such as substitution, insertion, and/or deletion in a polypeptide sequence. By "*amino acid substitution*" or "*substitution*" herein is meant the replacement of an amino acid at a particular position in a parent polypeptide sequence with another amino acid. By "*amino acid insertion*" or "*insertion*" is meant the addition of an amino acid at a particular position in a parent polypeptide sequence. By "*amino acid deletion*" or "*deletion*" is meant the removal of an amino acid at a particular position in a parent polypeptide sequence. The amino acid substitutions may be conservative. A "*conservative substitution*" is the replacement of a given amino acid residue by another residue having a side chain ("R-group") with similar chemical properties (e.g., charge, bulk, and/or hydrophobicity). In general, a conservative amino acid substitution will not substantially change the functional properties of a protein or peptide. Conservative substitutions and the corresponding rules are well-described in the state of the art. In some embodiments of the present invention, the one or more amino acid substitutions are conservative.

As used herein, the term "*variant sequence*" refers to a polypeptide sequence that includes an "*amino acid change*" or "*amino acid changes*."

As used herein, "Z" present in the amino acid sequences refers either to glutamine (Q) or glutamate (E). The position of "Z" residue refers to the position of a glutamine residue amenable to deamidation catalyzed by tTG or consistent with the amino-acid motif that predicts susceptibility to deamidation by tTG, i.e., Q->E in the motif QXP where X is not a glycine or a proline, or in the motif QXB where B is a hydrophobic amino acid (e.g., a phenylalanine, a tyrosine, a tryptophan, a leucine or an isoleucine).

As used herein, a "*polypeptide construct*" refers to a non-naturally occurring, synthetic, or recombinant compound comprising one or several polypeptide regions, namely, one or more polymers of amino acid residues. Said polypeptide regions may be directly linked to each other, and/or may be linked to each other via one or more linkers. The polypeptide construct may be linear, branched, or cyclic. In some embodiments, the polypeptide construct is linear. The linker(s), if used, may include peptide linker(s) and/or may include non-peptide linker(s). Any linker(s) in the polypeptide construct comprise moieties that do not participate in the induction of immunotolerance to gliadin. Instead, any linker(s) may link two or more of the polypeptide regions or building blocks together in the polypeptide construct. The linker(s) additionally may facilitate the chemical synthesis and/or may provide a physical separation between two vicinal building blocks, for example, so as to (i) prevent or avoid the formation of neo-epitope or cryptic epitopes, (ii) facilitate the cell processing of the building blocks into epitopes of interest, and/or (iii) facilitate the binding of the polypeptide construct to gliadin-specific antibodies. The linker(s) can be also designed so as to improve the solubility of the polypeptide construct in water and/or the ability of the polypeptide construct to penetrate into the epidermis. Non-limiting examples of linkers of interest are described further below.

Typically, the linker(s) present in the polypeptide construct is/are peptide linkers of 2 to 20, preferably from 2 to 10 amino acid residues in length. Amino acid residues of interest that can be present in the linkers are glycine (G), proline (P), arginine (R), lysine (K), cysteine (C), tyrosine (Y) and alanine (A).

As used herein "*at least one*" means one or several, and includes 1, 2, 3, 4, 5, 6, 7, 8, 9, 10, or more entities (e.g., epitopes) of interest.

As used herein, the term "*about*" means ± 10%, preferably ± 5%, preferably ± 2.5%, preferably ± 1% of the indicated value. For clarity purposes, when discussing percentages, it should be understood that the phrase, e.g., "about 50%" should be interpreted as meaning: (i) "45% to 55%" (when "about" means ± 10%), (ii) "47.5% to 52.5%" (when "about" means ± 5%), and (iii) "48.75% to 51.25%" (when "about" means ± 2.5%). In contrast, it should be understood that the phrase, e.g., "about 10%" does not mean: (i) "0% to 20%" (when "about" means ± 10%), (ii) "5% to 15%" (when "about" means ± 5%), and (iii) "7.5% to 12.5%" (when "about" means ± 2.5%).

Various numerical values may be presented herein in a range format. It should be understood that such a range format is used merely for convenience and/or brevity; a range format be interpreted flexibly to include not only the numerical values explicitly recited as the limits (i.e., endpoints) of the range but also to include all the individual numerical values or sub-ranges encompassed within that range as if each numerical value and sub-range is explicitly recited herein. When the term "about" modifies a numerical value provided as a range, the term "about" should be understood as independently modifying either or both limits (i.e., endpoints) of the range. For example, it should be understood that the range "about 1 to 10" can be interpreted as meaning "about 1 to about 10" (where both values are modified by "about"), "1 to about 10" (where only the second value is modified by "about"), and "about 1 to 10" (wherein only the first value is being modified by "about"). Moreover, when both values are modified by "about", each value is independently modified by "about"; meaning that it is envisioned that it is possible that if "about" means, e.g., ± 10% for the first value, "about" independently means either ± 10%, ± 5%, ± 2.5%, or ± 1% for the second value, and vice versa, etc.

Throughout this specification and claims, the word "*comprise*," or linguistic variations such as "*comprises*" or "*comprising*", denotes the presence of recited feature(s), element(s), property(ies), method step(s), etc. without the exclusion of the presence of additional feature(s), property(ies), element(s), method step(s), etc. The term "comprises", and variations thereof are thus to be understood as being inclusive or open-ended and as not excluding additional, unrecited elements or method steps, etc.

Similarly, the term "*consist of*", or linguistic variations such as "*consisting of*" or "*consists of*", should be understood as excluding any element, step, ingredient, etc. that is not specified.

Further, the term "*consist essentially of*", or linguistic variations such as "*consisting essentially of*" or "*consists essentially of*", should be understood as limiting the scope of what is being discussed to the specified elements, steps, ingredients, etc., and those that do not materially affect the basic and novel characteristic(s) of what is being discussed.

The phrase "*percent sequence identity*," in the context of two or more nucleic acid or polypeptide sequences, refers to two or more sequences or subsequences that have a specified percentage of nucleotides or amino acid residues that are the same, when compared and aligned for maximum correspondence, as measured using one of the sequence comparison algorithms described below (e.g., BLASTP and BLASTN or other algorithms available to persons of ordinary skill in the art). Depending on the application, the "*percent sequence identity*" can exist over a region of the sequence being compared, e.g., over a functional domain, or, alternatively, exist over the full length of the two sequences to be compared. For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, e.g., by the local homology algorithm of Smith & Waterman, Adv. Appl. Math. 2:482 (1981); by the homology alignment algorithm of Needleman & Wunsch, J. Mol. Biol. 48:443 (1970); by the search for similarity method of Pearson & Lipman, Proc. Nat'1. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.).

In the context of the present disclosure, the "*percentage of sequence identity*" between two polypeptides is preferably determined by global alignment. Accordingly, the sequence identity between two amino acid sequences is herein described by the parameter "*percentage of sequence identity*". For purposes of the present application, the "*percentage of sequence identity*" between two amino acid sequences (A) and (B) is determined by comparing the two sequences aligned in an optimal manner, through a window of comparison. Said alignment of sequences can be carried out by well-known methods, for instance, using the algorithm for global alignment of Needleman-Wunsch. Protein analysis software matches similar sequences using measures of similarity assigned to various substitutions, deletions, and other modifications, including conservative amino acid substitutions. Once the total alignment is obtained, the percentage of identity may be obtained by dividing the full number of identical amino acid residues aligned by the full number of residues contained in the longest sequence between the sequences (A) and (B).

Sequence identity is typically determined using sequence analysis software. For comparing two amino acid sequences, one may use, for example, the tool "Emboss needle" for pairwise sequence alignment of proteins provided by EMBL-EBI and available on www.ebi.ac.uk/Tools/services/web/toolform.ebi?tool=emboss_needle&context=protein, using default settings: (i) Matrix: BLOSUM62, (ii) Gap open: 10, (iii) gap extend: 0.5, (iv) output format: pair, (v) end gap penalty: false, (vi) end gap open: 10, (vii) end gap extend: 0.5.

As used herein, the term "*vector*" is any molecule or particle that is used to carry exogenous nucleic acids, such as a polynucleotide, into another cell. The "*vectors*" described herein can be any type of "*vector*" such as a plasmid, a viral vector, or an artificial chromosome, amongst others. The term "*vector*" and the term "*expression vector*" are used interchangeably herein.

As used herein, the term "*Cv6*" refers to the polypeptide of SEQ ID NO:23 wherein X is C, i.e., SEQ ID NO:49.

As used herein, the term "*Av6*" refers to the polypeptide of SEQ ID NO:23 wherein X is A, i.e., SEQ ID NO:50.

As used herein, the term "4K" refers to the polypeptide of SEQ ID NO:46 (which includes a linker of amino acid sequence KKKK).

As used herein, the term "*KKKK*" refers to a linker with an amino acid sequence of KKKK (i.e., SEQ ID NO:32).

### Polypeptide constructs and their manufacture and use

Despite the fact that CeD is a major health concern, there are currently no drugs approved, and the only option available to manage the disease is to maintain a strict, lifelong gluten-free diet, which is clearly insufficient for many patients. As explained above, restoring specific immune tolerance towards gluten, and in particular T-cell tolerance to gliadin, has been proposed as a therapeutic strategy of interest.

However, as of today, there is no such authorized immunotherapeutic treatment on the market in the US or in Europe.

The Nexvax2 immunotherapy vaccine, based on a combination of isolated immunopeptides from gliadin and hordein, failed in phase II for lack of efficacy as compared to placebo. Besides, the vaccine, administered by the subcutaneous route, was shown to result in a dramatic increase in the plasma concentrations of immunopeptides and in an exacerbation of gastro-intestinal symptoms.

Another investigational therapy under development (TAK-101 also called TIMP-GLIA) is based on repeated intravenous administrations of poly(lactide-co-glycolide) nanoparticles loaded with gliadin, said gliadin being partially deamidated by a pre-treatment with acetic acid. By being encapsulated in a nanoparticle, the gliadin is not expected to be recognized by the body's immune system until it reaches the spleen, whereby immune tolerance would be generated through non-inflammatory antigen presentation with low side effects. However, and without wishing to be bound by any theory, it is believed that using natural gliadin treated with acetic acid raises several issues.

The first issue resides in the use of natural gluten as the starting material of gliadin. Gluten (and gliadin obtained from gluten) shows variations in protein composition among wheat (*Triticum aestivum L*.) cultivars. In other words, depending on the cultivars and the conditions of culture, the protein composition of gliadin extracted from the wheat gluten may vary resulting in batch-to-batch composition variability in the final active ingredient.

Another issue relates to the use of acetic acid to trigger deamidation. The immunogenicity of gliadin relies on a few key deamidation sites recognized by tTG. Without wishing to be bound by any theory, it is thought that deamidation should be performed on particular, precise glutamine residues in gliadin (and not necessarily at all glutamine residues) in order to provide an effective immunogenicity. However, using acetic acid to treat gliadin may lead to random deamidation (in terms of glutamine position and/or number) which might impact both the immunogenicity and the efficacy of the final vaccine. Additionally, preparation of acetic acid-treated gliadin can be difficult to standardize, resulting in an active ingredient showing variations from batch-to-batch and thus for which characterization pursuant to regulatory requirements can be a real challenge.

Another drawback of the vaccines described in the prior art lies in the route of administration. Administration by subcutaneous or intravenous route can result in systemic passage of the immunogenic active ingredient which can reach and activate T cells in the intestine such as lamina propria and intraepithelial lymphocytes (IELs), thereby exacerbating gastrointestinal symptoms, as observed in the Nexvax2 trial. Besides, such administrations, in particular the intravenous route, are to be performed by health professionals. This is a real drawback for immunotherapeutic treatments since repeated and frequent administrations of the immunogen are often required to achieve tolerance. Furthermore, because of the pain and/or the stress due to injection, some patients can be reluctant to perform and/or can show poor compliance to treatments based on drug injections. Thus, the development of a treatment which can be easily self-administered, or administered within the family circle, without pain and/or stress can be a major advantage in terms of acceptability, compliance, and cost.

Based upon their expertise in immunotherapy, the Inventors sought to conceive a new immunotherapeutic treatment strategy to induce tolerance to gliadin in subjects suffering from CeD without the drawbacks of the methods disclosed in the prior art. As will be readily understood from the present disclosure, the present polypeptide constructs may be readily and easily prepared to have an essentially uniform protein composition from batch-to-batch, as well as essentially uniform deamidation from batch-to-batch, and thus may be essentially consistent in their immunogenic effect and readily characterized to meet regulatory requirements.

Preliminary studies were performed in mouse models of CeD. These studies showed that epicutaneous administration of pepsin/digested gliadin (PTD-gliadin) increased Tregs in the brachial lymph nodes in sensitized NOD Ab0 HLA-DQ8 mice and decreased intestinal inflammation in NOD Ab0 HLA-DQ8 IL-10^{-/-} mice that spontaneously develop celiac-like disease when put on a gluten-containing chow.

Thus, the epicutaneous route was identified as an administration route of interest to induce specific immune tolerance to gliadin, and more generally against gluten in celiac patients. Without wishing to be bound by any theory, it is believed that repeated applications of immunogenic compounds derived from gliadin on intact skin (e.g., by using a skin patch such as the VIASKIN^{®} patch) can progressively promote specific tolerance to gluten in CeD patients by leveraging the inherent ability of epidermal Langerhans Cells (LCs) to promote antigen-specific tolerance, the role of LCs being not only to carry the antigen to draining lymph nodes but also to transmit important tolerogenic signals that preferentially drive the differentiation of specific Tregs.

However, one major challenge to developing an immunotherapeutic treatment by the epicutaneous route remains, i.e., to conceive an effective immunogenic compound adapted to this particular route of administration. Gliadins are soluble in aqueous alcohols but insoluble in water or neutral salt solutions, due to their high content of glutamine and asparagine residues. The low solubility of gliadin hampers its administration by epicutaneous route in particular by means of a skin patch such as the VIASKIN^{®} patch in which the immunogenic compound is present in dry form and is then solubilized by transepidermal water loss (TEWL) to be delivered in the epidermis and reach the skin DCs. A possible strategy to increase the solubility of gliadins in water is treatment with acetic acid, so as to promote its deamidation. However, as fully explained above, such a method is not satisfactory. Another alternative was to increase solubility by enzymatic digestion with pepsin and trypsin, optionally followed by treatment with tTG to ensure deamidation. However, this enzymatic method shows the same drawbacks as acetic acid treatment: it uses natural gliadin as a starting material, it is difficult to standardize, and it can lead to a high degree of variability in the final active ingredient.

On the other hand, without wishing to be bound by any theory, it is believed that using a cocktail of small immunodominant epitopes, as in the case of the Nexvax2 vaccine, would not be satisfactory in terms of efficacy and safety, even by the epicutaneous route. Indeed, small peptides may directly bind to HLA molecules expressed at the surface of antigen-presenting cells, without being internalized and processed. It is believed that antigen capture and processing would be an important activation factor for antigen-presenting cells such as epidermal LCs, allowing them to acquire tolerogenic properties. Moreover, small peptides are more likely to reach deeper layers of the skin and to diffuse into the bloodstream, which may ultimately lead to the reactivation of gliadin-specific T cells within the gut and the exacerbation of gastrointestinal symptoms of CeD.

After much research, new immunogenic compounds suitable for providing immunotherapy for CeD were conceived. These immunogenic compounds are polypeptide constructs comprising several peptide regions (also called herein "building blocks") integrating key epitopes selected for encompassing the gliadin isoforms (namely, alpha-gliadin, gamma-gliadin, and/or omega-gliadin).

The peptide regions can include deamidation at specific glutamine positions identified as relevant to increase the affinity of the processed peptides to HLA-DQ and increase the solubility of the polypeptide constructs. The building blocks are covalently linked together directly and/or through one or more linkers. The linker(s), if used, can be or include non-peptide linker(s) and/or peptide linker(s) that can contribute to increased solubility in water, the penetration into the epidermis, and/or in the proper processing of the polypeptide constructs, as explained further below.

The immunogenic compounds provided herein show several technical features of interest: they can be fully characterized pursuant to regulatory standards *(since they can be prepared by a well-standardized process such as chemical synthesis or recombinant protein expression*), show specific deamidation at well-defined positions, and have been optimized (i) to possess a high level of solubility in water and in skin perspiration in order to be efficiently solubilized and delivered to the epidermis when used in combination with a VIASKIN^{®}-type patch, and (ii) to be properly internalized, processed, and loaded on MHC-II by LCs and/or skin DCs, promoting an effective activation of Tregs and thus an effective reorientation of the immune response to gliadin to tolerance. The immunogenic compounds provided herein have been designed to avoid the generation of junctional neoepitopes which might impair the immune response.

It is clear that the new immunogenic compounds (the present polypeptide constructs) may have various advantages over immunogenic products prepared by denaturing and/or deamidating natural gliadin as described above. It is also anticipated that the present immunogenic compounds may have improved tolerogenic properties over a mere mixture of small peptides comprising epitopes, as in the case of Nexvax2. For example, because the present polypeptide constructs may be internalized and processed by the LCs and/or DCs before being loaded on MHC class II molecules for presentation to CD4⁺ lymphocytes, it is expected that such processing may trigger key activation signals allowing the LCs and/or DCs to acquire tolerogenic properties. By contrast, small peptides (such as those present in Nexvax2) are directly loaded onto empty surface MHC class II molecules by LCs, DCs and/or other APCs ("antigen-presenting cells"), i.e., without being captured by surface receptors and processed internally and, thus, without triggering said key activation signals.

In addition to an improvement in tolerogenic properties, and without wishing to be bound by any theory, it is expected that the polypeptide constructs provided herein will show a good safety profile by epicutaneous route, as such polypeptide constructs will be mainly delivered into the non-vascularized stratum granulosum of the epidermis, with highly limited systemic diffusion and access to the gliadin-specific T cells located in the gut. Exacerbation of clinical symptoms in CeD patients as observed in the Nexvax2 trial is thus expected to be avoided or reduced.

Accordingly, the present disclosure relates to polypeptide constructs (as more fully described further below) and to their use to increase tolerance to gliadin - and more generally to gluten - in a subject in need thereof and, thus, relates to their use for treating or preventing CeD in a subject in need thereof. In a particular embodiment, the polypeptide is administered by epicutaneous route to a subject in need thereof.

More generally, the present disclosure relates to the use of polypeptide constructs provided herein for modulating a T cell response to gluten. In some embodiments, the polypeptide constructs provided herein modulate a T cell response to gliadin, in particular for inducing immune tolerance to gliadin, for treating CeD, and/or for delaying the onset of CeD or a symptom thereof in a subject who is sensitive to gluten. In some embodiments, modulating a T cell response includes reducing inflammation when the T cells are exposed to gliadin. In some other embodiments, the polypeptide construct provided herein modulates any T cell response. In other embodiments, the polypeptide construct provided herein modulates CD4 T cell responses.

In some embodiments, the present disclosure relates to the use of a polypeptide construct in a subject sensitive to gliadin or more generally to gluten, to modulate the cytokine secretion profile in the subject, e.g., by promoting the secretion of Th2 cytokines while decreasing the expression of Th1 cytokines.

The disclosure provides a method for increasing or inducing tolerance to gliadin, and more generally to gluten, in a subject in need thereof, the method comprising administering to the subject a polypeptide construct provided herein, preferably by the epicutaneous route.

More precisely, the disclosure provides an immunotherapeutic method for increasing or inducing tolerance to gliadin, and more generally to gluten, in a subject suffering from CeD wherein the subject is repeatedly administered a polypeptide construct by the epicutaneous route.

In a particular aspect, the disclosure provides a method for treating or delaying the onset of CeD in a subject which comprises administering to the subject a polypeptide construct provided herein, preferably by epicutaneous route and preferably under repeated administrations. In a particular aspect, the present methods may be used for decreasing the risk of onset of CeD, delaying the progression of CeD, or decreasing the severity of one or several symptoms or manifestations of CeD in the subject, such as intestinal damage.

In another aspect, the disclosure provides the use of a polypeptide construct such as provided herein, for the preparation of a pharmaceutical composition for increasing tolerance to gliadin, more generally to gluten, in a subject in need thereof, wherein the pharmaceutical composition is preferably repeatedly administered by the epicutaneous route. Similarly, the disclosure provides the use of a polypeptide construct such as provided herein, for the preparation of a pharmaceutical composition for treating or preventing CeD in a subject in need thereof, wherein the pharmaceutical composition is preferably repeatedly administered by the epicutaneous route. As fully described below, the pharmaceutical composition is preferably in dry form. The pharmaceutical composition is also preferably delivered by means of a skin patch. The pharmaceutical composition may comprise one or several pharmaceutically acceptable excipients.

In a further aspect, the disclosure provides a skin patch comprising the polypeptide construct provided herein, or comprising the pharmaceutical composition provided herein, said skin patch being adapted to provide the administration of said polypeptide construct or said pharmaceutical composition by epicutaneous route. As described further below, in certain embodiments, the skin patch device comprises a backing, the periphery of said backing being adapted to create with the skin a hermetically closed (occlusive) chamber, wherein the backing bears the polypeptide construct provided herein, in dry form, optionally in admixture with one or several pharmaceutically acceptable excipients, on its skin facing side within the chamber. Similarly, in certain embodiments, the skin patch device comprises a backing, the periphery of said backing being adapted to create with the skin a hermetically closed (occlusive) chamber, wherein the backing bears the pharmaceutical composition provided herein, in dry form, on its skin facing side within the chamber.

### The polypeptide construct

The application herein provides a polypeptide construct suitable for inducing tolerance to gliadin in a subject sensitive to gliadin, and in particular suitable for providing immunotherapy against celiac disease. In a preferred embodiment, the polypeptide construct is administered by epicutaneous route.

In some embodiments, the present polypeptide construct may comprise at least two of the following epitopes: an epitope from alpha-gliadin, an epitope from beta-gliadin, and an epitope from omega-gliadin.

In some embodiments, the polypeptide construct comprises (i) at least one epitope from alpha-gliadin and (ii) at least one epitope from gamma-gliadin.

In some embodiments, the polypeptide construct comprises (i) at least one epitope from alpha-gliadin and (ii) at least one epitope from omega-gliadin.

In some embodiments, the polypeptide construct comprises (i) at least one epitope from gamma-gliadin and (ii) at least one epitope from omega-gliadin.

In some embodiments, the polypeptide construct comprises (i) at least one epitope from alpha-gliadin, (ii) at least one epitope from gamma-gliadin and (iii) optionally at least one epitope from omega-gliadin.

In some embodiments, the polypeptide construct comprises (i) at least one epitope from alpha-gliadin, (ii) at least one epitope from omega-gliadin and (iii) optionally at least one epitope from gamma-gliadin.

In some embodiments, the polypeptide construct comprises (i) at least one epitope from gamma-gliadin, (ii) at least one epitope from omega-gliadin and (iii) optionally at least one epitope from alpha-gliadin.

In some embodiments, including in a preferred embodiment, the polypeptide construct comprises (i) at least one epitope from alpha-gliadin, (ii) at least one epitope from gamma-gliadin and (iii) at least one epitope from omega-gliadin.

In all the above embodiments, each of said at least one epitope from alpha-gliadin, and/or at least one epitope from gamma-gliadin and/or at least one epitope from omega-gliadin can be independently in deamidated form, in a partially deamidated form, or in non-deamidated form. In some embodiments, at least one epitope (such as 1, 2, 3, or all epitopes) from gliadin isoforms present in the polypeptide is/are in a deamidated form or in a partially deamidated form. In some embodiments, said epitopes are independently in non-deamidated form or in at least partially deamidated form.

In some embodiments, the polypeptide construct comprises an amino acid sequence comprising (i) at least one epitope from alpha-gliadin, (ii) at least one epitope from gamma-gliadin, and (iii) at least one epitope from omega-gliadin, and wherein the polypeptide construct is capable of increasing tolerance to gliadin in a subject sensitive to gliadin. Preferably, each epitope present in the polypeptide construct is independently in non-deamidated form or in at least partially deamidated form.

In some embodiments, any one or more of epitopes from alpha-, gamma-, and omega-gliadin binds to at least one haplotype of HLA-DQ.

HLA-DQ haplotypes of interest in the context of celiac disease encompass, without being limited to, HLA-DQ2, HLA-DQ2.5, HLA-DQ2.2, HLA-DQ8, HLA-DQ8.5, HLA-DQ7.5, and HLA-DQ2.3.

In some embodiments, at least one epitope from alpha-, gamma-, and omega-gliadin binds to an HLA-DQ haplotype selected from HLA-DQ8 and HLA-DQ8.5, and at least one epitope from alpha-, gamma-, and omega-gliadin binds to HLA-DQ2.5.

In some embodiments, the polypeptide construct further comprises at least one flanking sequence that is adjacent to any one or more of epitopes from alpha-, gamma-, and omega-gliadin, wherein the at least one flanking sequence is derived from a native gliadin sequence.

In some embodiments, the at least one flanking sequence comprises (a) a first flanking sequence that is 5' to any one or more of epitopes from alpha-, gamma-, and omega-gliadin; and (b) a second flanking sequence that is 3' to any one or more of epitopes from alpha-, gamma-, and omega-gliadin.

In other words, in some embodiments, each of epitopes from alpha-, gamma-, and omega-gliadin are flanked with a flanking sequence at its 3' and 5' terminal. Each flanking sequence may independently have from 1 to 20 amino acid residues in length such as from 1 to 10 or from 1 to 6 amino acid residues in length, or from 1 to 4 amino acid residues in length such as 1, 2, 3, or 4 amino acid residues in length. Each flanking sequence may be independently derived from a native gliadin sequence.

In certain embodiments, each epitope present in the polypeptide construct is flanked with a first and a second flanking region, each comprising from 1 to 6 amino acid residues, preferably 2 or 3 amino acid residues, in length.

In some embodiments, the polypeptide construct comprises one or more linkers as provided herein. The one or more linkers may connect one or more of the epitopes from alpha-, gamma-, and omega-gliadin.

In some embodiments, the polypeptide construct refers to a single, non-naturally occurring molecule comprising epitopes from several gliadin isoforms.

It is disclosed a polypeptide construct may comprise, consist essentially of, or consist of, a polypeptide, or alternatively may include both polypeptide regions and one or more non-polypeptide region(s). The polypeptide or polypeptide regions may comprise, consist essentially of, or consist of, building blocks or epitopes such as provided herein. Any non-polypeptide region(s) can, for example, include a non-polypeptide linker as described in more detail below.

It is disclosed a polypeptide construct which may be or include a linear molecule, a cyclic molecule, or a branched molecule, preferably a linear molecule or a branched molecule.

In some aspects, the polypeptide construct has a molecular weight of at most about 50 kDa, or at most about 40 kDa, preferably at most about 35, 30, 25, or 20 kDa, such as at most about 19, 18, 17, 16, or 15 kDa. The polypeptide construct may comprise at most about 300 amino acid residues, preferably at most about 250 amino acid residues, most preferably at most about 200 amino acid residues, such as at most about 180, at most about 150, or at most 120 amino acid residues. The polypeptide construct may comprise at least about 50 amino acid residues, such as at least about 60 amino acid residues, or at least about 70 amino acid residues. Typically, the polypeptide construct has from 60 to 120 amino acid residues in length.

As used herein, a gliadin epitope in "*non-deamidated form*" refers to an epitope from gliadin in which all of the naturally occurring glutamine residues are present.

As used herein, a gliadin epitope in "*at least partially deamidated form*" refers to a gliadin epitope wherein one or several naturally occurring glutamine residues are replaced by a glutamate residue. In some embodiments, the gliadin epitope can be totally "deamidated" which means that each naturally occurring glutamine residue present in its sequence is replaced by a glutamate residue.

It should be clear from the present disclosure that the present polypeptide constructs are non-naturally occurring, and, as such, any reference to "naturally occurring glutamine" is intended to refer to a particular residue in the present non-naturally occurring polypeptide constructs that corresponds to a residue that would be glutamine in the corresponding epitope from the naturally occurring gliadin isoform of interest.

Among the plurality of gliadin epitopes, a subset of epitopes corresponding to major antigenic determinants for each gliadin isoform of interest were identified. This subset of epitopes is also expected to encompass the majority of patients with CeD or those individuals at increased risk of developing CeD, namely, those with HLA-DQ2.5, HLA-DQ8, and HLA-DQ8.5 haplotypes. These epitopes (also called hereunder "core epitopes") are shown in Table 1:

**Table 1: Core Epitopes**

| **Epitopes from** | **Epitope name** | **SEQUENCE** | **SEQ ID** |
|---|---|---|---|
| α-gliadin | DQ2.5-glia-α1a | PFPQPZLPY | NO:1 |
| | DQ2.5-glia-α1b | PYPQPZLPY | NO:2 |
| | DQ2.5-glia-α2 | PQPZLPYPQ | NO:3 |
| | DQ8-glia-α1 / DQ8.5-glia-α1 | ZGSFQPSQZ | NO:4 |
| γ-gliadin | DQ2.5-glia-γ1 / DQ8-glia-γ2 / DQ8.5-glia-γ1 | PQQSFPZQZ | NO:5 |
| | DQ2.5-glia-γ4c / DQ8-glia-γ1a | ZQPZQPFPZ | NO:6 |
| | DQ2.5-glia-γ5 | QQPFPZQPQ | NO:7 |
| ω-gliadin | DQ2.5-glia-ω1 / DQ2.5-seq-1 / DQ2.5-hor-1 | PFPQPZQPF | NO:8 |
| | DQ2.5-glia-ω2 | PQPZQPFPW | NO:9 |

| | | | |
|---|---|---|---|
| Wherein each Z independently refers to glutamine residue (Q) or glutamate residue (E). | | | |

Accordingly, in some preferred aspects, the at least one epitope from alpha-gliadin, the at least one epitope from gamma-gliadin, and the at least one epitope from omega-gliadin are selected from the sequences shown in Table 1 hereabove. In some other aspects, each of the at least one epitope from alpha-gliadin, the at least one epitope from gamma-gliadin, and the at least one epitope from omega-gliadin corresponds to an amino acid sequence which differs from a sequence shown in Table 1 hereabove by one or two amino acid changes (substitutions, insertions, and/or deletions), preferably one or two amino acid substitutions.

In some aspects, the at least one epitope from α-gliadin comprises any one sequence of SEQ ID Nos: 1, 2, 3, and 4.

In some aspects, the at least one epitope from α-gliadin comprises any two sequences of SEQ ID Nos: 1, 2, 3, and 4.

In some aspects, the at least one epitope from α-gliadin comprises any three sequences of SEQ ID Nos: 1, 2, 3, or 4.

In some aspects, the at least one epitope from α-gliadin comprises all four sequences of SEQ ID Nos: 1, 2, 3, and 4.

In some aspects, the at least one epitope from γ-gliadin comprises any one sequence of SEQ ID Nos: 5, 6, and 7.

In some aspects, the at least one epitope from γ-gliadin comprises any two sequences of SEQ ID Nos: 5, 6, and 7.

In some aspects, the at least one epitope from γ-gliadin comprises all three sequences of SEQ ID Nos: 5, 6, and 7.

In some aspects, the at least one epitope from ω-gliadin comprises any one sequence of SEQ ID Nos: 8 and 9.

In some aspects, the at least one epitope from ω-gliadin comprises both sequences of SEQ ID Nos: 8 and 9.

In some aspects, the polypeptide construct may comprise one or several additional epitopes derived from any isoform of gliadins as described in Sollid et al. Immunogenetics (2020) 72:85-88.

It is disclosed a polypeptide construct which comprises (i) at least one epitope from alpha-gliadin, (ii) at least one epitope from omega-gliadin, and optionally (iii) at least one epitope from gamma-gliadin, each epitope being independently in non-deamidated form or in at least partially deamidated form, wherein:
(i) The at least one epitope from alpha-gliadin is selected from the group consisting of SEQ ID NO:1, a polypeptide which differs from SEQ ID NO:1 by one or two amino acid changes (substitutions, insertions, and/or deletions, preferably substitutions), SEQ ID NO:2, a polypeptide which differs from SEQ ID NO:2 by one or two amino acid changes (substitutions, insertions, and/or deletions, preferably substitutions), SEQ ID NO:3, a polypeptide which differs from SEQ ID NO:3 by one or two amino acid changes (substitutions, insertions, and/or deletions, preferably substitutions), SEQ ID NO:4, a polypeptide which differs from SEQ ID NO:4 by one or two amino acid changes (substitutions, insertions, and/or deletions, preferably substitutions), and combinations thereof,
(ii) The at least one epitope from omega-gliadin is selected from the group consisting of SEQ ID NO:8, a polypeptide which differs from SEQ ID NO:8 by one or two amino acid changes (substitutions, insertions, and/or deletions, preferably substitutions), SEQ ID NO:9, a polypeptide which differs from SEQ ID NO:9 by one or two amino acid changes (substitutions, insertions, and/or deletions, preferably substitutions), and combinations thereof, and
(iii) The optional at least one epitope from gamma-gliadin is selected from the group consisting of SEQ ID NO:5, a polypeptide which differs from SEQ ID NO:5 by one or two amino acid changes (substitutions, insertions, and/or deletions, preferably substitutions), SEQ ID NO:6, a polypeptide which differs from SEQ ID NO:6 by one or two amino acid changes (substitutions, insertions, and/or deletions, preferably substitutions), SEQ ID NO:7, a polypeptide which differs from SEQ ID NO:7 by one or two amino acid changes (substitutions, insertions, and/or deletions, preferably substitutions), and combinations thereof.

In some aspects, the polypeptide construct comprises (i) at least one epitope from alpha-gliadin, (ii) at least one epitope from omega-gliadin, and optionally (iii) at least one epitope from gamma-gliadin, each epitope being independently in non-deamidated form or in at least partially deamidated form, wherein:
(i) The at least one epitope from alpha-gliadin is selected from polypeptides having at least seven amino acid residues, or at least eight amino acid residues, in common with a sequence selected from the group consisting of SEQ ID NO:1, SEQ ID NO:2, SEQ ID NO:3, SEQ ID NO:4, and combinations thereof,
(ii) The at least one epitope from omega-gliadin is selected from polypeptides having at least seven amino acid residues, or at least eight amino acid residues, in common with a sequence selected from the group consisting of SEQ ID NO:8, SEQ ID NO:9, and combinations thereof, and
(iii) The optional at least one epitope from gamma-gliadin is selected from polypeptides having at least seven amino acid residues, or at least eight amino acid residues, in common with a sequence selected from the group consisting of SEQ ID NO:5, SEQ ID NO:6, SEQ ID NO:7, and combinations thereof.

In some aspects, the polypeptide construct comprises (i) at least one epitope from alpha-gliadin, (ii) at least one epitope from omega-gliadin, and optionally (iii) at least one epitope from gamma-gliadin, each epitope being independently in non-deamidated form or in at least partially deamidated form, wherein:
(i) The at least one epitope from alpha-gliadin is selected from the group consisting of SEQ ID NO:1, SEQ ID NO:2, SEQ ID NO:3, SEQ ID NO:4, and combinations thereof,
(ii) The at least one epitope from omega-gliadin is selected from the group consisting of SEQ ID NO:8, SEQ ID NO:9, and combinations thereof, and
(iii) The optional at least one epitope from gamma-gliadin is selected from the group consisting of SEQ ID NO:5, SEQ ID NO:6, SEQ ID NO:7, and combinations thereof.

In preferred aspects, the polypeptide construct comprises (i) at least one epitope from alpha-gliadin, (ii) at least one epitope from omega-gliadin, and (iii) at least one epitope from gamma-gliadin, each as described above. The at least one epitope from each of alpha-, omega-, and gamma-gliadin are preferably included in one or several peptidic fragments in the polypeptide construct (called herein building blocks), said building blocks being directly linked and/or linked via one or more linkers to form the polypeptide construct.

It is disclosed that the linker may be or include any chemical chain (e.g., hydrocarbon chain) to connect the building blocks together. The length and the chemical nature of the linker may be optimized to promote appropriate processing of the building blocks by the LCs and/or DCs, and/or to facilitate the synthesis of the polypeptide construct, and/or improve the solubility of the final polypeptide construct.

In some aspects of the disclosure, the linker is a linear or a branched moiety which can comprise one or more heteroatoms such as O, S, and N, and/or one or more cyclic moieties such as C₃₋₁₄cycloalkyl, C₃₋₁₄cycloalkenyl, aryl groups, heteroaryl groups, heterocycle groups, and/or heterocycloalkyl groups, such as heterocyclylC₁₋₄alkyl groups. Examples and definitions of each of these moieties/groups may be found at, e.g., p. 125, line 1 - p. 127, line 18 and p. 129, lines 30-31 of WO 2017/100726 A1 (published on June 15, 2017, titled "Methods for Treating Huntington's Disease").

The linker may comprise up to 500 carbon atoms, preferably from 2 to 200 carbon atoms, such as from 5 to 100 carbon atoms or from 5 to 50 carbon atoms or from 5 to 30 atom carbons. The linker(s) can be or can include a peptide moiety or a non-peptide moiety.

Examples of linkers include, without being limited, homopolymers, copolymers and block polymers, polypeptides, and saturated or unsaturated hydrocarbon chains, said saturated or unsaturated hydrocarbon chains being optionally interrupted by one or several heteroatoms or heteroatomic groups (e.g., S, O, Se, P, -C(O)-, -NHC(O)-, -OC(O)-, -N(R)-with R being H or C₁-C₃ alkyl), and/or by one or several cyclic or heterocyclic moieties (as defined above), and/or optionally having a heteroatom or heteroatomic group (such as S, O, -C(O)-, -NHC(O)-, -OC(O)-, -N(R)- with R being H or C₁-C₃ alkyl, -O-N(R)- with R being H or C₁-C₃ alkyl, or -N(C₁-C₃ alkoxy)-) at at least one of its extremities, and/or optionally being substituted by one or several substituents (e.g., hydroxyl, halogens, C₁-C₃ alkoxy, - CN, -CF₃, or C₁-C₃ alkyl), and combinations thereof.

As used herein, "*combinations*" means that the linker may comprise several hydrocarbon chains, oligomer chains, or polymeric chains (e.g., 2, 3, 4, 5 or 6) linked (or connected) by any appropriate group, such as -O-, -S-, -N(R)- with R being H or C₁-C₃ alkyl, -C(O)-, -NHC(O)-, -OC(O)-, -C(O)-O-C(O)-, -NH-CO-NH-, -O-CO-NH-, -NH-(CS)-NH-, -NH-CS-, phosphodiester groups, phosphorothioate groups, cyclic groups, or heterocyclic groups (as defined above).

Typically, the group(s) (also called connectors) used to link the several hydrocarbon chains, oligomer chains, or polymeric chains together result from the reactions used to connect these different chains together. For instance, the connector may be -NHC(O)- in case of amide coupling reaction, "N" in case of reductive amination, or a triazole derivative in case of click chemistry involving the reaction of an azido with an alkyne group.

In some aspects, the linker may be selected from the group consisting of a polyether such as polyethylene glycol (PEG) or polypropylene glycol; polyvinyl alcohol (PVA); a polyester such as polylactate, polyacrylate, polymethacrylate, or polysilicon ester; a polyamide such as polycaprolactone or poly(N-(2-hydroxypropyl)methacrylamide) (pHPMA); poly(D,L-lactic-co-glycolic acid) (PLGA); polymers of alkyl diamines; a hydrocarbon chain which may be unsaturated or saturated, branched or unbranched, and may optionally have an heteroatom such as O, N, and S, on at least one end; and combinations thereof.

As used herein, alkyl diamine refers to -NH₂-(CH₂)ᵣ-NH₂- wherein r is an integer from 1 to 20, for instance from 2 to 10, such as 2, 3, 4, and 5. A polymer of alkyl diamines (also known as polyamines) refers to a compound of formula -NH₂-[(CH₂)ᵣ-NH]ₜ- with r being as defined above and t is an integer of at least 2, for example of at least 3, 4, 5, 10, or more.

In a particular aspects, the linker is selected from the group consisting of: a polypeptide of 2 to 40 amino acids in length, preferably from 2 to 20 amino acids in length; a linear or branched C₂-C₂₀ alkylene chain; polyethylene glycol; polypropylene glycol; pHPMA; PLGA; a polymer of diamino alkyl; and combinations thereof. Preferably said polyethylene glycol, polypropylene glycol, PLGA, pHPMA or polymer of diamino alkyl comprise from 2 to 40 monomers, preferably from 2 to 10 or from 10 to 20 monomers.

In some aspects, the linker is, or includes a hydrocarbon chain (for instance, an alkyl chain) having from 2 to 100 carbon atoms, or from 2 to 40 carbon atoms, or from 2 to 30 carbon atoms, or from 2 to 20 carbon atoms, optionally interrupted by:
- one or more heteroatoms or heteroatomic groups selected from -O-, -S-, -C(O)-, -NHC(O)-, -OC(O)-, -C(O)-O-C(O)-, -N(R)- with R being H or a C₁-C₃ alkyl, -NH-CO-NH-, -O-CO-NH-, -NH-(CS)-NH-, and -NH-CS-; and/or
- C₅-C₂₀ carbocyclic moieties such as cycloalkyl, cycloalkenyl, or aromatic groups (as defined above); and/or
- one or more heterocyclic moieties having 5 to 20 ring atoms, such as heterocycloalkyls or heteroaryls having 5 to 20 ring atoms;
and optionally having at at least one of its extremities, a heteroatom or heteroatomic group selected from -O-, -S-, -N(R)- with R being H or C₁-C₃ alkyl, -O-N(R)- with R being H or C₁-C₃ alkyl, -N(C₁-C₃ alkoxy), -C(O)-, -NHC(O)-, -OC(O)-, -C(O)-O-C(O)-, -NH-, -NH-CO-NH-, -O-CO-NH-, -NH-(CS)-NH-, and -NH-CS-.

The heterocycle (or "heterocyclic moiety") may be, for instance, a triazolyl or a triazolyl fused with another cyclic group which can result from the click reaction between an azido and an alkyne.

In someaspects, the linker is branched and comprises three or more branches. In some embodiments, the linker may be a branched polypeptide comprising three or more branches. For instance, the linker may be a tri-antennary linker, such as a tri-antennary polypeptide linker.

In some other aspects, the linker may be a dendrimer. For instance, the polypeptide construct may be or include a dendrimer decorated by one or several copies (e.g., 2, 3, or 4 copies) of the at least one epitope from each of alpha-, gamma-, and omega gliadin, or the building blocks as described herein.

The linker(s) present in the polypeptide construct of the inventions are peptide linkers, in particular linear peptide linkers. It is disclosed peptide linkers which have from 2 to 20, in particular from 2 to 10 (such as from 2 to 6) amino acids in length. Peptide linkers can comprise naturally occurring amino acids, non-naturally occurring amino acids, modified amino acids, and the D and L stereoisomers of each. In preferred aspects, the amino acid residues present in the peptide linker(s) are selected from glycine (G), proline (P), arginine (R), lysine (K), cysteine (C), tyrosine (Y), and alanine (A). In particular, the one or several peptide linkers present in the polypeptide construct comprise at least one KK moiety. Examples of such linkers include KKKK (SEQ ID NO:32), KK, GPRRKK (SEQ ID NO:33), GPKK (SEQ ID NO:34), GARRKK (SEQ ID NO:35), KGKK (SEQ ID NO:45), AYY, RR, and GCRRKK (SEQ ID NO:36).

In some embodiments, the polypeptide construct is for epicutaneous administration. In such a case, the polypeptide construct has preferably an isoelectric point ("pI") above 7.0, such as from 8.0 to 10.0, e.g., from 9.0 to 10.0. Accordingly, the linkers can be selected so that the pI of the polypeptide construct is above 7.0.

In some aspects of the disclosure in addition to the above-listed epitopes, the polypeptide construct provided herein can comprise additional epitopes such as those of SEQ NO: 13 (ZGSVQPQZL) wherein each Z is independently Q or E.

Preferably, among epitopes from each of alpha-, gamma-, and omega-gliadin, at least one epitope reacts with HLA-DQ8 and/or HLA-DQ8.5. In some further embodiments, among the epitopes from each of alpha-, gamma-, and omega-gliadin, at least one epitope reacts with HLA-DQ8 and/or HLA-DQ8.5, and at least one epitope reacts with HLA-DQ2 and/or HLA-DQ2.5.

Accordingly, in a preferred aspect, the polypeptide construct comprises at least one epitope selected from SEQ ID NO:4, SEQ ID NO:5, and SEQ ID NO:6, preferably an epitope of SEQ ID NO:6.

In a particular aspect, the epitope specific for HLA-DQ8 and/or DQ8.5 is selected from SEQ ID NO:4, PQQSFPEQE (SEQ ID NO:10), EQPEQPFPE (SEQ ID NO:12), and combinations thereof.

In a preferred aspect, the polypeptide construct comprises an epitope of SEQ ID NO:4. Indeed, epitope of SEQ ID NO:4 cross-reacts with both HLA-DQ8 and HLA-DQ8.5 haplotypes. Thus, the presence of said epitope in the polypeptide construct can allow the DQ8 patient subset to be addressed as well.

In addition to epitope of SEQ ID NO:4, the polypeptide construct may also comprise the epitope of SEQ ID NO:5 from gamma-gliadin isoform. Depending on its deamidation status, the specificity of the epitope can shift from HLA-DQ2.5 haplotype to HLA-DQ8 and HLA-DQ8.5 haplotypes. Without wishing to be bound by any theory, it is believed that an epitope of SEQ ID NO:5 with the two Z present in the sequences being E (namely, the epitope of SEQ ID NO:10), would effectively bind both HLA-DQ8/8.5 and HLA-DQ2.5 haplotypes.

Alternatively, in addition to epitope of SEQ ID NO:4, the polypeptide construct may also comprise the epitope of SEQ ID NO:6 from gamma-gliadin isoform. Depending on its deamidation status, the specificity of the epitope can shift from HLA-DQ2.5 haplotype to HLA-DQ8. Without wishing to be bound by any theory, it is believed that an epitope of SEQ ID NO:12 would effectively bind HLA-DQ8 haplotypes.

In an addition to epitopes of SEQ ID NO:4 and SEQ ID NO:5 or in addition to epitopes of SEQ ID NO:4 and SEQ ID NO:6, the polypeptide construct provided herein may also comprise at least one epitope selected from SEQ ID NO:1, SEQ ID NO:2, and SEQ ID NO:3 (which correspond to major antigenic determinant from alpha-gliadin) and at least one epitope selected from SEQ ID NO:8 and SEQ ID NO:9 (which correspond to major determinants from omega-gliadin). Of note, in their deamidated state (namely Z is E), said epitope comprises the QPEQPFP (SEQ ID NO:11) moiety, which is recognized by serum gluten-specific antibodies. Without wishing to be bound by any theory, it is believed that the presence of QPEQPFP (SEQ ID NO:11) moiety within the polypeptide construct may promote the formation of an immune complex between the polypeptide construct and the preexisting anti-gluten antibodies present in skin, such immune complex promoting the addressing of the polypeptide construct to skin LCs and/or DCs. Accordingly, in some aspect, the polypeptide construct provided herein comprises an epitope of SEQ ID NO:8 and/or SEQ ID NO:9, in which Z is E.

In a particular aspect, the polypeptide construct provided herein comprises:
(i) at least one epitope from alpha-gliadin selected from the group consisting of SEQ ID NO:1, SEQ ID NO:2, SEQ ID NO:3, and combinations thereof,
(ii) at least one epitope from alpha-gliadin specific for HLA-DQ8, preferably selected from epitope of SEQ ID NO:4, preferably an epitope of SEQ ID NO: 29, and combinations thereof,
(iii) at least one epitope from gamma-gliadin selected from peptides of SEQ ID NO:6, and combinations thereof, preferably an epitope of SEQ ID NO: 12, and
(iv) at least one epitope from omega-gliadin selected from the group consisting of SEQ ID NO:8, SEQ ID NO:9, and combinations thereof. Preferably, Z present in SEQ ID NO:8 and in SEQ ID NO:9 is E.

In a further particular aspect, the polypeptide construct provided herein comprises:
(i) at least two epitopes from alpha-gliadin selected from the group consisting of SEQ ID NO:1, SEQ ID NO:2, and SEQ ID NO:3,
(ii) an epitope from alpha-gliadin specific for HLA-DQ8, which is an epitope of SEQ ID NO:4,
(iii) an epitope from gamma-gliadin which is an epitope of SEQ ID NO:6, and
(iv) an epitope from omega-gliadin which is an epitope of SEQ ID NO:8 and/or SEQ ID NO:9.

Said epitopes can be in any order and can be directly linked to one another, or can be linked to one another via one or more linkers, or a combination of direct linkage and linkage via linker. As mentioned above, the linkers are preferably peptide linkers comprising from 2 to 20, such as from 2 to 15 or 2 to 10, amino acids in length, as described herein. In some embodiments, each peptide linker in the polypeptide construct comprises at least one KK moiety.

It is disclosed a polypeptide that can comprise one, two, or three copies of any one of epitopes of SEQ ID NO:1-4, 6, 8, and 9. In some aspects, the polypeptide construct comprises one copy of each epitope.

In an additional aspect, the polypeptide construct is such that it comprises the said epitopes in non-deamidated form, which means that all "Z" present in the above listed sequences are glutamines (Q).

IThe polypeptide construct of the invention is such that all the "Z" present in the above listed sequences are glutamates (E).

In other aspects of the disclosure, a subset of the Z residues present in the sequence of the building blocks is E, and the remaining Z residues present in the sequence of the building blocks are Q.

The epitopes of SEQ ID NO:1-4, 6, 8, and 9 are present in deamidated form (all Z = E) in the polypeptide construct of the invention.

In a particular aspect, the polypeptide construct can further comprise one or several additional epitopes from gliadin isoforms, e.g., as described in Sollid et al. Immunogenetics (2020) 72:85-88.

In certain aspects, said one or several additional epitopes can be selected from the group consisting of epitopes of SEQ ID NO:5, SEQ ID NO:7 (epitopes from γ-gliadin), SEQ ID NO: 13 (epitope from α-gliadin specific to HLA-DQ2.2), and combination thereof.

The polypeptide construct of the invention comprises an epitope of SEQ ID NO:1, an epitope of SEQ ID NO:2, and an epitope of SEQ ID NO:3. It is disclosed that the polypeptide construct can comprise a single copy or several copies (for instance, 2 copies) of one or several of these epitopes. The epitopes of SEQ ID NO: 1, SEQ ID NO:2, and SEQ ID NO:3 can be present in isolated regions in the polypeptide construct, wherein "isolated" means not adjacent or overlapping. Alternatively, as they contain common amino acid residues, the sequences of these epitopes can overlap.

In some aspects of the disclosure, the polypeptide construct comprises a polypeptide having at least about 40%, at least about 50%, at least about 60%, at least about 70%, at least about 80%, at least about 85%, at least about 90%, or at least about 95% sequence identity with SEQ ID NO: 14 (PFPQPZLPYPQPZLPYPQPZLPYPQ), wherein Z is either E or Q.

In a further aspect, the polypeptide construct comprises a polypeptide of SEQ ID NO:14, or a polypeptide which differs from SEQ ID NO:14 by one, two, three, four, or five amino acid changes (substitutions, insertions, and/or deletions, preferably substitutions).

The polypeptide construct of the invention comprises a polypeptide of SEQ ID NO: 14, wherein the amino acid residues Z are E.

The polypeptide construct of the invention comprises an epitope of SEQ ID NO:8 and an epitope of SEQ ID NO:9.

It is disclosed that the polypeptide construct can comprise a single copy or several copies (for instance, 2 copies) of one or both of these epitopes. The epitopes of SEQ ID NO:8 and SEQ ID NO:9 can be present in isolated regions in the polypeptide construct, wherein "isolated" means not adjacent or overlapping. Alternatively, as the epitopes of SEQ ID NO:8 and SEQ ID NO:9 share common amino acid residues, the sequences of these epitopes can overlap. Accordingly, in certain aspects, the polypeptide construct comprises a polypeptide of SEQ ID NO:28, namely PFPQPZQPFPW, or which differs from SEQ ID NO:28 by virtue of 1 or 2 amino acid changes (substitutions, insertions, and/or deletions, preferably substitutions). In some embodiments, Z is E in SEQ ID NO:28.

In a particular aspect, the polypeptide construct provided herein comprises:
(i) a polypeptide of SEQ ID NO: 14,
(ii) at least one epitope from alpha-gliadin specific for HLA-DQ8, preferably selected from peptides of SEQ ID NO:4,
(iii) at least one epitope from gamma-gliadin selected from peptides of SEQ ID NO:6, and
(iv) a polypeptide of SEQ ID NO:28.

In certain aspects, the polypeptide constructs provided herein may comprise the polypeptide moieties shown below in Table 2, said polypeptide moieties being preferably connected linearly by means of linkers such as peptide linkers, in any order:

### Table 2: Polypeptide Moieties containing core epitopes.

| **Epitopes from** | **POLYPEPTIDE MOIETIES (SEQUENCE)** | **SEQ ID** |
|---|---|---|
| α-gliadin | PFPQPZLPYPQPZLPYPQPZLPYPQ | NO:14 |
| | ZGSFQPSQZ | NO:4 |
| γ-gliadin | ZQPZQPFPZ | NO:6 |
| ω-gliadin | PFPQPZQPFPW | NO:28 |

In a particular aspect, the polypeptide construct provided herein can further comprise one or several additional epitopes selected from the group consisting of SEQ ID NO:5, SEQ ID NO:7, SEQ ID NO:13, and combinations thereof.

The polypeptide construct of the invention comprises:
(i) a polypeptide comprising epitopes from alpha-gliadin, said polypeptide being of SEQ ID NO:14, wherein all Z are E,
(ii) an epitope from alpha-gliadin specific for HLA-DQ8 of SEQ ID NO:4, wherein all Z are E (namely EGSFQPSQE (SEQ ID NO:29)),
(iii) an epitope from gamma-gliadin of SEQ ID NO:6, preferably of SEQ ID NO:12, and
(iv) a polypeptide comprising epitopes from omega-gliadin of SEQ ID NO:28, wherein Z is E (namely PFPQPEQPFPW (SEQ ID NO:30)).

It is disclosed that said epitopes and polypeptides can be present in any order in the polypeptide construct. As mentioned above, said polypeptide and epitopes present in the polypeptide construct are preferably linked through linkers (e.g., peptide linkers) as described herein.

In a particular aspect of the disclosure, the polypeptide construct can further comprise one or several additional epitopes selected from the group consisting of SEQ ID NO:5, SEQ ID NO:7, SEQ ID NO:13, and combinations thereof.

The polypeptide of SEQ NO:14 was identified as the major antigenic determinant of alpha-gliadin specific to the HLA-DQ2.5 haplotype. SEQ ID NO:14 is advantageous in that it comprises a central sequence made of overlapping epitopes of SEQ ID NO:1, SEQ ID NO:2, and SEQ ID NO:3. SEQ ID NO:14 corresponds to the central region from position 5 to 29 of the 33-mer polypeptide of SEQ ID NO:31.

The amino acids from position 1 to 4 and 30 to 33 in the 33-mer of SEQ ID NO:31 correspond to flanking residues from the native alpha-gliadin residues. Without wishing to be bound by any theory, it is believed that these flanking regions can increase the affinity of the core epitope for HLA-DQ following processing of the polypeptide construct by APCs, thus improving the presentation of the core epitope to T cells.

In a more general aspect and without wishing to be bound by any theory, it is believed that incorporating flanking amino acid residues from the native gliadin sequences around each core epitope of interest present in the polypeptide construct can increase their affinity for HLA-DQ and improve their presentation to T cells. The flanking region may have from 1 to 10, preferably from 1 to 6, amino acids in length, and more preferably from 1 to 3 amino acids in length (such as 2 or 3 amino acids in length). Indeed, without wishing to be bound by any theory, it is believed that flanking regions of 2 or 3 amino acid residues in length and derived from native gliadin isoform sequences may improve the presentation of the core epitope to T cells while avoiding or limiting the generation of junctional neoepitopes.

The core epitopes with flanking amino acid residues correspond to the so-called building blocks provided herein.

Non-limiting examples of building blocks of interest that may be used in the present polypeptide construct are shown in Table 3 below:

**Table 3: Exemplary Building Blocks**

| **isoform** | **N° building block** | **Sequence** | **SEQ ID NO:** | **Core Epitopes present** |
|---|---|---|---|---|
| α-gliadin | BB1 | QLQPFPQPZLPYPQPZLPYPQPZLPYPQPQP | 15 | SEQ ID NO:1, SEQ ID NO:2, and SEQ ID NO:3 |
| γ-gliadin | BB2a | | 16 | SEQ ID NO:6 |
| | BB2b | PQPZQPZQPFPZQPQ QQFPQPZQPZQPFPZQPQQQF | 17 | |
| ω-gliadin | BB3a | PQQPFPQPZQPFPWQPQ PQQPQQPFPQPZQPFPWQPQQPF | 20 | SEQ ID NO:8 and SEQ ID NO:9 |
| | BB3b | | 21 | |
| α-gliadin | BB4a | PSGZGSFQPSQZNPQ QQYPSGZGSFQPSQZNPQAQG | 18 | SEQ ID NO:4 |
| | BB4b | | 19 | |

Accordingly, in a particular aspect, the polypeptide construct comprises at least one building block such as at least one, two, three, or four building blocks selected from the group consisting of polypeptides having at least about 40%, at least about 50%, at least about 60%, at least about 70%, at least about 80%, at least about 85%, at least about 90%, or at least about 95% sequence identity with a building block shown in Table 3.

In a particular aspect, it is disclosed a polypeptide construct comprising, consisting of, or consisting essentially of: (i) at least one building block comprising an epitope from alpha-gliadin, (ii) at least one building block comprising an epitope from gamma-gliadin, and (iii) at least one building block comprising an epitope from omega-gliadin, each epitope being independently in non-deamidated form or in at least partially deamidated form.

In some aspects:
(i) The at least one building block comprising an epitope from alpha-gliadin is selected from the group consisting of: polypeptides having at least about 40%, at least about 50%, at least about 60%, at least about 70%, at least about 80%, at least about 85%, at least about 90%, or at least about 95% sequence identity with SEQ ID NO:15; polypeptides having at least about 40%, at least about 50%, at least about 60%, at least about 70%, at least about 80%, at least about 85%, at least about 90%, or at least about 95% sequence identity with SEQ ID NO:18; polypeptides having at least about 40%, at least about 50%, at least about 60%, at least about 70%, at least about 80%, at least about 85%, at least about 90%, or at least about 95% sequence identity with SEQ ID NO:19; and combinations thereof,
(ii) The at least one building block comprising an epitope from gamma-gliadin is selected from the group consisting of: polypeptides having at least about 40%, at least about 50%, at least about 60%, at least about 70%, at least about 80%, at least about 85%, at least about 90%, or at least about 95% sequence identity with SEQ ID NO:16; polypeptides having at least about 40%, at least about 50%, at least about 60%, at least about 70%, at least about 80%, at least about 85%, at least about 90%, or at least about 95% sequence identity with SEQ ID NO:17; and combinations thereof, and
(iii) The at least one building block comprising an epitope from omega-gliadin is selected from the group consisting of: polypeptides having at least about 40%, at least about 50%, at least about 60%, at least about 70%, at least about 80%, at least about 85%, at least about 90%, or at least about 95% sequence identity with SEQ ID NO:20; polypeptides having at least about 40%, at least about 50%, at least about 60%, at least about 70%, at least about 80%, at least about 85%, at least about 90%, or at least about 95% sequence identity with SEQ ID NO:21; and combinations thereof.

As mentioned, in each building block, each "Z" residue independently refers to E or Q.

In some aspects, one or several building blocks present in the polypeptide construct may differ from a building block shown in Table 3 by one, two, three, or four amino acid changes (substitutions, insertions, and/or deletions, preferably substitutions). The amino acid changes (substitutions, insertions, and/or deletions, preferably substitutions) preferably occur in the flanking regions.

In some other aspects, the polypeptide construct comprises, consists essentially of, or consists of: (i) at least one building block comprising an epitope from alpha-gliadin, (ii) at least one building block comprising an epitope from gamma-gliadin, and (iii) at least one building block comprising an epitope from omega-gliadin, each epitope being independently in non-deamidated form or in at least partially deamidated form, wherein:
(i) The at least one building block comprising an epitope from alpha-gliadin is selected from the group consisting of SEQ ID NO:15, SEQ ID NO:18, SEQ ID NO:19, and combinations thereof,
(ii) The at least one building block comprising an epitope of from gamma-gliadin is selected from the group consisting of SEQ ID NO:16, SEQ ID NO:17, and combinations thereof, and
(iii) The at least one building block comprising an epitope from omega-gliadin is selected from the group consisting of SEQ ID NO:20; SEQ ID NO:21, and combinations thereof.

Without wishing to be bound by any theory, it is believed that the presence of a building block BB1 of SEQ ID NO:15, which derives from 33-mer alpha-gliadin, and which can be either in non-deamidated or in at least partially deamidated form, can play an important role to reorientate the immune response, as this building block recapitulates the immunodominant peptides from this gliadin isoform. Thus, in preferred embodiments, the polypeptide construct provided herein comprises the BB1 of SEQ ID NO:15. Without wishing to be bound by any theory, it is believed that the building block BB1 of SEQ ID NO:15 represents a particularly useful building block derived from the 33-mer alpha-gliadin of SEQ ID NO:31 for avoiding or limiting the generation of neoepitopes.

Furthermore, as explained above, without wishing to be bound by any theory, it is believed that the presence of epitopes of SEQ ID NO:4, SEQ ID NO:6, and SEQ ID NO:8 and SEQ ID NO:9 may encompass antigenic determinants of interest from alpha-gliadin, gamma-gliadin, and omega-gliadin, respectively, while addressing the most frequent haplotypes found in celiac patients.

In a particular aspect, it is disclosed a polypeptide construct which comprises at least one, at least two, at least three, or at least four building blocks, preferably different building blocks, i.e., BB1, BB2, BB3, and BB4, in which:
- BB1 is the polypeptide of at least about 40%, at least about 50%, at least about 60%, at least about 70%, at least about 80%, at least about 85%, at least about 90%, or at least about 95% sequence identity with SEQ ID NO:15,
- BB2 is selected from the polypeptide of at least about 40%, at least about 50%, at least about 60%, at least about 70%, at least about 80%, at least about 85%, at least about 90%, or at least about 95% sequence identity with SEQ ID NO:16 (BB2a) or at least about 40%, at least about 50%, at least about 60%, at least about 70%, at least about 80%, at least about 85%, at least about 90%, or at least about 95% sequence identity with SEQ ID NO:17 (BB2b),
- BB3 is selected from the polypeptide of at least about 40%, at least about 50%, at least about 60%, at least about 70%, at least about 80%, at least about 85%, at least about 90%, or at least about 95% sequence identity with SEQ ID NO:20 (BB3a) or at least about 40%, at least about 50%, at least about 60%, at least about 70%, at least about 80%, at least about 85%, at least about 90%, or at least about 95% sequence identity with SEQ ID NO:21 (BB3b), and
- BB4 is selected from the polypeptide of at least about 40%, at least about 50%, at least about 60%, at least about 70%, at least about 80%, at least about 85%, at least about 90%, or at least about 95% sequence identity with SEQ ID NO:18 (BB4a) or at least about 40%, at least about 50%, at least about 60%, at least about 70%, at least about 80%, at least about 85%, at least about 90%, or at least about 95% sequence identity with SEQ ID NO:19 (BB4b).

In some aspects, each at least one building block comprising an epitope of interest in the polypeptide construct differs from a building block shown in Table 3 by one, two, three, or four amino acid changes (substitutions, insertions, and/or deletions, preferably substitutions). The amino acid changes (substitutions, insertions, and/or deletions, preferably substitutions) preferably occur in the flanking regions.

In some aspects, the polypeptide construct comprises one of the following combinations of building blocks:
- Combination 1: BB1, BB2 is BB2a, BB3 is BB3a, BB4 is BB4a;
- Combination 2: BB1, BB2 is BB2b, BB3 is BB3b, BB4 is BB4b;
- Combination 3: BB1, BB2 is BB2a, BB3 is BB3a, BB4 is BB4b;
- Combination 4: BB1, BB2 is BB2a, BB3 is BB3b, BB4 is BB4a;
- Combination 5: BB1, BB2 is BB2b, BB3 is BB3a, BB4 is BB4a;
- Combination 6: BB1, BB2 is BB2b, BB3 is BB3b, BB4 is BB4a;
- Combination 7: BB1, BB2 is BB2b, BB3 is BB3a, BB4 is BB4b;
- Combination 8: BB1, BB2 is BB2a, BB3 is BB3b, BB4 is BB4b;
- Combination 9: BB1, BB2 is BB2a or BB2b, BB4 is BB4a or BB4b, BB3 is BB3a or BB3b;
- Combination 10: BB1, BB3 is BB3a or BB3b, BB2 is BB2a or BB2b, BB4 is BB4a or BB4b;
- Combination 11: BB1, BB3 is BB3a or BB3b, BB4 is BB4a or BB4b, BB2 is BB2a or BB2b;
- Combination 12: BB1, BB4 is BB4a or BB4b, BB2 is BB2a or BB2b, BB3 is BB3a or BB3b;
- Combination 13: BB1, BB4 is BB4a or BB4b, BB3 is BB3a or BB3b, BB2 is BB2a or BB2b;
- Combination 14: BB2 is BB2a or BB2b, BB1, BB3 is BB3a or BB3b, BB4 is BB4a or BB4b;
- Combination 15: BB2 is BB2a or BB2b, BB3 is BB3a or BB3b, BB1, BB4 is BB4a or BB4b;
- Combination 16: BB2 is BB2a or BB2b, BB1, BB4 is BB4a or BB4b, BB3 is BB3a or BB3b;
- Combination 17: BB2 is BB2a or BB2b, BB4 is BB4a or BB4b, BB1, BB3 is BB3a or BB3b;
- Combination 18: BB2 is BB2a or BB2b, BB3 is BB3a or BB3b, BB4 is BB4a or BB4b, BB1;
- Combination 19: BB2 is BB2a or BB2b, BB4 is BB4a or BB4b, B3 is BB3a or BB3b, BB1;
- Combination 20: BB3 is BB3a or BB3b, BB1, BB2 is BB2a or BB2b, BB4 is BB4a or BB4b;
- Combination 21: BB3 is BB3a or BB3b, BB2 is BB2a or BB2b, BB1, BB4 is BB4a or BB4b;
- Combination 22: BB3 is BB3a or BB3b, BB1, BB4 is BB4a or BB4b, BB2 is BB2a or BB2b;
- Combination 23: BB3 is BB3a or BB3b, BB2 is BB2a or BB2b, BB4 is BB4a or BB4b, BB1;
- Combination 24: BB3 is BB3a or BB3b, BB4 is BB4a or BB4b, BB1, BB2 is BB2a or BB2b;
- Combination 25: BB3 is BB3a or BB3b, BB4 is BB4a or BB4b, BB2 is BB2a or BB2b, BB1;
- Combination 26: BB4 is BB4a or BB4b, BB3 is BB3a or BB3b, BB1, BB2 is BB2a or BB2b;
- Combination 27: BB4 is BB4a or BB4b, BB3 is BB3a or BB3b, BB2 is BB2a or BB2b, BB1;
- Combination 28: BB4 is BB4a or BB4b, BB2 is BB2a or BB2b, BB1, BB3 is BB3a or BB3b;
- Combination 29: BB4 is BB4a or BB4b, BB2 is BB2a or BB2b, BB3 is BB3a or BB3b, BB1;
- Combination 30: BB4 is BB4a or BB4b, BB1, BB2 is BB2a or BB2b, BB3 is BB3a or BB3b; and
- Combination 31: BB4 is BB4a or BB4b, BB1, BB3 is BB3a or BB3b, BB2 is BB2a or BB2b.

Preferred combinations herein are Combination 1 or Combination 2, most preferably Combination 1.

In some aspects, the construct comprises one or more of the same building blocks:
- Combination 32: BB1, BB1, BB1, BB1;
- Combination 33: BB2a, BB2a, BB2a, BB2a;
- Combination 34: BB2b, BB2b, BB2b, BB2b;
- Combination 35: BB3a, BB3a, BB3a, BB3a;
- Combination 36: BB4a, BB4a, BB4a, BB4a;
- Combination 37: BB1, BB2a, BB2a, BB1;
- Combination 38: BB1, BB3a, BB3a, BB1;
- Combination 39: BB1, BB2a, BB2a, BB4;
- Combination 40: BB1, BB3a, BB3a, BB4;
- Combination 41: BB4a, BB4b, BB4a, BB4b; and
- Combination 42: BB3a, BB3a, BB2a, BB2a.

As will be understood, in addition to the above combinations, the specific building blocks (BB1, BB2a, BB2b, BB3a, BB3b, BB4a, BB4b) can be in any combination of four building blocks, and each and every one of the combinations is encompassed by the present invention.

In some aspects, the construct comprises two building blocks, which may be either the same or different building blocks:
- Combination 43: BB1, BB2a;
- Combination 44: BB1, BB3a;
- Combination 45: BB2a, BB4a;
- Combination 46: BB3a, BB4a;
- Combination 47: BB3a, BB3a; and
- Combination 48: BB3a, BB4b.

As will be understood, in addition to the above combinations, the specific building blocks (BB1, BB2a, BB2b, BB3a, BB3b, BB4a, BB4b) can be in any combination of two building blocks, and each and every one of the combinations is encompassed by the present invention.

In some aspects, the construct comprises three building blocks, which may be either the same or different building blocks:
- Combination 49: BB1, BB2a, BB3a;
- Combination 50: BB4a, BB2a, BB3a;
- Combination 51: BB1, BB4a, BB3a;
- Combination 52: BB2a, BB2a, BB2a;
- Combination 53: BB2a, BB2b, BB2a;
- Combination 54: BB4b, BB2a, BB3b; and
- Combination 55: BB4a, BB2a, BB3b.

As will be understood, in addition to the above combinations, the specific building blocks (BB1, BB2a, BB2b, BB3a, BB3b, BB4a, BB4b) can be in any combination of three building blocks, and each and every one of the combinations is encompassed by the present invention.

The building blocks BB1, BB2, BB3, and BB4 can be linked together by any means, and in any order. In some embodiments, the building blocks are not linked together but are instead mixed together. In some embodiments, some building blocks are linked together and other building locks are not linked together, with both the linked and unlinked building blocks mixed together.

The building blocks can be directly linked to each other and/or via one or more linkers which can be or include peptide moieties or non-peptide moieties, e.g., as described herein.

The building blocks can be linearly connected directly or by the means of one or more linkers so as to form a linear polypeptide construct.

In some other aspects, the building blocks may be connected via a branched linker comprising two or more branches, such as a bi-, tri-, or tetra-antennary linker.

For example, one can use the platform described in Forner et al., J. Org. Chem. 2020, 85, 1626-1634,.

In certain aspects, the linker(s) present in the polypeptide construct is/are peptide linkers, in particular linear peptide linkers. Preferred peptide linkers are from 2 to 20 amino acids in length, in particular from 2 to 10 amino acids in length, such as from 2 to 6 amino acids in length. Peptide linkers can comprise naturally occurring amino acids, non-naturally occurring amino acids, modified amino acids, and the D and L stereoisomers of each. For instance, the amino acid residues present in the peptide linker(s) may be selected from glycine (G), proline (P), arginine (R), lysine (K), cysteine (C), tyrosine (Y), and alanine (A).

In some aspects, the linker is a cleavable linker.

As used herein, "a cleavable linker" refers to any linker between the building blocks that promotes or enables the release of the building blocks by cleavage (e.g., by endopeptidases, proteases, or by any other means that may occur in antigen-presenting cells ["APC"]), and preferably during processing by APCs. Typically, such a cleavable linker can comprise a cleavable moiety, e.g., of 2 to 6 amino acids in length (e.g., 2, 3, 4, or 5 amino acids in length). The cleavable moiety can be selected from the group consisting of RR, RVRR (SEQ ID NO:37), GAGA (SEQ ID NO:38), AGAG (SEQ ID NO:39), KGKG (SEQ ID NO:40), GPRRKK (SEQ ID NO:33), GPKK (SEQ ID NO:34), GARRKK (SEQ ID NO:35), AYY, GCRRKK (SEQ ID NO:36) valine-citrulline, VR, VK, VA, FR, K, KK, KKKK (SEQ ID NO:32), and GPGPG (SEQ ID NO:41). For instance, the linker may comprise or consist of an amino acid sequence selected from KKKK (SEQ ID NO:32), KK, GPRRKK (SEQ ID NO:33), GPKK (SEQ ID NO:34), GARRKK (SEQ ID NO:35), AYY, RR, and GCRRKK (SEQ ID NO:36).

When the polypeptide construct is typically for administration by epicutaneous route, e.g., by means of a skin patch with a condensation chamber such as the VIASKIN^{®} patch, the linkers can be selected so that the isoelectric point (pI) of the polypeptide construct is above 7.0, preferably from 8.0 to 10.0. Without wishing to be bound by any theory, such pI would render the polypeptide construct soluble in lightly acidic aqueous medium such as perspiration.

In some embodiments, the polypeptide construct of the invention bears C-terminal amidation and/or N-terminal acetylation.

In some aspects, the linkers are of formula:

[CC1]ₐ₋[SP]_{b}-[CC2]_{c}

wherein a, b, and c are independently 0 or 1, CC1 and CC2 are cleavable moieties (preferably as defined above), and SP is a spacer group as described elsewhere herein. For example, SP can be a non-peptide spacer, or it can be a peptide spacer, e.g., comprising from 2 to 20 amino acids in length, for example from 2 to 10 amino acids in length, such as from 2 to 6 amino acids in length.

In some preferred aspects, each building block is linked to peptide moieties which are cathepsin cleavage sites (such as KK, AYY, RR, or GPGPG (SEQ ID No:41)) on at least one of the building block's extremities. Without wishing to be bound by any theory, it is believed that the presence of said peptide moieties which are cathepsin cleavage sites may promote a correct processing of the polypeptide construct into epitopes of interest to be presented in DCs, while avoiding the generation of junctional neoepitopes which might occur in case of juxtaposition of the C-terminal extremity of one building block to the N terminal extremity of the vicinal building block.

In some aspects, the polypeptide construct is linear. In such a case, the polypeptide construct provided herein may comprise, consist essentially of, or consist of, a moiety of formula (I):

[BBw]-[L1]ₙ₁-[BBx]-[L2]ₙ₂-[BBy]-[L3]ₙ₃-[BBz] (I)

wherein:
- -BBw, BBx, Bby, and BBz are building blocks selected from BB1, BB2, BB3, and BB4 as defined above, with the proviso that each building block is different and that the polypeptide construct comprises at least one BB1, one BB2, one BB3, and one BB4;
- n1, n2, and n3 are independently 0 or 1;
- each L1, L2, and L3 is a linker, each linker being of the following formula (II):

   -[CC1]ₐ-[SP1]_{b}-[CC2]_{c}- (II)

   wherein:
   - each a, b, and c is independently 0 or 1;
   - CC1 and CC2 are independently selected from peptides comprising from 2 to 10 amino acid in length, preferably selected from G, P, A, R, K, and Y; and
   - SP1 is a spacer group.

The spacer group may be any chemical chain (e.g., hydrocarbon chain). The length and the chemical nature of the spacer may be selected to facilitate appropriate processing of the building blocks, and/or facilitate the synthesis of the polypeptide construct, and/or improve the solubility of the final polypeptide construct.

For example, the spacer may be or include: i) a linear moiety which can comprise one or more heteroatoms, and/or ii) one or more cyclic moieties such as cycloalkyl, cycloalkenyl, aryl groups, or heterocyclic moieties (such as heterocycloalkyl or heteroaryl). In some embodiments, the spacer may comprise up to 500 carbon atoms, preferably from 2 to 200 carbon atoms, more preferably from 5 to 100 carbon atoms, and even more preferably from 5 to 50 carbon atoms; in some embodiments, the spacer may comprise from 5 to 30 atom carbons or from 5 to 20 carbon atoms. The spacer can be or include a peptide moiety or a non-peptide moiety. The spacer may, in some embodiments, be selected from the group consisting of polymers (including - but not limited to - homopolymers, copolymers and block polymers), peptides, and saturated or unsaturated hydrocarbon chains, wherein the hydrocarbon chains are optionally interrupted by one or several heteroatoms or heteroatomic groups (e.g., S, O, Se, P, -C(O)-, -NHC(O)-, -OC(O)-, and -N(R)- with R being H or C₁-C₃ alkyl), and/or by one or several cyclic or heterocyclic moieties, and/or optionally having a heteroatom or heteroatomic group (such as S, O, -C(O)-, -NHC(O)-, -OC(O)-, -N(R)- with R being H or C₁-C₃ alkyl, -O-N(R)- with R being H or C₁-C₃ alkyl, or -N(C₁-C₃ alkoxy)-) at at least one of its extremity, and/or optionally being substituted by one or several substituents (e.g., hydroxyl, halogens, C₁-C₃ alkoxy, -CN, -CF₃, or C₁-C₃ alkyl), and combinations thereof.

As used herein, *"combinations"* means that the spacer may comprise several hydrocarbon chains, oligomer chains, or polymeric chains (e.g. 2, 3, 4, 5, or 6 chains) linked (or connected) by any appropriate group, such as -O-, -S-, -N(R)- with R being H or C₁-C₃ alkyl, -C(O)-, -NHC(O)-, -OC(O)-, -C(O)-O-C(O)-, -NH-CO-NH-, -O-CO-NH-, -NH-(CS)-NH-, -NH-CS-, phosphodiester groups, or phosphorothioate groups as well as cyclic or heterocyclic groups.

Typically, the group(s) (also called connectors) used to link the several hydrocarbon chains, oligomer chains, or polymeric chains together result from the reactions used to connect these different chains together. For instance, the connector may be -NHC(O)- in case of amide coupling reaction, "N" in case of reductive amination, or a triazole derivative in case of click chemistry involving the reaction of an azido with an alkyne group.

In some embodiments, the spacer may be selected from the group consisting of polyethers such as polyethylene glycol (PEG) and polypropylene glycol; polyvinyl alcohol (PVA); polyesters such as polylactate, polyacrylate, polymethacrylate, and polysilicon ester; polyamide such as polycaprolactone and poly(N-(2-hydroxypropyl)methacrylamide) (pHPMA); poly(D,L-lactic-co-glycolic acid) (PLGA); polymers of alkyl diamines; unsaturated or saturated, branched or unbranched, hydrocarbon chains optionally having a heteroatom such as O, NH, and S on at least one end; and combinations thereof.

In a particular aspect, the spacer is selected from the group consisting of a polypeptide comprising from 2 to 40 amino acids in length; a polypeptide comprising from 2 to 20 amino acids in length; a polypeptide comprising from 2 to 10 amino acid residues in length; linear or branched C₂-C₂₀ alkylene chains; polyethylene glycol; polypropylene glycol; pHPMA; PLGA; polymers of diamino alkyl; and combinations thereof. Preferably, said polyethylene glycol, polypropylene glycol, PLGA, pHPMA, and polymer of alkyl diamines comprise from 2 to 40 monomers, preferably from 2 to 10 monomers or from 10 to 20 monomers.

In some aspects, the spacer is a hydrocarbon chain (for instance, an alkyl chain) having from 2 to 100 carbon atoms, 2 to 40 carbon atoms, 2 to 30 carbon atoms, or 2 to 20 carbon atoms, optionally interrupted by:
- one or more heteroatoms or heteroatomic groups selected from -O-, -S-, -C(O)-, -NHC(O)-, -OC(O)-, -C(O)-O-C(O)-, -N(R)- with R being H or a C₁-C₃ alkyl, -NH-CO-NH-, -O-CO-NH-, NH-(CS)-NH-, and -NH-CS-; and/or
- C₅-C₂₀ carbocyclic moieties, such as (but not limited to) cycloalkyl, cycloalkenyl, or aromatic groups (as defined above); and/or
- one or more heterocyclic moieties having 5 to 20 ring atoms, such as (but not limited to) heterocycloalkyls or heteroaryls having 5 to 20 ring atoms (as defined above);
and optionally having at at least one of its extremities, a heteroatom or heteroatomic group selected from -O-, -S-, -N(R)- with R being H or C₁-C₃ alkyl, -O-N(R)- with R being H or C₁-C₃ alkyl, -N(C₁-C₃ alkoxy), -C(O)-, -NHC(O)-, -OC(O)-, -C(O)-O-C(O)-, -NH-, -NH-CO-NH-, -O-CO-NH-, NH-(CS)-NH-, and NH-CS-.

The heterocycle (or "heterocyclic moiety") may be, for instance, a triazolyl or a triazolyl fused with another cycle which can result from the click reaction between an azido and an alkyne.

L1, L2, and L3 can be the same or can be different. In some embodiments, L1, L2, and L3 are the same.

In someaspects, each b and c present in L1, L2, and L3 is 0. Accordingly, it is disclosed a polypeptide construct pwhich comprises, consists essentially of, or consists of, formula (Ia):

[BBw]-[CCw]ₐ₁-[BBx]-[CCx]ₐ₂-[BBy]-[CCy]ₐ₃-[BBz] (Ia)

wherein:
- a1, a2, and a3 are independently 0 or 1,
- CCw, CCx, and CCy, are independently a peptide comprising a cathepsin cleavage site and comprising from 2 to 20 amino acid residues in length, or from 2 to 10 amino acid residues in length such as from 2 to 6 amino acid residues in length.

In certain aspects, each CCw, CCx, and CCy comprise a cathepsin cleavage site selected from KK, AYY, RR, and GPGPG).

In some aspect, each CCw, CCx, and CCy comprises at least one KK moiety.

In additional or alternative aspects, at least one (preferably most or all) of the amino acid residues present in CCw, CCx, and CCy is selected from G, P, A, C, Y, R, and K.

In the polypeptide construct of the invention, each CCw, CCx, and CCy independently comprises, consists essentially of, or consists of, a peptide comprising a cathepsin cleavage site, selected from the group consisting of KKKK (SEQ ID NO:32), KK, GPRRKK (SEQ ID NO:33), GPKK (SEQ ID NO:34), GARRKK (SEQ ID NO:35), KGKK (SEQ ID NO:45), AYY, RR, and GCRRKK (SEQ ID NO:36), in particular from the group consisting of GPRRKK, GPKK, GARRKK, and GCRRKK.

It is disclosed a polypeptide construct whichcomprises, consists essentially of, or consists of, a molecule of formula (I), in particular a molecule of formula (Ia), each as defined above:

[BBw]-[L1]n1-[BBx]-[L2]n2-[BBy]-[L3]n3-[BBz] (I)

[BBw]-[CCw]a1-[BBx]-[CCx]a2-[BBy]-[CCy]a3-[BBz] (Ia)

wherein:
- BBw, BBx, BBy, and BBz are each building blocks independently selected from BB1, BB2a, BB3a, and BB4a as defined above, with the proviso that each building block is different from each other and that the polypeptide construct comprises at least one BB1, at least one BB2a, at least one BB3a, and at least one BB4a.

Without wishing to be bound by any theory, it is believed that positioning BB1 in the first position may limit the formation of junctional neoepitopes. Thus, in some embodiments, BBw is BB1 of SEQ ID NO:15.

In some other aspects, the polypeptide construct provided herein comprises, consists essentially of, or consists of, a molecule of formula (I), in particular a molecule of formula (Ia), wherein: BBw is BB1, BBx is BB2a, BBy is BB3a, and BBz is BB4a. In the polypeptide construct of the invention all the Z residues present in the sequence of the building blocks are E. In other aspects, all the Z residues present in the sequence of the building blocks are Q.

In other aspects, a subset of the Z residues present in the sequence of the building blocks is E, and the remaining Z residues present in the sequence of the building blocks are Q.

Polypeptide constructs appropriate for delivery by epicutaneous route (EPIT) while exposing appropriate epitopes for inducing immune gluten tolerance and avoiding or limiting the generation of neoepitopes by cell processing were conceived.
Polypeptide constructs soluble and stable in aqueous medium at pH 4-6, which corresponds to the pH of skin perspiration, were also conceived.

In some aspects, the polypeptide construct comprises the building blocks as shown in Table 4 hereunder, or comprises building blocks which differ from those shown in Table 4 by virtue of one or two amino acid change(s) (substitutions, insertions, and/or deletions):

**Table 4: building blocks.**

| **Isoform** | **N° building block** | **Sequence** | **SEQ ID NO:** | **Core Epitopes present*:** |
|---|---|---|---|---|
| α-gliadin | BB1 | QLQPFPQPZLPYPQPZLPYPQPZLPYPQPQP | 15 | SEQ ID NO:1-3 |
| γ-gliadin | BB2a | PQPZQPZQPFPZQPQ | 16 | SEQ ID NO:6 |
| ω-gliadin | BB3a | PQQPFPQPZQPFPWQPQ | 20 | SEQ ID NO:8 and SEQ ID NO:9 |
| α-gliadin | BB4a | PSGZGSFQPSQZNPQ | 18 | SEQ ID NO:4 |

The polypeptide construct of the invention comprises the building blocks BB1, BB2a, BB3a, and BB4a wherein Z refers to E or Q. In the polypeptide construct of the invention , all Z residues refer to E.

It is disclosed that BB1, BB2a, BB3a, and BB4a can be connected to each other in any order, either directly or by means of linkers, or a combination thereof.

It is disclosed a polypeptide construct is of formula (Ib):

[BBw]-[CCw]-[BBx]-[CCx]-[BBy]-[CCy]-[BBz] (Ib)

wherein:
- BBw, BBx; BBy, and BBz are selected from the group consisting of BB1, BB2a, BB3a, and BB4a,
- Each CCw, CCx, and CCy is independently a peptide comprising a cathepsin cleavage site and comprising from 2 to 20 amino acid residues in length, preferably from 2 to 10 amino acid residues in length, more preferably from 2 to 6 amino acid residues in length.

In some aspects, each CCw, CCx, and CCy comprises at least one KK moiety. In additional or alternative embodiments, the amino acid residues present in CCw, CCx, and CCy are selected from G, P, A, C, Y, R, and K.

In the polypeptide construct of the invention, each CCw, CCx, and CCy is independently selected from the group consisting of KKKK (SEQ ID NO:32), KK, GPRRKK (SEQ ID NO:33), GPKK (SEQ ID NO:34), GARRKK (SEQ ID NO:35), KGKK (SEQ ID NO:45), AYY, RR, and GCRRKK (SEQ ID NO:36), in particular GPRRKK, GPKK, GARRKK, and GCRRKK.

Without wishing to be bound by any theory, it is believed that linkers GPRRKK (SEQ ID NO:33), GPKK (SEQ ID NO:34), GARRKK (SEQ ID NO:35), and GCRRKK (SEQ ID NO:36) would be those enabling an increase in the solubility of the polypeptide construct in perspiration while avoiding or limiting the generation of neoepitopes.

In a particular embodiment, the polypeptide construct of the invention may further comprise a modification at the C- or N-terminal extremity so as to improve the solubility of the polypeptide construct. In a particular embodiment, the polypeptide construct comprises amidation at its C-terminal end (namely -CONH₂) and/or an acetylation at its N-terminal end (namely CH₃CONH-). In addition, or alternatively, the polypeptide construct may also comprise one or several additional amino acids selected from lysine and arginine at the C- and/or N-terminal ends.

In the context of the invention, BBw is BB1, BBx is BB2a, BBy is BB3a, and BBz is BB4a.

The polypeptide construct of the invention is of formula (Ic):

[BB1]-[CCw]-[BB2a]-[CCx]-[BB3a]-[CCy]-[BB4a] (Ic)

wherein CCw, CCx, and CCy are as defined in formula (Ib).

The Z residues in BB1, BB2a, BB3a, and BB4a are all E.

In a particular embodiment, the polypeptide construct of the invention is of formula (Ic) wherein:
- CCw is GPRRKK (SEQ ID NO:33),
- CCx is GPRRKK (SEQ ID NO:33), GCRRKK (SEQ ID NO:36), or GARRKK (SEQ ID NO:35), and
- CCy is GPKK (SEQ ID NO:34).

In a particular aspect, it is disclosed a polypeptide construct which comprises, consists essentially of, or consists of, an amino acid sequence having at least about 40%, at least about 50%, at least about 60%, at least about 70%, or at least about 80%, preferably at least about 85%, at least about 90%, or at least about 95% sequence identity with SEQ ID NO:22: 
QLQPFPQPZLPYPQPZLPYPQPZLPYPQPQPGPRRKKPQPZQPZQPFPZQPQGXRRK
KPQQPFPQPZQPFPWQPQGPKKPSGZGSFQPSQZNPQ (SEQ ID NO:22)
 wherein X is either P, C, or A. In some aspects, the polypeptide construct comprises, consists essentially of, or consists of, an amino acid sequence that is a variant of SEQ ID NO:22.

In some aspects, the variant comprises any one or more of an amino acid change (substitutions, insertions, and/or deletions) to the amino acid sequence set forth in SEQ ID NO:22. In some embodiments, the variant comprises 1, 2, 3, 4, or 5 amino acid changes (substitutions, insertions, and/or deletions) to the amino acid sequence set forth in SEQ ID NO:22.

In a further aspect the polypeptide construct comprises, consists essentially of, or consists of, an amino acid sequence having at least about 40%, at least about 50%, at least about 60%, at least about 70%, at least about 80%, preferably at least about 85%, at least about 90%, or at least about 95% sequence identity with SEQ ID NO:23: 
QLQPFPQPELPYPQPELPYPQPELPYPQPQPGPRRKKPQPEQPEQPFPEQPQGXRRK
KPQQPFPQPEQPFPWQPQGPKKPSGEGSFQPSQENPQ (SEQ ID NO:23)
 wherein X is either P, C, or A.

In some aspects, the polypeptide construct comprises, consists essentially of, or consists of, an amino acid sequence that is a variant of SEQ ID NO:23. In some embodiments, the variant comprises any one or more amino acid change(s) (substitutions, insertions, and/or deletions) to the amino acid sequence set forth in SEQ ID NO:23. In some embodiments, the variant comprises 1, 2, 3, 4, or 5 amino acid change(s) (substitutions, insertions, and/or deletions) to the amino acid sequence set forth in SEQ ID NO:23.

In a further aspects, the polypeptide construct comprises, consists essentially of, or consists of, an amino acid sequence of SEQ ID NO:22, in particular of SEQ ID NO:23, or an amino acid sequence which differs from SEQ ID NO:22, in particular from SEQ ID NO:23, by virtue of 1, 2, 3, 4, or 5 amino acid changes(s) (substitutions, insertions, and/or deletions).

For instance, the polypeptide construct may differ from SEQ ID NO:22 or SEQ ID NO:23 by virtue of 1, 2, 3, 4, or 5 amino acid additions, preferably at the N- and/or C-terminal ends, e.g., by virtue of 1, 2, 3, 4, or 5 additions of residues selected from K and R at the C- and/or N-terminal ends.

As indicated above, the polypeptide construct of the invention may bear amidation at the C-terminal end and/or acetylation or a pyroglutamate residue at the N-terminal end.

In some preferred embodiments, the polypeptide construct bears amidation at the C-terminal end and acetylation at the N-terminal end.

In some additional or other embodiments, the polypeptide construct bears 1, 2, or 3 amino acid residues selected from K and R at C- or N-terminal ends.

When present, the amino acid change(s) (substitutions, insertions, and/or deletions) preferably occur within the flanking regions, within the linkers, and/or at N- or C-terminal end(s). In other words, the polypeptide construct preferably has an amino acid sequence in which the following regions are present (conserved):
(i) a polypeptide of SEQ ID NO:14 wherein all Z are E,
(ii) an epitope from alpha-gliadin of SEQ ID NO:4, preferably wherein all Z are E (namely SEQ ID NO:29),
(iii) an epitope from gamma-gliadin of SEQ ID NO:6, preferably from SEQ ID NO:12, and
(iv) an epitope from omega-gliadin of SEQ ID NO:28, preferably wherein Z is E (namely SEQ ID NO:30).

In some embodiments, the C-terminal amino acid residue in the polypeptide construct is amidated (namely bears -CONH₂ instead of -COOH) and the N-terminal amino acid residue in the polypeptide construct is acetylated (namely -CH₃CONH- instead of -NH₂).

In other embodiments, the C-terminal amino acid residue in the polypeptide construct is amidated (namely bears -CONH₂ instead of -COOH) and the N-terminal amino acid residue in the polypeptide construct is a pyroglutamate (pE).

The polypeptide construct as disclosed herein preferably is at most 180, preferably at most 150, at most 140, at most 130, at most 120, at most 110, or at most 100, amino acid residues in length.

Of note, the polypeptide construct comprising, consisting essentially of, or consisting of, SEQ ID NO:22 or 23 is expected to be soluble and stable at pH 4-6 and thus appropriate for an administration through epicutaneous route, in particular by using the VIASKIN^{®} patch as a mean of delivery. Besides, it is expected that the cell processing of said polypeptide construct does not release significant junctional epitopes.

It was further showed that such polypeptide constructs can be synthesized with high yield and purity.

In alternative aspects, it is disclosed a polypeptide construct which comprises, consists essentially of, or consists of, an amino acid sequence having at least about 40%, at least about 50%, at least about 60%, at least about 70%, at least about 80%, preferably at least about 85%, at least about 90%, or at least about 95% sequence identity with an amino acid sequence selected from SEQ ID NO:24, SEQ ID NO:25, SEQ ID NO:26, SEQ ID NO:27, SEQ ID NO:42, SEQ ID NO:43, and SEQ ID NO:44, preferably from SEQ ID NO:24, SEQ ID NO:25, SEQ ID NO:26, and SEQ ID NO:27.

The polypeptide construct may comprise, consist essentially of, or consist of, i) an amino acid sequence selected from SEQ ID NO:24, SEQ ID NO:25, SEQ ID NO:26, SEQ ID NO:27, SEQ ID NO:42, SEQ ID NO:43, and SEQ ID NO:44, or ii) an amino acid sequence which differs from any of SEQ ID NO:24, SEQ ID NO:25, SEQ ID NO:26, SEQ ID NO:27, SEQ ID NO:42, SEQ ID NO:43 and SEQ ID NO:44 by virtue of 1, 2, 3, 4, or 5 amino acid change(s) (substitutions, insertions, and/or deletions).

The amino acid change(s) (substitutions, insertions, and/or deletions) preferably occur within the flanking regions or within the linkers. In other words, the polypeptide construct preferably has an amino acid sequence in which the following regions are present (conserved):
(i) a polypeptide of SEQ ID NO:14 wherein all Z are E,
(ii) an epitope from alpha-gliadin of SEQ ID NO:4, preferably wherein all Z are E (namely SEQ ID NO:29),
(iii) an epitope from gamma-gliadin of SEQ ID NO:6, preferably from SEQ ID NO:12, and
(iv) an epitope from omega-gliadin of SEQ ID NO:28, preferably wherein Z is E (namely SEQ ID NO:30).

In some embodiments, the C-terminal amino acid residue in the polypeptide construct is amidated (namely bears -CONH2 instead of -COOH) and the N-terminal amino acid residue in the polypeptide construct is acetylated (namely CH₃CONH- instead of -NH₂)

In other embodiments, the C-terminal amino acid residue in the polypeptide construct is amidated (namely bears -CONH₂ instead of -COOH) and the N-terminal amino acid residue in the polypeptide construct is a pyroglutamate (pE).

The polypeptide construct as disclosed herein preferably is at most 180, preferably at most 150, 140, 130, 120, 110, or 100 amino acid residues in length.

As mentioned above, the polypeptide construct is preferably for epicutaneous administration. Accordingly, the polypeptide construct has preferably an isoelectric point above 7.0, such as from 8.0 to 10.0, e.g., from 9.0 to 10.0. Accordingly, the linkers and/or C- or N-terminal modifications can be selected so that the pI of the polypeptide construct is above 7.0.

Any of the polypeptide constructs as described herein can comprise one or more non-naturally occurring amino acid residues, and/or amino acids with modification, preferably a chemical modification. The modification may provide a chemical moiety (typically by substitution of a hydrogen, for example, of a C-H bond), such as an amino, acetyl, acyl, carboxy, hydroxy, or halogen (for example, fluorine) group, or a carbohydrate group. Typically, the modification is present on a terminal amino acid residue of the polypeptide construct, in particular on the N- or C-terminal. The chemical modification can be made to improve resistance to degradation or optimise solubility properties or otherwise improve bioavailability by the epicutaneous route of the polypeptide construct. Such modifications include N-amidation e.g., with an acetal or a pyroglutamate group on an N-terminal, or amidation on a C-terminal. As another alternative, the polypeptide construct may be PEGylated at one of its extremities or fused or conjugated to an adjuvant or to another chemical or biological entity (e.g., a chemical drug or a peptide moiety) enabling an increase in its solubility, and/or the penetration through the epidermis, and/or allowing its specific targeting to skin dendritic cell (e.g., by enabling the formation of an immune complex with anti-gliadin antibodies in the skin).

The polypeptide construct provided herein can be prepared by any suitable manner known by the skilled artisan including chemical synthesis, recombinant production, and/or enzymatic synthesis, or any suitable combination thereof.

For example, the polypeptide construct can be synthetically produced. The polypeptide construct may be synthesised by standard chemistry techniques, including synthesis by automated procedure using a commercially available peptide synthesiser. In general, the polypeptide construct may be prepared by standard peptide synthesis such as solid-phase peptide synthesis methodology. Solid-phase peptide synthesis methodology typically involves the successive coupling and deprotection of protected amino acid residues to a solid support such as a 4-methylbenzhydrylamine resin or chloromethyl resin. The formation of an amide bond (also known as a peptide bond) between two successive amino acids can be performed in standard conditions, in the presence of coupling agents such as carbodiimides (e.g., dicyclohexylcarbodiimide (DCC), diisopropylcarbodiimide (DIC), or 1-Ethyl-3-(3-dimethylaminopropyl)carbodiimide (EDC)), optionally in the presence of agents such as (hydroxybenzotriazole (HOBt) and 1-Hydroxy-7-azabenzotriazole (HOAt)). The protecting groups typically used in solid phase peptide synthesis are generally the so-called Boc/benzyl and Fmoc/tert.butyl approaches. After the last residue has been attached, the protected peptide-resin is treated with an appropriate reagent to cleave the peptide from the resin, as well as deprotect the side chain functional groups. The resulting crude product can be further purified by standard techniques such as gel filtration, high-pressure liquid chromatography (HPLC), partition chromatography, ion-exchange chromatography, size-exclusion chromatography, affinity chromatography, and/or recrystallization.

When the polypeptide construct comprises several building blocks, each building block can be prepared separately, e.g., by solid-phase peptide synthesis or recombinantly produced, and then assembled to form the polypeptide construct by a chemical or enzymatic way. In other words, the polypeptide construct provided herein can be prepared by convergent strategy.

For instance, a "chemical ligation" strategy may be used. Chemical ligation refers to a set of techniques used for creating long peptide or protein chains. It is the second step of a convergent approach. First, smaller peptides containing, e.g., 20-70 amino acids are prepared by conventional chemical peptide synthesis and then ligated, optionally after partial or complete deprotection, by means of chemo-selective reaction to give a unique reaction product, usually in aqueous solution. With several coupling steps, proteins of up to 200-300 amino acids can be produced by such a strategy. Chemical ligation can be performed, e.g., by Staudinger ligation, Ser/Thr ligation, α-Ketoacid-Hydroxylamine (KAHA) Amide-Forming Ligation or Native Chemical Ligation (NLC) (*based on the formation of a thioester-linked intermediate that can spontaneously rearrange in amide ('peptide') bond at the ligation site -* Dawson et al., Science, 1994, 266, 776-779*)* and methodology variants of NLC.

For a review about NLC and extended or variant NLC strategies, one can refer to Agouridas et al., Chem. Rev. 2019, 119, 12, 7328-7443 and Kulkarni et al, 2018, Nature Reviews Chemistry, Vol. 2, article number 0122.

One can also use bioconjugation reactions which refers to reactions between amino acids such as lysine, cysteine, or tyrosine with reactive groups as detailed in Koniev, O., Wagner, A, Chem. Soc. Rev., 44, 5495 (2015).

For instance, the functional moiety may comprise a maleimide group or a squarane moiety, which can react with cysteine or tyrosine residues, respectively. Bioconjugation reactions are for instance depicted in the below table:

| | |
|---|---|
| a) Amine conjugation | c) Thiol conjugation |
| 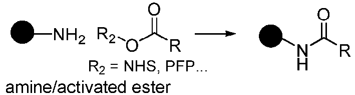 | 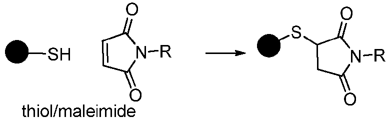 |
| 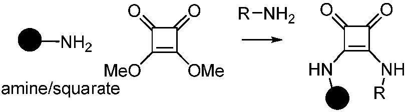 | 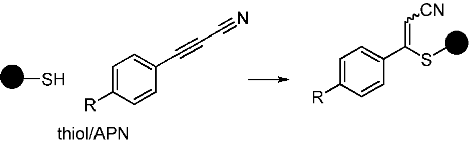 |
| b) Bioconjugation via carbon-nitrogen double bonds. | 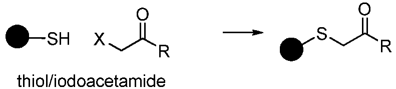 |
| 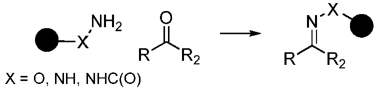 | |

The assemblage of the building blocks, in particular when the final polypeptide construct comprises one or several non-peptide linkers, can be also performed by implementing click chemistry reactions. "Click-reaction" or "Click-chemistry" is a concept introduced by Sharpless in 2001. "Click chemistry" generally refers to chemical reactions characterized by high yields and high chemoselectivity, which are simple to conduct, and which generate inoffensive by-products. "Click reactions" can be typically conducted in complex media with high efficiency. Click reactions are typically used to create covalent heteroatom links (C-X-C) between two entities of interest. For a review of click chemistry, one can refer to Kolb et al., Angew. Chem. Int. Ed. 2001, 40, 2004-2021 and to Rudolf et al., Current Opinion in Chemical Biology, 2013, 17:110-117. Examples of click reactions encompass, without being limited to, copper-catalyzed azide-alkyne dipolar cycloadditions (CuAAC), strain-promoted alkyne-azide cycloaddition (SPAAC), Diels-Alder reactions with tetrazines and strained alkynes or alkenes, tetrazine-isonitrile cycloadditions, thiol-alkene click reactions such as maleimide-cysteine cycloadditions, Staudinger azide-triarylphosphine conjugation, and sydnone-alkyne cycloadditions. In some aspects, the click reaction may be "bioorthogonal" or "biocompatible", meaning that the reagents involved in the click reaction may react selectively and rapidly with each other without side reactions with other entities. For instance, biocompatible or biorthogonal click reactions encompass metal-free click-reactions (i.e., which do not require metal catalysts). Examples of metal-free click reactions are depicted hereunder:

| | |
|---|---|
| | Approach 4: Chloro-oxime/Norbornene reaction |
| 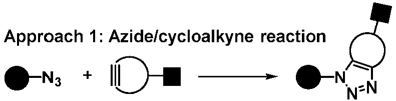 | 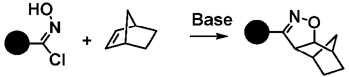 |
| Approach 2: Tetrazine/Cyclooctene reaction | |
| 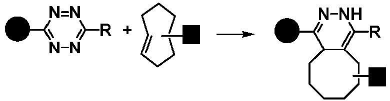 | |
| Approach 3:Tetrazole/Alkene reaction | |
| 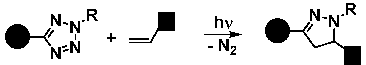 | |

In some embodiments, the polypeptide construct is a polypeptide and does not contain a non-peptide region. In that case, the polypeptide construct may be prepared by standard peptide synthesis, e.g., SPPS method. Alternatively, the polypeptide construct can be prepared by ligation chemistry. For this purpose, one can contemplate adding (or replacing) one of the amino acid residues in a linker region with a cysteine to enable the chemical conjugation.

After the chemical ligation is performed, the cysteine can be converted into alanine to avoid the formation of intermolecular S-S bridges between polypeptide construct molecules.

For instance, the polypeptide construct can comprise a linker of QGCRRKK or QGARRKK as a result of the ligation chemistry step.

Alternatively, the polypeptide can be fully produced recombinantly by using a cell-free translation system such as cell-free wheat germ lysate and reticulocyte lysates. Alternatively, the polypeptide construct may be produced by transfecting host cells with expression vectors that comprise a polynucleotide that encodes the polypeptide construct. For recombinant production, a polynucleotide comprising a sequence which encodes for the polypeptide construct is introduced into host cells by conventional methods such as calcium phosphate transfection, DEAE-dextran mediated transfection, microinjection, cationic lipid-mediated transfection, electroporation, transduction, scrape loading, ballistic introduction, or infection. Suitable host cells encompass, for example, mammalian cells (for example, COS, CHO, BHK, 293 HEK, VERO, HeLa, HepG2, MDCK, W138, or NIH 3T3 cells), yeast (for example, *Saccharomyces or Pichia),* bacteria (for example, *E. coli, P. pastoris, or B. subtilis*), insect cells (for example, baculovirus in Sf9 cells) or other cells under the control of appropriate promoters using conventional techniques. Following transformation of the suitable host strain and growth of the host strain to an appropriate cell density, the cells are harvested by centrifugation, disrupted by physical or chemical means, and the resulting crude extract is recovered for further purification to obtain the present polypeptide construct.

Suitable expression vectors include, for example, chromosomal, non-chromosomal, and synthetic polynucleotides, for example, derivatives of SV 40, bacterial plasmids, phage DNAs, yeast plasmids, vectors derived from combinations of plasmids and phage DNAs, viral DNA such as vaccinia viruses, adenovirus, adeno-associated virus, lentivirus, baculovirus, herpes virus, and retrovirus. The polynucleotide may be introduced into the expression vector by conventional procedures known in the art.

The polynucleotide which encodes for the polypeptide construct may be operatively linked to an expression control sequence, i.e., a promoter, which directs mRNA synthesis. Representative examples of such promoters include the LTR or SV 40 promoter, the *E. coli* lac or trp, the phage 10 lambda PL promoter, and other promoters known to control expression of genes in prokaryotic or eukaryotic cells or in viruses. In some embodiments, the polynucleotide sequence is operatively linked to a ubiquitous promoter. In some embodiments, the polynucleotide sequence is operatively linked to a constitutive promoter. In some embodiments, the expression vectors may include regulatory regions that impact mRNA synthesis such as any one or more of enhancers, silencers, and insulators.

The expression vector may also contain a ribosome binding site for translation initiation and a transcription terminator. The expression vectors may also include an origin of replication and a selectable marker, such as the ampicillin resistance gene of *E. coli* to permit selection of transformed cells, i.e., cells that are expressing the heterologous polynucleotide. The nucleic acid molecule encoding the polypeptide construct may be incorporated into the vector in frame with translation initiation and termination sequences.

The present patent application also relates to the polynucleotide per se encoding for the polypeptide construct provided herein, and the expression vector, as well as the host cell transfected with such expression vector.

### The subject

The subject refers to any human being in need of immunotherapy with the present polypeptide construct. The subject is a human. The subject may be of any gender and of any age. The subject may be an infant, a child, a teenager, or an adult. In some embodiments, the subject is under 18 years old, preferably under 14 years old. For example, the subject is a child from 1 year old to 11 years old, such as from 4 to 11 years old. In other embodiments, the subject is an adult. Preferably, the subject is of haplotypes HLA DQ2 or HLA DQ8.

In some embodiments, the subject is clinically symptomatic of CeD, which means that the subject suffers from CeD and exhibits one or several clinical symptoms of CeD, in particular if exposed to gluten intake. The subject may exhibit any grade of CeD, e.g., from a minimal or moderate form to a severe form such as hyperplastic villous atrophy.

In some further embodiments, the subject suffers from CeD but is clinically asymptomatic or clinically pauci-symptomatic, which means that the subject does not experience the common symptoms of CeD such as gastro-intestinal symptoms. In this form of CeD (also known as silent CeD or asymptomatic CeD), the subject does not complain of any symptoms, but still experiences villous atrophy damage to the small intestine. Studies show that even though patients feel they do not have symptoms, they report better health and a reduction in acid reflux, abdominal bloating, distention, and flatulence after adopting a strict gluten-free diet. In a particular embodiment, the subject is clinically asymptomatic or clinically pauci-symptomatic for CeD but is positive for anti-gliadin and/or anti-transglutaminase antibodies.

In some other embodiments, the subject has not developed CeD, but he/she is at risk of developing CeD, e.g., because the subject is of haplotypes HLADQ2 or HLA-DQ8 and/or has a family history of CeD and/or dermatitis herpetiformis and/or suffers from type 1 diabetes, Down or Turner syndrome, autoimmune thyroid disease, microscopic colitis, IgA deficiency, or Addison's disease.

In a preferred embodiment, the subject suffers from CeD and is symptomatic or already experienced CeD's symptoms. In some embodiments, the subject has been diagnosed as suffering from CeD and is on a gluten-free diet.

In another preferred embodiment, the subject is sensitive to gluten but does not suffer from wheat allergy, in particular from IgE-mediated wheat allergy.

### The administration route and means for providing such administration

The polypeptide construct provided herein can be administered by any route such as by oral, buccal, sublingual, mucosal, intraocular, or parenteral route such as by intravenous (including administration by bolus or by perfusion), intraarterial, rectal, intraperitoneal, intracavity, intramuscular, intranasal, topical, transdermal, intradermal, subcutaneous, or epicutaneous route. The epicutaneous route is preferred.

Accordingly, the polypeptide construct is formulated according to the route of administration. For instance, the polypeptide construct can be in the form of tablets, troches, lozenges, aqueous or oily suspensions, dispersible powders or granules, emulsions, hard or soft capsules, syrups, elixirs, injectable forms, solid form such as freeze-dried powder to be resuspended before injection, aerosol liquid dispersion, gel, ointment, cream, and the like. Aerosol liquid dispersions are preferred; when sprayed onto, e.g., a patch (via electrospray techniques known in the art), the liquid dispersion dries into a cohesive powder formulation.

In embodiments, the disclosed polypeptide constructs are administered as a pharmaceutical composition. In embodiments, the pharmaceutical composition can be formulated in a wide variety of dosage forms, including but not limited to nasal, pulmonary, oral, topical, or parenteral dosage forms for clinical application. The pharmaceutical composition can also be formulated for injection, insufflation, infusion, or intradermal exposure. For instance, an injectable formulation may comprise the disclosed polypeptide constructs in an aqueous or non-aqueous solution at a suitable pH and tonicity.

In some embodiments, the polypeptide construct is in a formulation that allows for controlled release, sustained release, delayed release, or immediate release of the polypeptide construct. In some embodiments, the polypeptide construct is in a formulation that allows for any combination of controlled release, sustained release, delayed release, or immediate release of the polypeptide construct.

As mentioned above, preferred routes of administration for the present polypeptide construct are transdermal, intradermal, subcutaneous, or epicutaneous route, the most preferred being the epicutaneous route.

Epicutaneous application is typically performed by applying the present polypeptide construct in such a way that said polypeptide construct is in contact with the surface of the skin area in the subject. The application is maintained for a period of time sufficient to allow the penetration of the polypeptide construct in the superficial layers of the skin (namely epidermis) so that the polypeptide construct reaches the epidermal DCs (i.e., LCs) and can be processed by said cells.

In preferred embodiments, the polypeptide construct of interest is applied on an intact skin. More precisely, the skin area on which the compound is applied does not undergo any pretreatment such as microporation, skin abrasion, stripping, or chemical treatment.

In some embodiments, the skin area is healthy, which means that the skin area where the polypeptide construct is applied is devoid of any skin disorder such as wound, skin irritation, skin inflammation, or skin disease such as eczema or dermatitis herpetiformis.

Epicutaneous administration is preferably performed using a device suitable to maintain contact between the compounds of interest and the skin of the subject. Such devices may include a patch, a tape, a dressing, a sheet, a gel such as hydrogel, hydrocolloids, or any other form known to those skilled in the art.

The preferred device for administration is a skin patch.

Accordingly, the application also relates to a skin device, in particular a skin patch, comprising the polypeptide construct provided herein.

The device may be suitable to deliver the polypeptide construct in the superficial layers of skin, mostly in the epidermis with no significant passage in the bloodstream.

In some embodiments, the polypeptide construct may be in the form of a liquid composition and applied using known devices, such as occlusive devices having a reservoir and a perforated membrane.

In preferred embodiments, the skin device is a patch, even more preferably an occlusive patch wherein the polypeptide construct is present in dry form. For instance, the present polypeptide construct can be carried by, and administered using, a skin patch device as described by the Applicant in, e.g., International Patent Publication Nos. WO2011/128430, WO02/071950, WO2007/122226, and WO 2023/066846.

In a specific embodiment, such a device is occlusive and is configured to use the polypeptide construct in dry form, preferably as a spray-dried cohesive powder.

In some embodiments, the patch comprises a backing wherein the polypeptide construct is deposited preferably in dry form, preferably as a spray-dried cohesive powder.

In some embodiments, including in some preferred embodiments, the polypeptide construct may be maintained on the patch through electrostatic forces without the means of any adhesive.

In a particularly preferred embodiment, the portion of the backing of the patch bearing the polypeptide construct is not in direct contact with the skin. For the performance of the present polypeptide construct, it is particularly suited to use a device comprising a backing adapted to create with the skin a hermetically closed condensation chamber. The polypeptide construct is deposited on this backing. The polypeptide construct can be absorbed on the backing or adhered to it through electrostatic forces such as Van der Waals forces. Upon application of the patch to the skin, moisture increases in the chamber, leading to the solubilization of the polypeptide construct and contact with the skin.

In other words, when applied on the skin, the patch forms an occlusive, impermeable chamber where the polypeptide construct deposited on the inner surface of the chamber in dry form is solubilized by trans-epidermal water loss (TEWL) and perspiration and can then cross the *stratum corneum* and penetrate into epidermis so as to reach epidermal DCs. The term "perspiration" means the production of a fluid by the skin of mammals. This fluid contains mainly water but also various dissolved minerals and trace elements. In the present embodiment, perspiration secreted by the skin evaporates and condenses within the hermetically closed chamber.

It is believed that the hermetically closed chamber increases the permeability of the skin facilitating the passage of the polypeptide(s) into the first layers of the epidermis. Of note, such a device does not result in a significant passage of the polypeptide construct into the bloodstream.

The height of the condensation chamber defined by the backing, the periphery of the backing, and the skin is in the range of 0.1 mm to 1 cm, typically from 0.1 mm to 5 mm such as 0.1 to 1 mm.

The backing (also called herein the support) of the patch may be of glass or a polymer selected from the group consisting of cellulose plastics (CA (cellulose acetate), CP (cellulose propionate)), polyvinyl chloride (PVC), polypropylenes, polystyrenes, polyurethanes, polycarbonates, polyacrylics in particular poly(methyl methacrylate (PMMA)), polyesters, polyethylenes (PE), polyethylene terephthalate (PET), fluoropolymers (PTFE for example), and ethylene vinyl acrylates (EVA).

In some embodiments, the occlusive skin patch may comprise a breathable over-adhesive (also called dressing), a backing preferably made of polyethylene terephthalate (PET), and an adhesive crown, the adhesive crown and the backing being adapted to create with the skin a hermetically closed chamber. The adhesive crown can be any appropriate polymeric foam and is typically selected so as to form an airtight joint between the backing of the patch and the skin of the subject. The breathable over-adhesive (or adhesive dressing) is used to ensure the adhesiveness of the patch on the skin.

The occlusive patch may further comprise a release liner and a paper applicator.

An example of an appropriate patch to administer the present polypeptide construct is the VIASKIN^{®} patch developed by the Applicant. Other examples of patches of interest can be found, e.g., in International Publication Nos. WO 2023/066846 and WO2007/122226.

Typically, the polypeptide construct is present in dry form on the skin facing side of the backing. The polypeptide construct may have been deposed onto the backing by spray drying or by electrospray as described in WO2009/095591. Alternatively, the polypeptide construct can adhere to the backing by means of an adhesive, e.g., an acrylic adhesive.

When electrospray is used, the backing is typically coated with a conductive layer, e.g., coated with metal particles or metal oxide particles such as titanium oxide, aluminum, or gold, so as to enable positive or negative charges to be distributed over the backing during the electrospray process and the deposit of the compounds of interest.

The polypeptide construct can be present in pure form or as an admixture with pharmaceutically acceptable excipient(s) (e.g., with or without adjuvant(s)) in the patch.

Preferably, the polypeptide construct is not combined with any adjuvant in the patch. In other words, the polypeptide construct is administered in the form of an adjuvant-free preparation by the epicutaneous route.

Pharmaceutically acceptable excipients that may be used are, in particular, described in the Handbook of Pharmaceuticals Excipients, American Pharmaceutical Association (Pharmaceutical Press; 6^{th} revised edition, 2009),. Examples of appropriate pharmaceutically acceptable excipients include, but are not limited to, solvents such as water or water/ethanol solutions, fillers, carriers, diluents, binders, osmotic agents, salts, buffering agents, stabilizers, antioxidants, preservatives, surfactants, solubilizing agents, disintegrating agents, thickeners, wetting agents, and the like.

For instance, the compounds of interest, namely the polypeptide construct, may be formulated together with one or several pharmaceutically acceptable excipients selected from buffering agents, diluents, surfactants, and stabilizers typically in the form of a liquid formulation (e.g., as a hydroalcoholic solution) said formulation being deposited, for instance by electrospray, on the skin facing backing of the patch in condition enabling the evaporation of the solvent and the formation of a dry deposit on the backing.

### Posology and regimen

The methods provided herein typically comprise the repeated administration of the polypeptide construct to the subject, leading to a progressive increase in tolerance to gluten, e.g., gliadin, in the subject. As mentioned above, preferred administration route is epicutaneous one.

The posology regimen (dose, frequency, duration of the treatment) can be determined by the skilled artisan in view of the subject, the nature of the polypeptide construct preparation(s), the type of device used to provide epicutaneous administration, etc. The posology regimen may vary from subject to subject depending on the subject's age, body size, general condition, possible comorbidities or cotreatment, the severity of the disease, the route of administration, and the like.

In some examples, the method comprises the application of the polypeptide construct at least once a month over a period of several months (e.g., about 6, about 12, about 18, about 24, about 36, or about 48 months) until a sufficient increase in tolerance is obtained.

In some embodiments, the polypeptide construct is administered at least once a week, preferably once a day or once every two days, during a period of at least about 6 months such as at least about 1 year, at least about 2 years, or at least about 3 years.

For instance, the method provided herein may comprise the application of a new patch comprising the polypeptide construct once every two days or once every day. In some examples, the duration of contact of the patch with the skin for each application is in the range of about 1 to 48 hours, about 2 to 48 hours, about 3 to 48 hours, about 4 to 48 hours, about 5 to 48 hours, about 6 to 48 hours, about 7 to 48 hours, about 8 to 48 hours, about 1 to 36 hours, about 2 to 36 hours, about 3 to 36 hours, about 4 to 36 hours, about 5 to 36 hours, about 6 to 36 hours, about 7 to 36 hours, about 8 to 36 hours, about 1 to 24 hours, about 2 to 24 hours, about 3 to 24 hours, about 4 to 24 hours, about 5 to 24 hours, about 6 to 24 hours, about 7 to 24 hours, about 8 to 24 hours, about 12 to 48 hours, about 12 to 36 hours, or about 12 to 24 hours; in some embodiments, the duration of contact of the patch with the skin for each application is about 6 hours, about 8 hours, about 12 hours, about 18 hours, about 24 hours, about 36 hours, or about 48 hours.

In some examples, the polypeptide construct on each patch is typically in the range of about 0.1 to about 1000 µg/cm² of patch surface, preferably in the range of about 20 to about 500 µg/cm² of patch surface, more preferably in the range of about 20 to about 200 µg/cm² of patch surface. In some examples, the patch surface is in the range of about 1 cm² to 10 cm², preferably in the range of about 1 cm² to 5 cm².

The polypeptide construct is administered at an effective amount. The term "effective amount" means the amount sufficient to provide the desired therapeutic or physiological effect when administered under appropriate or sufficient conditions. As explained above, this amount can vary from subject to subject depending on the subject's age, body size, general condition, possible comorbidities or cotreatment, the severity of the disease, and the like.

In some examples, the amount of the polypeptide construct may be from about 1 µg to about 5 mg, preferably from about 10 µg to about 2 mg such as from about 50 µg to about 500 µg or about 500 µg to about 1000 µg per patch. In some embodiments, the amount of the polypeptide construct is about 1 µg to about 4 mg, about 1 µg to about 3 mg, about 1 µg to about 2 mg, about 1 µg to about 1 mg, about 1 µg to about 1000 µg, about 1 µg to about 500 µg, about 10 µg to about 5 mg, about 10 µg to about 4 mg, about 10 µg to about 3 mg, about 10 µg to about 2 mg, about 10 µg to about 1 mg, about 10 µg to about 1000 µg, about 10 µg to about 500 µg*,* 50 µg to about 5 mg, about 50 µg to about 4 mg, about 50 µg to about 3 mg, about 50 µg to about 2 mg, about 50 µg to about 1 mg, about 50 µg to about 1000 µg, about 50 µg to about 500 µg, about 500 µg to about 5 mg, about 500 µ to about 4 mg, about 500 µg to about 3 mg, about 500 µg to about 2 mg, about 500 µg to about 1 mg, or about 500 µg to about 1000 µg.

To efficiently increase the tolerance of the subject to gliadin and more generally to gluten, the polypeptide construct is administered in a dose sufficient to induce an immune reaction in the subject.

The method provided herein may comprise an initiation phase wherein the contact duration of the patch with the skin is progressively increased, e.g., from about 3 hours a day to about 12 hours a day, or to about 24 hours a day, etc. In some examples, the initiation phase may last 1 to 4 weeks.

In some examples, after the initiation phase and during the regular treatment, the patch is preferably applied once a day at least until a sufficient increase in tolerance is achieved.

In preferred embodiments, the subject is on a gluten-free diet during the treatment, in particular during the initiation and the regular treatment.

For application, the patch device is applied directly to the skin, preferably on an intact area of the skin and more preferably on a dry, clean, healthy area of the skin, in particular a non-inflamed area of the skin. Typically, the patch can be applied to the shoulders, scapula, arms, or thighs.

In some embodiments, the patch is applied on the skin after the deposit of a drop of water or a biocompatible solvent (such as ethanol) on the skin. Such a drop of water or biocompatible solvent may improve the solubilization of the polypeptide construct present in the patch. In some particular embodiments, the skin may be pretreated prior to application of the polypeptide construct. The pretreatment of the skin is preferably contemplated when the subject is an adult. The pretreatment of the skin is preferably superficial. Typically, the skin pretreatment can alter the stratum corneum and optionally one or several epidermis cell layers while maintaining the integrity of the dermis and the hypodermis of the skin. Preferably, the skin pretreatment does not alter the epidermis basal layer as well. Skin treatments encompass, without being limited to, skin microporation, such as laser microporation, skin cleansing, gentle skin stripping, skin exfoliation, application of penetration-enhancing compounds (e.g., in a topical formulation, e.g., creams, sprays, ointments, etc.), and the like. When the subject is an infant or a child under the age of 12 years, skin pretreatment may be avoided.

Preferably, the skin does not undergo any pre-treatment before the application of the patch and/or no drop of water or biocompatible solvent is deposited on the skin before applying the patch.

In preferred embodiments, the polypeptide construct provided herein is in a dosage form (e.g., integrated into a skin patch) enabling self-administration at least in adults. In examples in which the subject is an infant or child, the dosage form (e.g., skin patch) optionally may be applied by an adult caregiver, as opposed to by a medical professional. Such a self-administration or application by an adult caregiver may facilitate regular use and changing/re-application of the dosage form (i.e., a second, third, fourth, etc. patch), by reducing the burden of traveling to a medical professional for application.

The method provided herein may induce tolerance in a subject sensitive to gluten by promoting an immune deviation from a dominant Th1 profile to a more balanced Th1/Th2 profile.

In celiac subjects, the balance Th1/Th2 is altered and the Th1 profile is predominant. An immune deviation from a dominant Th1 profile to a more balanced Th1/Th2 profile means a deviation from a pathologic state to a tolerant state. This deviation, associated with an increase in Treg cells, can be evaluated by any method known by the skilled person, such as the analysis of cytokine production and/or the analysis by enumeration of CD4⁺ T-cells in PBMCs or whole blood from patients subjected to an oral gluten challenge after the treatment.

Indeed, the repeated administration of the polypeptide construct by epicutaneous route in a human subject sensitive to gliadin will progressively promote an immune deviation towards tolerance and Treg induction, as evidenced by a significant decrease in IFN-γ, IL-2, and IL-17 (decrease in gliadin-specific Th1/Th17 responses).

By inducing immune tolerance to gluten, the method provided herein may abrogate, reduce, or delay the onset of one or several symptoms of CeD. The method provided herein may also treat, e.g., remove, reduce, or delay the progression of intestinal damages associated with celiac disease such as mucosa-villous atrophy.

Moreover, the method provided herein may fully or partially protect the subject from disorders or symptoms resulting from the intake of gluten, typically by preventing or inhibiting the activation of CD4⁺ T cells triggered by the gluten intake. In other words, once tolerance to gliadin is increased or obtained by the method provided herein, the subject can have a less strict gluten-free diet, and even a diet with a controlled intake of gluten (or even a normal diet) without experiencing significant symptoms of CeD. The damage in the intestine can be prevented or reduced as well.

As suggested above, the immunotherapeutic method provided herein can be monitored by several means known by the skilled artisan.

For instance, by:
(a) The analysis of the frequency and phenotype of individual CD4⁺ T cells specific for gliadin: This analysis may be performed by direct enumeration of cells, for example, by flow cytometric analysis. Oral ingestion of gluten in patients with CeD normally following a gluten-free diet is known to stimulate T cells specific for the peptides and gluten generally, and to induce rapid mobilization of those memory T-cells from gut-associated lymphoid tissue into the peripheral blood and hence increase circulating numbers. A clinical test such as a gluten challenge may be used to assess the T cells induced in blood or other tissues. The phenotype of isolated T cells may then be assessed fresh or following short-term expansion *in vitro.* Assays of T cells may rely upon MHC-peptide complexes, antigen-stimulated intracellular cytokine, or other cell surface markers induced on antigen-activated T cells. Functional status of CD4⁺ T cells is correlated with the presence of various cell-surface and intra-cellular markers, for example, correlated with the presence of activation markers including CD25 and CD69, or correlated with the presence of "tolerance" and regulatory T cell function, for example, GITR and FOXP3. Production of cytokines such as IFN-y, IL-2, and IL-17A would be considered pro-inflammatory and associated with the disease, while secretion of IL-10 and TGF-β would be associated with tolerogenic immune responses. Without wishing to be bound by any theory, it is expected that the present polypeptide constructs would cause markers of pro-inflammatory immune responses to decline and/or would cause markers of tolerogenic immune responses to be enhanced.
(b) Histological examination of the tissues exposed to gluten before, during, and after the treatment using the present polypeptide constructs: histological analysis can be performed typically in the small bowel, by techniques such as biopsy or direct visualization by endoscopy including capsule endoscopy. Histological readouts such as the infiltration of intraepithelial lymphocytes (IEL) and the ratio between duodenum villi height and crypt depth (Marsh classification) can be used to evaluate the severity of the disease. Without wishing to be bound by any theory, it is expected that the present polypeptide constructs would reduce IEL infiltration, increase the height of duodenum villi, and/or decrease the depth of duodenum crypts.

The following examples are given for purposes of illustration only and not by way of limitation.

### WORKING EXAMPLES

### EXAMPLE 1: Design of building blocks and polypeptide constructs.

A polypeptide construct suitable to promote tolerance to gluten when administered through the epicutaneous route was conceived. Firstly, key epitopes from α-, γ- and ω-gliadin were selected based upon immuno-dominance and immuno-prevalence in CeD patient populations. The epitope sequences (9-mer core epitope sequences) were modified with Q->E substitutions at key deamidation sites and by adding flanking residues from native gliadin sequences so as to obtain building blocks. Then peptidic linkers to link together the building blocks were conceived. The polypeptide constructs were designed so as to (i) improve processing by antigen-presenting cells (APCs), (ii) limit/avoid the generation of junctional neoepitopes (irrelevant epitopes composed of linkers and a part of the adjacent sequences of interest that could potentially bind to MHC-II), and (iii) improve the overall level of solubility in water, physiological buffer, and/or artificial perspiration (for epicutaneous delivery using a patch). The C-terminal extremity of polypeptide constructs was amidated to improve stability. In addition, the N-terminal extremity of polypeptide constructs was acetylated to improve stability and to prevent the conversion of the first glutamine [Q] residue into a pyroglutamine under the acidic conditions required for purification. The suitability of the different polypeptide constructs was validated by *in-silico* prediction tools. In particular, the solubility was predicted using PepCalc peptide property calculator (Innovagen), and the presence/number of junctional neoepitopes was predicted using NetMHCIIpan 4.0 EL (DTU Health Tech) and a panel of 27 HLA reference alleles from which all HLA-DQ alleles other than HLA-DQ2 or HLA-DQ8 have been removed.

As an example, physicochemical properties of three polypeptide constructs of interest were compared to those of the four individual building blocks (denoted as BB1, BB2a, BB3a, and BB4a), as shown in Table 5. Note that all building blocks were amidated at their C-terminal extremity. The building block starting with a glutamine [Q] residue (SEQ ID NO:15 [BB1]) was also acetylated at its N-terminal extremity. Polypeptide constructs of interest (SEQ ID NO:23 wherein X is C [Cv6, i.e., SEQ ID NO:49], SEQ ID NO:23 wherein X is A [Av6, i.e., SEQ ID NO:50] and SEQ ID NO:46 [4K]) are predicted to have an isoelectric point significantly above pH 7.0 (e.g., pH 8.0 or 9.0, or even higher) and are thus expected to be fully soluble in water and lightly acidic solutions such as artificial skin perspiration (which would not be the case for some of the individual building blocks as shown below).

**Table 5: Physicochemical properties of polypeptide constructs, compared to individual building blocks*.**

| **Sequence ID** | **N residues** | **Molecular weight** | **Isoelectric point** | **Net charge at pH 7** | **Predicted solubility ^{§}** |
|---|---|---|---|---|---|
| **SEQ ID NO:49 [Cv6]** ^{‡} | 94 | 10.9 kDa | 8.75 | 0.9 | Good |
| **SEQ ID NO:50 [Av6]** ^{‡} | 94 | 10.9 kDa | 9.5 | 1 | Good |
| **SEQ ID NO:46 [4K]** ^{‡} | 90 | 10.6 kDa | 9.9 | 3 | Good |
| **SEQ ID NO:15 wherein Z is E [BB1]** ^{‡} | 31 | 3.7 kDa | 0 | -3 | Poor |
| **SEQ ID NO:16 wherein Z is E [BB2a]** ^{†} | 15 | 1.8 kDa | 3.9 | -2 | Good |
| **SEQ ID NO:20 wherein Z is E [BB3a] ^{†}** | 17 | 2.1 kDa | 7.3 | 0 | Poor |
| **SEQ ID NO:18 wherein Z is E [BB4a] ^{†}** | 15 | 1.6 kDa | 4.2 | -1 | Good |

| | | | | | |
|---|---|---|---|---|---|
| ** **Properties were computed using Innovagen PepCalc peptide property calculator*** *^{‡} **C-terminal amide and N-terminal acetyl*** *^{†} **C-terminalamide*** *^{§} **Predicted solubility in water*** | | | | | |

### EXAMPLE 2: Synthesis and purification.

### Synthesis and purification of polypeptide constructs and individual building blocks:

The three polypeptide constructs and the four peptide fragments corresponding to individual building blocks, designed in accordance with Example 1, were produced by standard solid phase peptide synthesis (SPPS).

Purification of crude polypeptide constructs and individual building blocks was performed by preparative high-performance liquid chromatography (HPLC) on a reversed phase column coupled to a UV detector, using an appropriate gradient of eluent. Purification of polypeptide constructs was performed using TFA salts as counterion, followed by a desalting step in acetate salts. The purified polypeptide constructs as well as individual building blocks were lyophilized and stored frozen at -20°C.

As an alternative way, the polypeptide construct can be prepared by Fragment Assembly peptide syntehsis. This method of synthesis is expected to increase the yield of production.

### Preparation of standard acetic acid-treated gliadin (control):

To prepare acetic acid-treated gliadin, commercial gliadin from wheat (Sigma, ref. G-3375) was resuspended in prewarmed 50 mM acetic acid and stirred for 1 hour at 40°C. The solution was then centrifuged for 10 min at 250xg with centrifuge brake set to 0, and the supernatant was collected. Supernatant containing acetic acid treated gliadin was aliquoted and stored frozen at -20°C.

### EXAMPLE 3: Analysis of purified materials.

### Analysis of polypeptide constructs and individual building blocks:

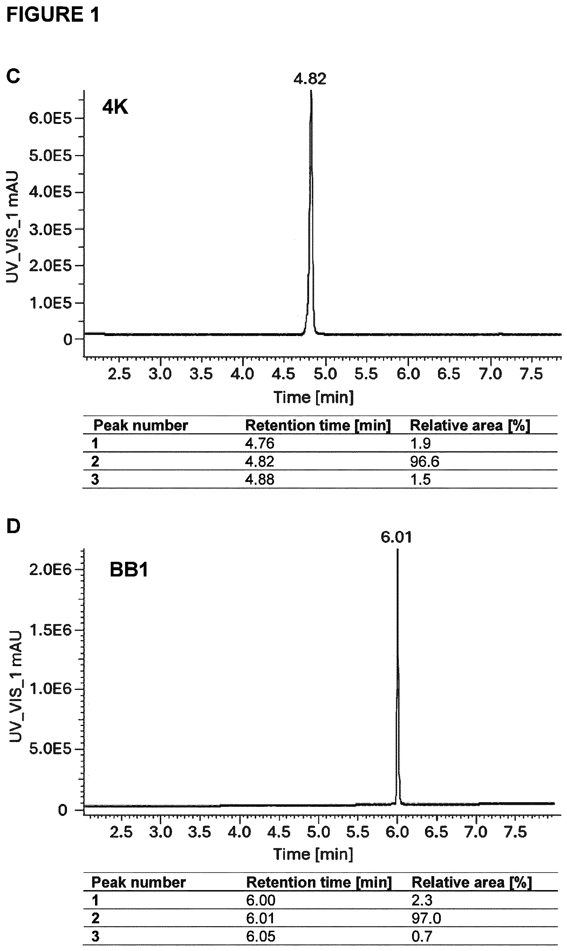

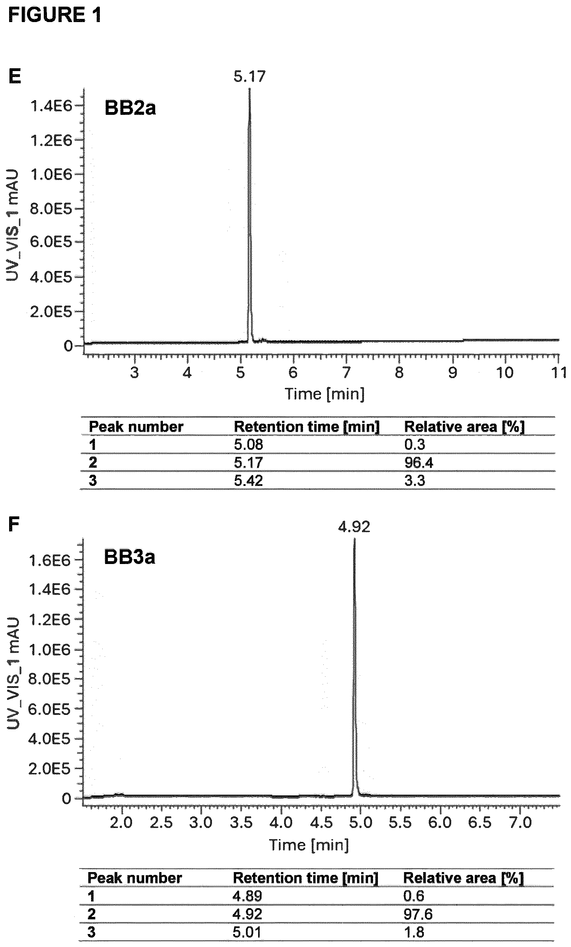

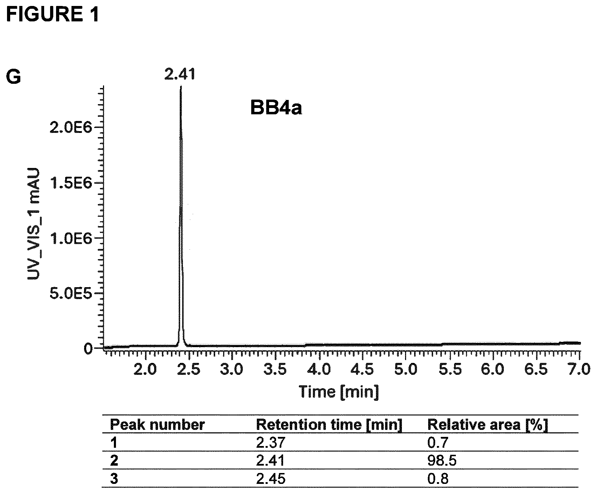

The three polypeptide constructs and the four individual building blocks described in Example 1 were produced and purified as described in Example 2. They were then analyzed by RP-HPLC at 60°C on a Thermo Scientific Vanquish equipped with a reversed phase column (WATERS Acquity CSH C18 1.7µm 130Å [2.1x100mm]), using an appropriate gradient of increasing concentration of Buffer B (0,1% TFA in H2O / CH3CN [20:80, v:v]) in Buffer A (0,1% TFA in H2O / CH3CN [5:95, v:v]). The column eluate was analyzed using a UV detector at 215 nm. HPLC chromatograms showing elution of purified materials from the HPLC column with time are described in Figure 1. Purity and yield of the final products are summarized in Table 6. A purity superior to 95% was obtained for all the polypeptide constructs and individual building blocks, and no degradation was observed.

**Table 6: Purity and production yield of polypeptide constructs, compared to individual building blocks.**

| **Sequence ID** | **Production yield [%]** | **Final purity [%]** |
|---|---|---|
| **SEQ ID NO:49 [Cv6] ^{‡}** | 22.7 | 96.2 |
| **SEQ ID NO:50 [Av6] ^{‡}** | 14.3 | 95.3 |
| **SEQ ID NO:46 [4K] ^{‡}** | 22.0 | 96.6 |
| **SEQ ID NO:15 wherein Z is E [BB1] ^{‡}** | 46.5 | 97.0 |
| **SEQ ID NO:16 wherein Z is E [BB2a] ^{†}** | 65.1 | 96.4 |
| **SEQ ID NO:20 wherein Z is E [BB3a] ^{†}** | 58.9 | 97.6 |
| **SEQ ID NO:18 wherein Z is E [BB4a] ^{†}** | 43.6 | 98.5 |

| | | |
|---|---|---|
| *^{‡} **C-terminal amide and N-terminal acetyl*** *^{†} **C-terminal amide*** | | |

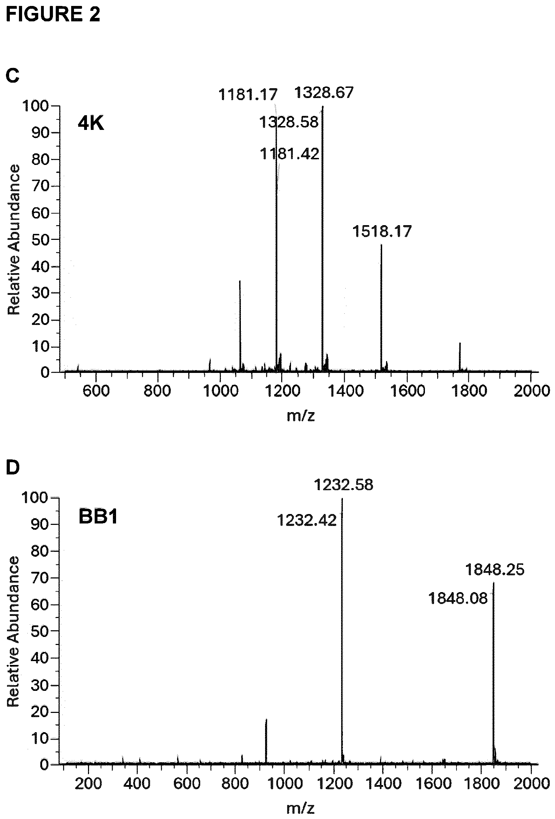

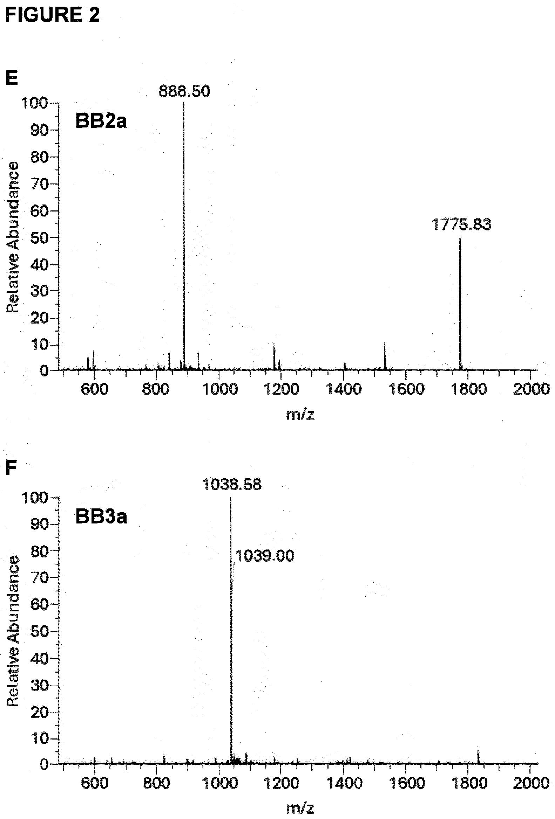

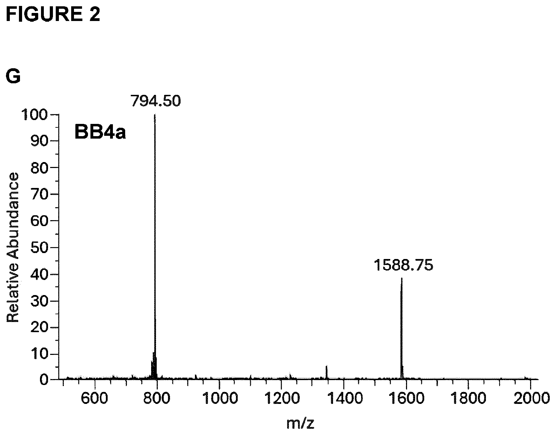

The mass spectra of the polypeptide constructs and individual building blocks were acquired on a linear ion trap mass spectrometer (Thermo Scientific LTQ XL). Calculated masses were based on an average isotope composition. Mass spectra of the final products are shown in Figure 2.

### Analysis of acetic acid-treated gliadin:

Acetic acid-treated gliadin was prepared as described in Example 2 and analyzed by RP-HPLC at 60°C on a Thermo Scientific Vanquish equipped with a reversed phase column (WATERS BEH C4 3.5µm 300Å [2.1x100mm]), using an optimal gradient of increasing concentration of Buffer B (0,1% TFA in H2O / CH3CN [20:80, v:v]) in Buffer A (0,1% TFA in H2O / CH3CN [5:95, v:v]). The column eluate was monitored using a UV detector at 215 nm. As shown in the HPLC chromatogram presented in Figure 3, acetic acid-treated gliadin contained a heterogeneous mix of a wide range of uncharacterized peptides/fragments that could not be separated under these analytical conditions.

### Conclusions on the analysis and control of purified materials:

Despite their length (90-94 amino acids), the three polypeptide constructs (i.e., Cv6, Av6, and 4K) were efficiently produced through standard SPPS, leading to the obtention of well-characterized protein materials with a high level of purity (> 95%). In contrast, the digestion of native gliadin using acetic acid led to the obtention of an undefined product whose actual content in key gliadin epitopes cannot be determined. Those findings suggest that a synthetic immunogen comprising key gliadin epitopes would be better suited for the development of a well-characterized pharmaceutical product than acetic acid-treated gliadin.

### EXAMPLE 4: Solubility and stability analyses.

### Solubility of polypeptide constructs, individual building blocks, and acetic acid-treated gliadin:

Polypeptide constructs (in particular, those of SEQ ID NO:22, 23, and 46) have been carefully designed to improve stability and solubility in water at about pH 7.0, as well as in skin perspiration with more acidic pH. Therefore, it is believed that the polypeptide constructs would be fully adaptable to epicutaneous administration, in particular when part of an epicutaneous system / patch device.

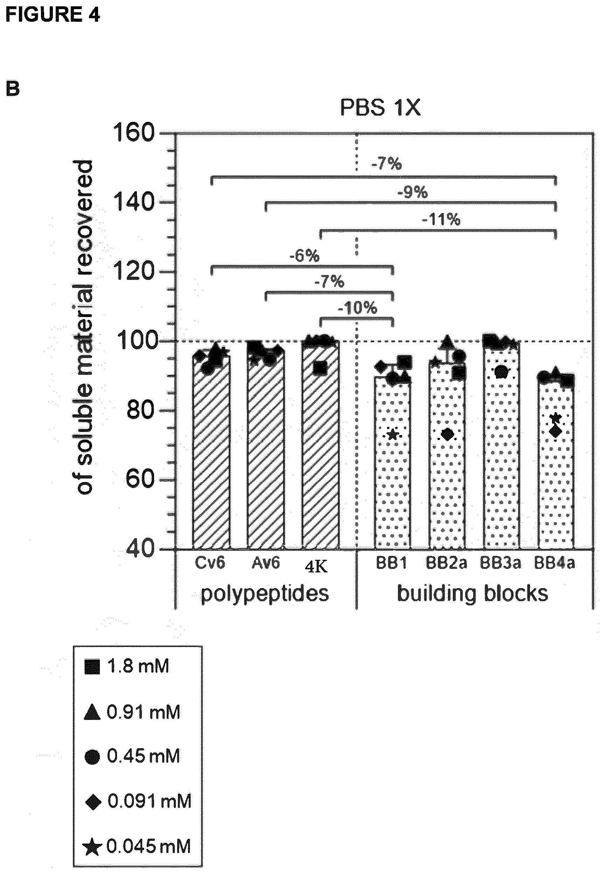

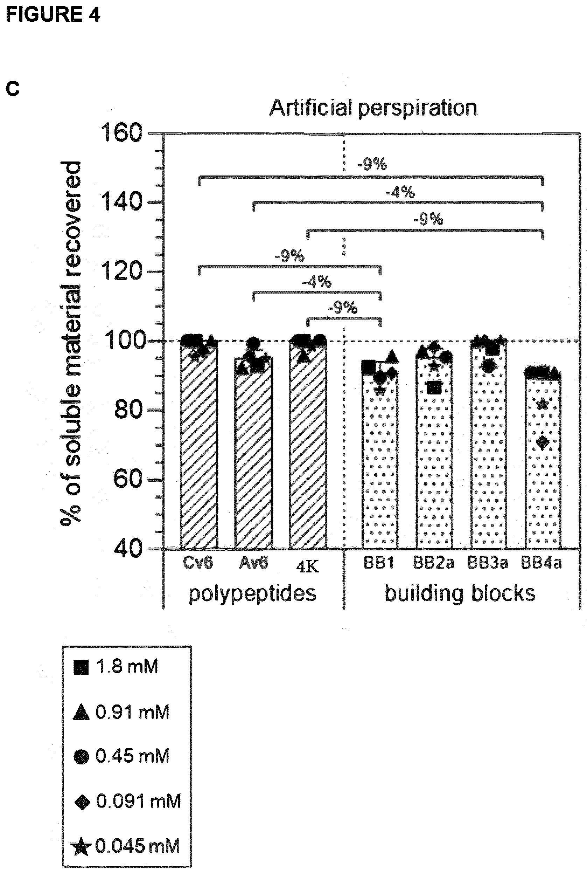

In a preliminary evaluation of their solubility, lyophilized aliquots of three polypeptide constructs (SEQ ID NO:23 wherein X is C [Cv6, i.e., SEQ ID NO:49], SEQ ID NO:23 wherein X is A [Av6, i.e., SEQ ID NO:50], and SEQ ID NO:46 [4K]) and four individual building blocks (SEQ ID NO:15 wherein Z is E [BB1], SEQ ID NO:16 wherein Z is E [BB2a], SEQ ID NO:20 wherein Z is E [BB3a], and SEQ ID NO:18 wherein Z is E [BB4a]), produced and purified as described in Example 2, were dissolved at five different molarities (approximately 1.8, 0.91, 0.45, 0.091, and 0.045 mM, corresponding to respective concentrations of 20, 10, 5, 1, and 0.5 mg/mL for the polypeptide construct Cv6) with either pure water, phosphate-buffered saline (PBS 1X) at pH 7.0, or artificial perspiration at pH 4.5. Then, the concentration in soluble material was measured by HPLC at 215nm. All polypeptide constructs and individual building blocks demonstrated acceptable levels of solubility in the three buffers used for the dissolution of lyophilizates (Figure 4). The solubility of the three polypeptide constructs was high in pure water, PBS 1X, and artificial perspiration, with a median percentage of soluble material of between 94% and 100%. Of note, a slight decrease in solubility was observed for the individual building blocks BB1 and BB4a, compared to polypeptide construct: i) a decrease in the median percentage of soluble BB1 of 3%, 4%, and 8% in pure water; of 6%, 7%, and 10% in PBS 1X; and of 9%, 4% and 9% in artificial perspiration; compared to Cv6, Av6, and 4K polypeptides, respectively; and ii) a decrease in the median percentage of soluble BB4a of 8%, 9%, and 13% in water; of 7%, 9%, and 11% in PBS 1X; and of 9%, 4%. and 9% in artificial perspiration; compared to Cv6, Av6, and 4K polypeptides, respectively).

The stability of the polypeptide constructs in solution was evaluated over time in each of pure water, PBS 1X at pH 7.0, and artificial perspiration at pH 4.5 (in molarities of approximately 1.8, 0.91, 0.45, 0.091, and 0.045 mM), at room temperature and at elevated temperature (32°C). The Av6 and the 4K constructs were found to be stable in solution for at least 24 hours, and no precipitate was observed. For the Cv6 construct, good stability in each of the solutions tested up to 24 hours was also seen. Further HPLC and LC-MS studies indicated that the Cv6 polypeptide construct forms some dimers in solution, likely through the formation of disulfide bonds between the cysteines in the second linker. Dimerization appeared to be faster in artificial perspiration and at higher concentration.

To assess solubility at higher concentrations, the apparent maximum concentration that can be achieved without impairing solubility was evaluated. In the conditions of this test, the Av6 polypeptide construct was still soluble at the highest concentration tested of ~500 mg/mL (~45.8 mM).

The solubility of the acetic acid-treated gliadin prepared as described in Example 2 was also evaluated by reconstituting lyophilized aliquots at five different concentrations (20, 10, 5, 1, and 0.5 mg/mL) in each of pure water, PBS 1X at pH 7.0, and artificial perspiration at pH 4.5, followed by measuring the concentration in soluble material by HPLC at 215nm. Acetic acid-treated gliadin was fully soluble in pure water, with a median percentage of soluble material of 100% (Figure 5A). However, the level of solubility dropped dramatically in PBS 1X or artificial perspiration (median percentages of soluble material of 37.6% and 42.3% in PBS 1X and artificial perspiration, respectively) (Figures 5B and 5C).

### Solubility of polypeptide constructs, individual building blocks, and acetic acid-treated gliadin upon deposition on patch:

In order to evaluate their solubility when used in combination with an epicutaneous patch, lyophilized aliquots of three polypeptide constructs (SEQ ID NO:23 wherein X is C [Cv6, i.e., SEQ ID NO:49], SEQ ID NO:23 wherein X is A [Av6, i.e., SEQ ID NO:50], and SEQ ID NO:46 [4K]) and four individual building blocks (SEQ ID NO:15 wherein Z is E [BB1], SEQ ID NO:16 wherein Z is E [BB2a], SEQ ID NO:20 wherein Z is E [BB3a], and SEQ ID NO:18 wherein Z is E [BB4a]), produced and purified as described in Example 2, were dissolved in pure water, then spotted on a polyethylene terephthalate titanium (PET-Ti) backing film (a type of backing film used in, and useful for, epicutaneous patches), and dried at ~30°C in an oven. Immediately after drying, deposits were reconstituted by pipetting up and down with each of pure water, PBS 1X at pH 7.0, and artificial perspiration at pH 4.5, each at five different molarities (approximately 1.8, 0.91, 0.45, 0.091, and 0.045 mM). The concentration in soluble material was measured by HPLC at 215nm. The three polypeptide constructs and building blocks BB1 and BB3a were efficiently resolubilized by each of pure water, PBS 1X, and artificial perspiration upon drying on the PET-Ti patch backing film, with median percentages of soluble material recovered from the patch backing film being between 93% and 100% (Figure 6). However, the solubilities of building blocks BB2a and BB4a, while above 80% overall for each, were systematically lower than the solubilities of the polypeptide constructs, regardless of the type of solvent used for the reconstitution of the dry deposit: i) (the median percentage of soluble BB2a recovered from the patch backing film decreased by 11%, 9%, and 14% in pure water; by 13%, 11%, and 15% in PBS 1X; and by 12%, 8%, and 14% in artificial perspiration, compared to Cv6, Av6, and 4K polypeptides, respectively; and ii) the median percentage of soluble BB4a recovered from the patch backing film decreased by 13%, 12%, and 16% in water; by 13%, 11%, and 15% in PBS 1X; and by 12%, 8%, and 14% in artificial perspiration, compared to Cv6, Av6, and 4K polypeptides, respectively.

The solubility of acid-treated gliadin upon deposition on a patch backing film was also evaluated: aliquots prepared as described in Example 2 were reconstituted in pure water, then spotted on a PET-Ti backing film, and then dried for at ~30°C in an oven. Immediately after drying, patch deposits were reconstituted by pipetting up and down with each of pure water, PBS 1X at pH 7.0, and artificial perspiration at pH 4.5, at five different concentrations (20, 10, 5, 1, and 0.5 mg/mL). The concentration in soluble material was measured by HPLC at 215nm. As shown in Figures 7A, 7B, and 7C, the proportion of soluble material recovered from the patch backing film was very low, regardless of the type of solvent used for the reconstitution of the dry deposit: the median percentages of soluble material recovered from the patch backing film was 26.4%, 12.7%, and 8.9% in pure water, PBS 1X, and artificial perspiration, respectively. This poor recovery of soluble materials was linked to the formation of precipitates, as illustrated in Figure 7D.

### Solubility of polypeptide constructs and individual building blocks upon deposition on patch and storage for 1 to 3 months at room temperature or 5°C:

In order to evaluate their solubility upon deposition on a patch backing film and a period of storage, the three polypeptide constructs and the four individual building blocks were each dried on a PET-Ti backing film as described above. Backing films were stored at room temperature or 5°C for 1 or 3 months. At each time point, dry deposits were reconstituted with each of pure water, PBS 1X at pH 7.0, and artificial perspiration at pH 4.5, at five different molarities each (approximately 1.8, 0.91, 0.45, 0.091, and 0.045 mM). The concentration in soluble material was measured by HPLC at 215nm. Of note, the solubility of acetic acid-treated gliadin on the patch backing film after a period of storage was not assessed due to the very low levels of solubility observed in previous experiments (see Figure 7).

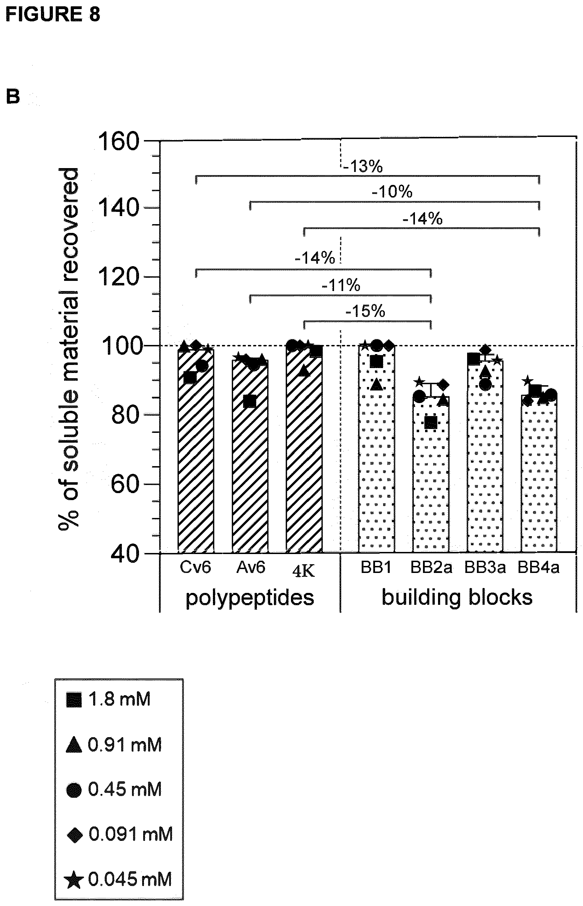

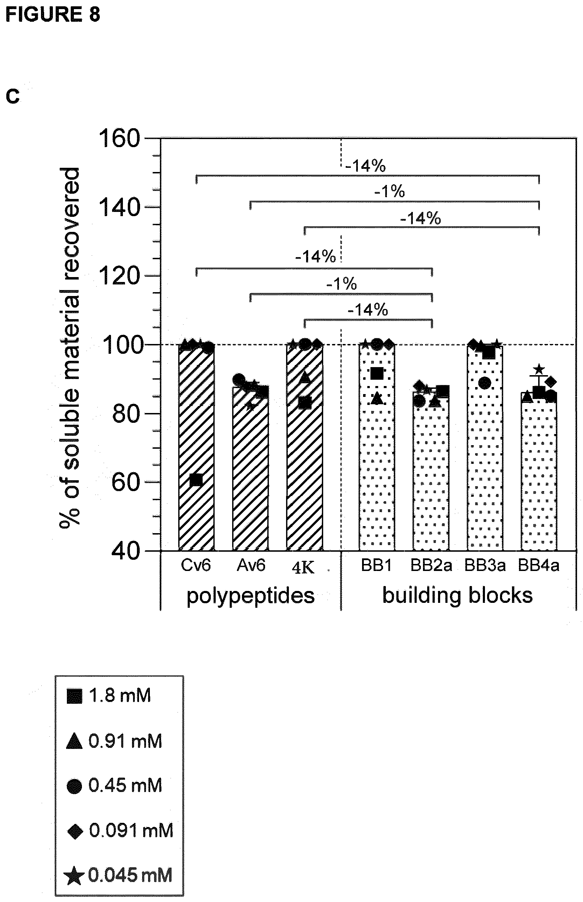

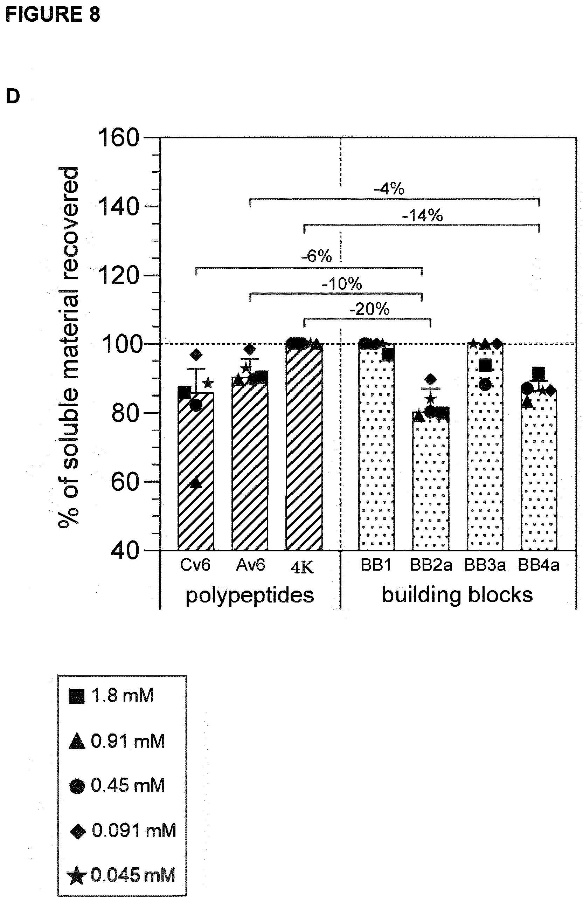

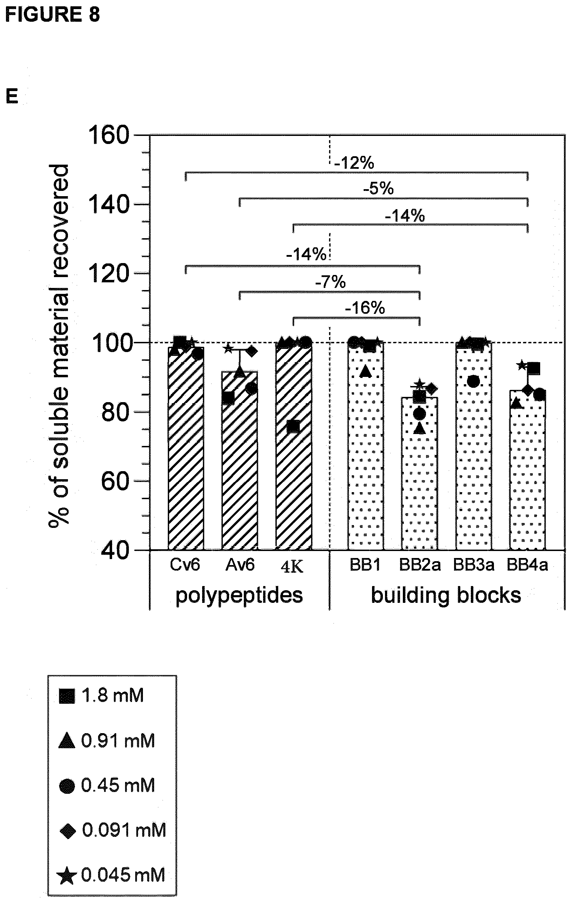

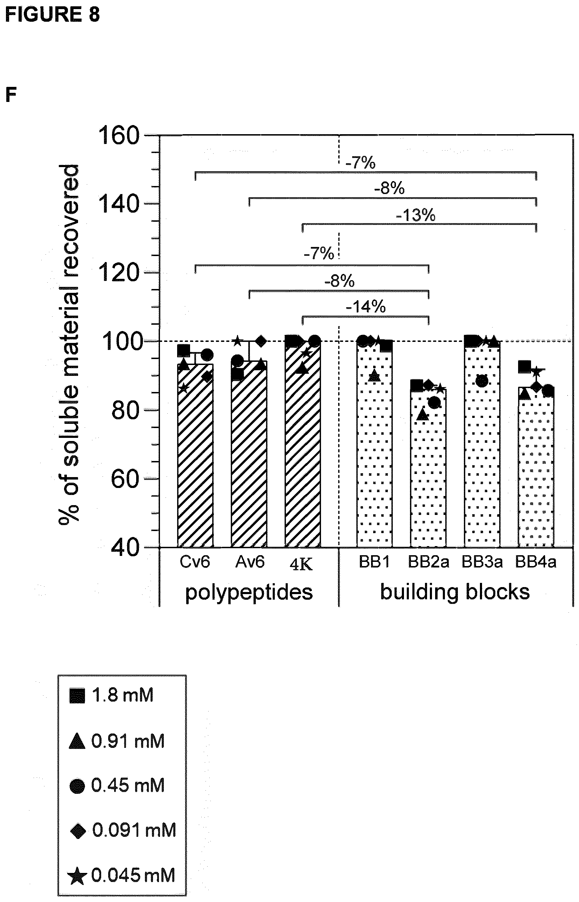

As shown in Figure 8, all polypeptide constructs and individual building blocks can be retrieved from the patch backing film after 1 month of storage, regardless of the type of solvent used for the reconstitution of the dry deposit, with percentages of soluble material recovered being greater than 80%. However, and as previously observed, the solubilities of building blocks BB2a and BB4a were lower than those of the polypeptide constructs under most of the experimental conditions: i) the median percentage of soluble BB2a recovered from the patch backing film stored at room temperature for 1 month decreased by 13%, 9%, and 18% in water; by 14%, 11%, and 15% in PBS 1X; and by 14%, 1%, and 14% in artificial perspiration, compared to Cv6, Av6, and 4K polypeptides, respectively; ii) the median percentage of soluble BB4a recovered from the patch backing film stored at room temperature for 1 month decreased by 11%, 7%, and 15% in water; by 13%, 10%, and 14% in PBS 1X; and by 14%, 1%, and 14% in artificial perspiration, compared to Cv6, Av6, and 4K polypeptides, respectively; iii) the median percentage of soluble BB2a recovered from the patch backing film stored at 5°C for 1 month decreased by 6%, 10%, and 20% in water; by 14%, 7%, and 16% in PBS 1X; and by 7%, 8%, and 14% in artificial perspiration, compared to Cv6, Av6, and 4K polypeptides, respectively; and iv) the median percentage of soluble BB4a recovered from the patch backing film stored at 5°C for 1 month decreased by 0%, 4%, and 14% in water; by 12%, 5%, and 14% in PBS 1X ;and by 7%, 8%, and 13% in artificial perspiration, compared to Cv6, Av6, and 4K polypeptides, respectively.

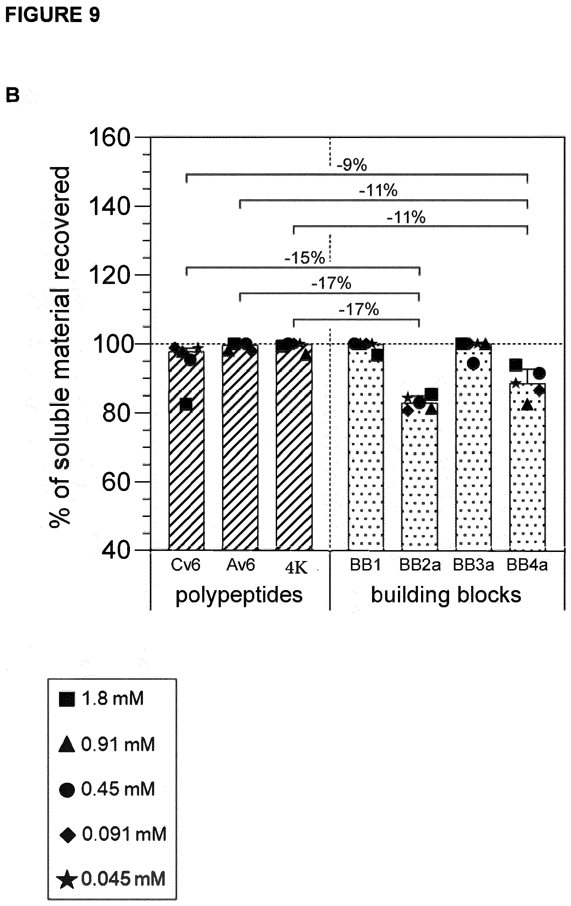

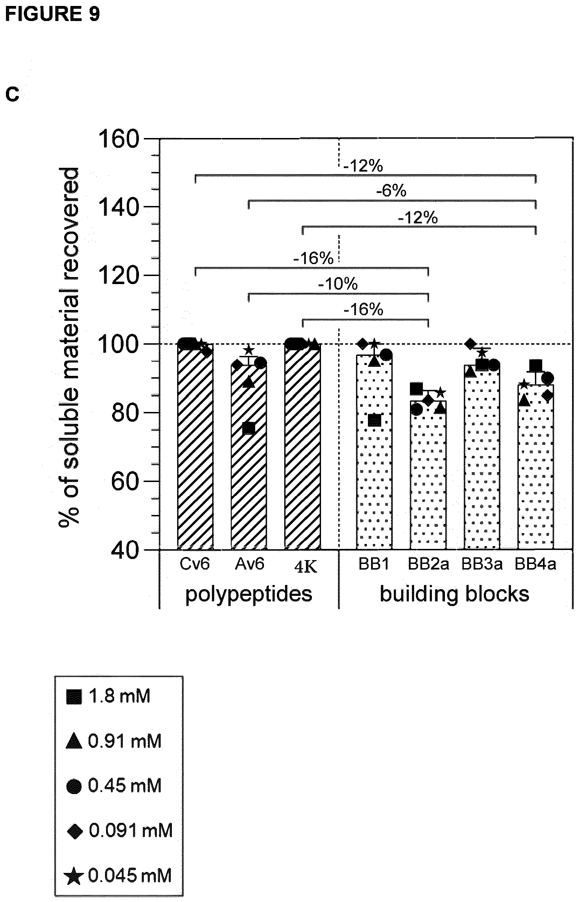

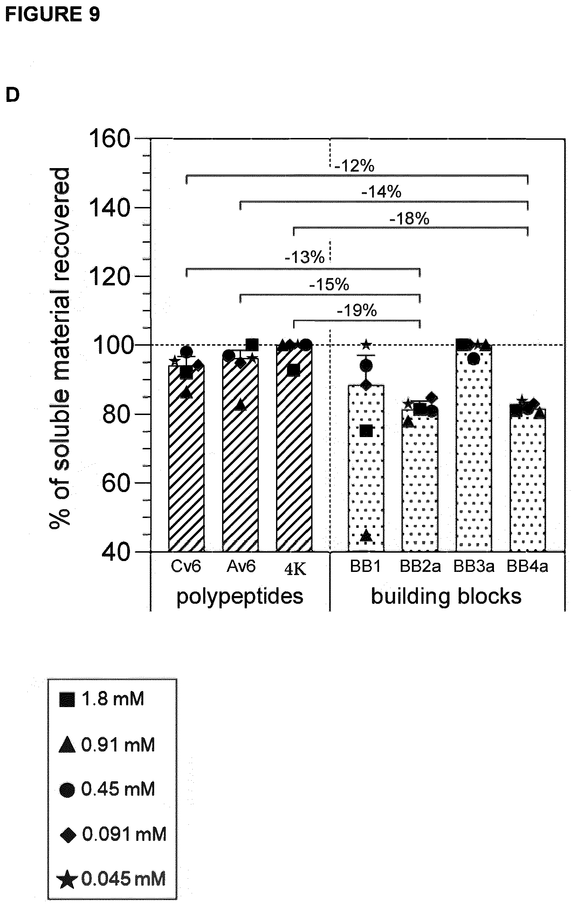

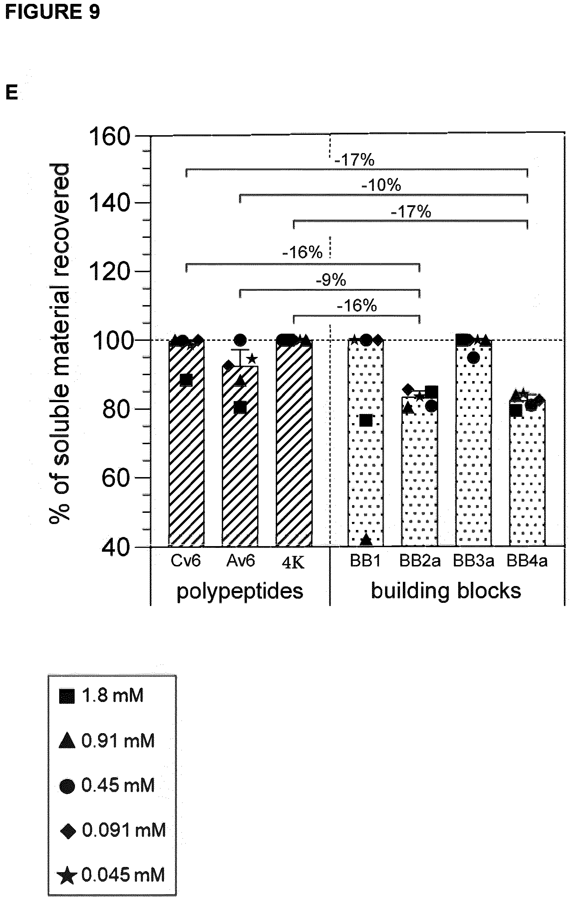

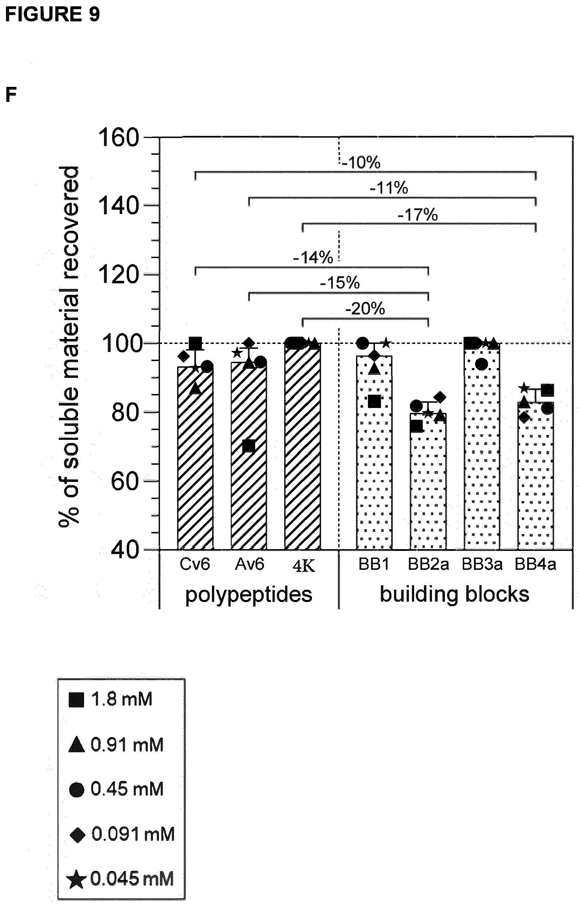

A similar trend was observed after 3 months of storage (Figure 9), with the solubilities of BB2a and BB4a being systematically lower than those of polypeptide constructs, regardless of the temperature of storage or the type of solvent used for the reconstitution of the dry deposit: i) the median percentage of soluble BB2a recovered from the patch backing film stored at room temperature for 3 months decreased by 4%, 10%, and 15% in water; by 15%, 17%, and 17% in PBS 1X; and by 16%, 10%, and 16% in artificial perspiration, compared to Cv6, Av6, and 4K polypeptides, respectively; ii) the median percentage of soluble BB4a recovered from the patch backing film stored at room temperature for 3 months decreased by 2%, 9%, and 13% in water; by 9%, 11%, and 11% in PBS 1X; and by 12%, 6%, and 12% in artificial perspiration, compared to Cv6, Av6, and 4K polypeptides, respectively; iii) the median percentage of soluble BB2a recovered from the patch backing film stored at 5°C for 3 months decreased by 13%, 15%, and 19% in water; by 16%, 9%, and 16% in PBS 1X; and by 14%, 15%, and 20% in artificial perspiration, compared to Cv6, Av6, and 4K polypeptides, respectively; and iv) the median percentage of soluble BB4a recovered from the patch backing film stored at 5°C for 3 months decreased by 12%, 14%, and 18% in water; by 17%, 10%, and 17% in PBS 1X; and by 10%, 11%, and 17% in artificial perspiration, compared to CV6, Av6, and 4K polypeptides, respectively.

### Conclusions on the solubility of purified materials after a period of storage:

Overall, these results confirmed that the three polypeptide constructs (i.e., Cv6, Av6, and 4K) are fully soluble in a range of solvents recapitulating the physicochemical conditions found at the surface of the skin, even after being stored as dry deposits on a backing film (i.e., a type of film used in, and useful for, epicutaneous patches). This strongly suggests that the three polypeptide constructs would be fully suitable for use in epicutaneous treatment using, e.g., a dry epicutaneous patch. By contrast, data suggest that acetic acid-treated gliadin would be unsuited for use in epicutaneous treatment using, e.g., a dry epicutaneous patch, because of its propensity to precipitate in artificial perspiration, *a fortiori* when dried on a patch backing film. The three polypeptide constructs remained soluble up to at least 3 months at room temperature after being dried on a patch backing film and showed a high level of solubility following reconstitution with pure water, PBS 1X, or artificial perspiration. The polypeptide constructs had an advantage with respect to solubility as compared to the individual building blocks, as evidenced by the lower proportion of soluble BB2a and BB4a being recovered from the patch backing film. This was likely due to their unique design featuring peptidic linkers comprising positively charged and hydrophilic amino acids. Therefore, the polypeptide constructs are each more suitable for use in combination with an epicutaneous patch than a mix of small gliadin peptides.

### EXAMPLE 5: Evaluation of selected gliadin polypeptides to reactivate blood cells obtained from CeD patients by Cytokine Release Assay.

### Introduction to the Cytokine Release Assay (CRA):

The overarching objective of this assay was to validate that the polypeptide constructs (Cv6, Av6, and 4K) can be recognized and processed by human APCs, and they can then promote the activation of gluten-specific effector/memory CD4⁺ T cells in CeD patients. A Cytokine Release Assay (CRA) was used (previously described in the literature: Anderson et al., Clinical & Experimental Immunology, 2021 Jun; 204(3): 321-334; and in Hardy et al., Frontiers in Immunology, 2021, Vol. 12, Article 661662). This assay quantifies IL-2 produced by circulating gluten-specific CD4⁺ T cells present in the blood of CeD patients upon *in vitro* stimulation with gluten-derived antigens. Due to the extremely high sensitivity of this CRA, no oral exposure to gluten was required prior to blood sampling.

### Material and methods:

For the CRA assay, approximately 50 mL of peripheral blood was collected by venipuncture from ten (10) participants who have biopsy-confirmed and treated CeD. Informed consent was obtained from all subjects prior to blood collection. Participant demographics are shown in Table 7.

**Table 7. Participant demographics for polypeptide screening by CRA.**

| **Participant number** | **Participant ID** | **HLA haplotype** |
|---|---|---|
| **1** | 0650 | DQ2.5/x |
| **2** | 0198 | DQ2.5/x |
| **3** | 1848 | DQ2.5/x |
| **4** | 6041 | DQ2.5/2.2 |
| **5** | 1596 | DQ2.5/x |
| **6** | 1752 | DQ2.5/x |
| **7** | 1599 | DQ2.2/8 |
| **8** | 0466 | DQ8 |
| **9** | 0602 | DQ8 |
| **10** | 0324 | DQ8/7 |

Unfractionated blood (225 µL per well of a 96-well plate) was then rapidly restimulated for 24 hours with each of three of the polypeptide constructs provided herein (SEQ ID NO:23 wherein X is C [Cv6, i.e., SEQ ID NO:49], SEQ ID NO:23 wherein X is A [Av6, i.e., SEQ ID NO:50], and SEQ ID NO:46 [4K]) and with each of four individual building blocks (SEQ ID NO:15 wherein Z is E [BB1], SEQ ID NO:16 wherein Z is E [BB2a], SEQ ID NO:20 wherein Z is E [BB3a], and SEQ ID NO:18 wherein Z is E [BB4a]). The concentrations of the polypeptide constructs ranged from 0.5 to 10 micromolar, and the concentration for each individual building block was 10 micromolar. As comparators, two immunogenic peptides known to activate CD4⁺ T cells from CeD patients were used: (i) a highly immunogenic peptide derived/taken from α-gliadin 33-mer sequence (LQPFPQPELPYPQPQ) (SEQ ID NO:47) (herein termed "Nex01"), and (ii) a highly immunogenic peptide contained within ω-gliadin sequence (QPFPQPEQPFPWQP) (SEQ ID NO:48) (herein termed "Nex02"), as described in the literature (Sollid et al., Immunogenetics, 2020 Feb;72(1-2):85-88; Tye-Din et al., Sci Transl Med, 2010, Vol. 2, Issue 41, 41-51). These two peptides comprise an N-terminal pyroglutamate and a C-terminal amide. Nex01 and Nex02 were the two gliadin peptides used in the Nexvax2 immunotherapeutic vaccine that was intended to restore immune tolerance to gluten in HLA-DQ2.5+ CeD patients. A positive control consisted of blood cells stimulated with phytohemagglutinin (PHA) (10 µg/mL) which activates all T cells regardless of their antigen specificity (i.e., a polyclonal activator). Negative controls consisted of cells treated with buffer only (PBS 1X).

After incubation for 24 hours at 37°C and 5% CO₂, plasma was separated from whole blood by centrifugation. Plasma was collected from each well and replicates (n=6 replicates per test) pooled. All plasma samples were frozen at -80°C until analysis. Single plasma aliquots (100 µL) were thawed and analyzed in duplicate using MSD IL-2 S-plex according to the manufacturer's instructions.

### Results:

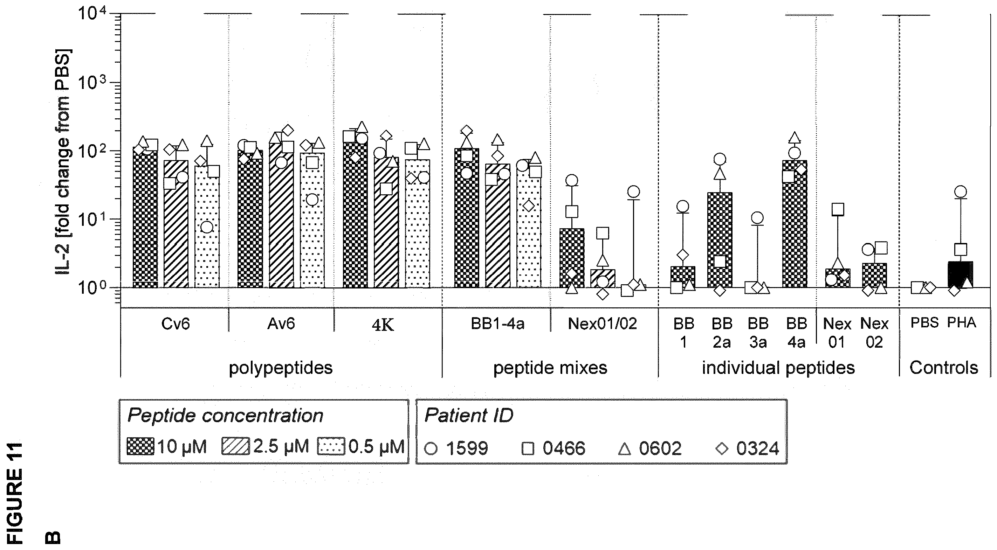

As seen in Figure 10 and Figure 11, all of the six HLA-DQ2+ and all of the four HLA-DQ8+ donors had a positive response to the three polypeptides of interest compared to buffer alone (PBS 1X control), in a dose-specific manner. Responses to the Cv6, Av6, and 4K polypeptides were very robust, with polypeptide 4K inducing the strongest responses overall (mean IL-2 titers in HLA-DQ2+ participants of 4858 fg/mL, 6664 fg/mL and 7199 fg/mL for Cv6, Av6 and 4K polypeptides at 10 µM, respectively; mean IL-2 titers in HLA-DQ8+ participants of 4528 fg/mL, 3695 fg/mL and 6300 fg/mL for Cv6, Av6 and 4K polypeptides at 10 µM, respectively).

At the highest concentration of 10 µM, the polypeptide constructs Av6 and 4K induced slightly stronger T cell recall responses than the mix containing Nex01 and Nex02 peptides (Nex01/02) in HLA-DQ2+ participants (mean IL-2 titers of 5684 fg/mL for Nex01/02 peptide mix) (Figure 10). However, the superiority of the polypeptide constructs was plainly evident in HLA-DQ8+ participants where Cv6, Av6 and 4K polypeptides induced mean IL-2 titers that were respectively 11-, 9- and 15-fold higher than that induced by the Nex01/02 peptide mix (mean IL-2 titers of 417 fg/mL for Nex01/02 peptide mix). IL-2 responses induced by individual building blocks (BB1, BB2a, BB3a, or BB4a) or individual Nexvax peptides (Nex01 or Nex02) perfectly reflected immunodominant epitopes relevant in HLA-DQ2+ or HLA-DQ8+ participants, thus fully validating the quality and the specificity of the assay. Indeed, individual building blocks specific for DQ2-glia epitopes (i.e., BB1 and BB3a) induced potent IL-2 responses in HLA-DQ2+ donors (Figure 10) but no or low responses in HLA-DQ8+ donors (Figure 11), while individual building blocks specific for DQ8-glia epitopes (i.e., BB2a and BB4a) induced potent IL-2 responses in HLA-DQ8+ donors (Figure 11) but no or low response in HLA-DQ2+ donors (Figure 10).

### Conclusion on the activation of fresh blood cells obtained from CeD patients:

These experiments demonstrate that the three polypeptide constructs (i.e., Cv6, Av6, and 4K) are efficiently captured and presented by APCs contained in human blood (i.e., circulating monocytes, B cells, and/or peripheral blood DCs), leading to a consistent activation of gluten-specific circulating CD4⁺ T cells in all ten of the CeD patients enrolled in the study, as evidenced by a significant increase in IL-2 production *in vitro.* Importantly, the level of T cell reactivation observed with the polypeptide constructs in HLA-DQ2+ donors was similar or even superior to that observed with the two mixes of peptides containing either the four individual building blocks BB1, BB2a, BB3a, and BB4a (BB1-4a) or two Nexvax2 peptides Nex01 and Nex02 (Nex01/02). But more strikingly, the IL-2 titers induced by the polypeptide constructs in HLA-DQ8+ donors were by far higher than those induced by the Nex01/02 peptide mix.

These results fully confirmed that the polypeptide constructs can be recognized and processed by the human immune system and can engage with gluten-specific CD4⁺ T-cells in CeD patients of both HLA-DQ2 and HLA-DQ8 haplotypes, thus validating their immunotherapeutic potential and their ability to address most CeD patients.

### EXAMPLE 6: Evaluation of selected gliadin polypeptides to reactivate T cell clones. Introduction to the T cell clone assay:

The overarching objective of this assay was to demonstrate that the polypeptide constructs are able to reactivate human CD4⁺ T cell clones propagated from CeD patients *in vitro.* For that purpose, five individual CD4⁺ T cell clones specific for a selection of key gliadin epitopes have been used. The experimental readout was the enumeration of IFN-γ producing T-cells (Th1 cells) by ELISpot, as previously described: Hardy MY, et al., J Autoimmun 2015; 56:56-65; Anderson et al., Clinical & Experimental Immunology, 2021, Issue 3, June 2021, Pages 321-334.

### Material and methods:

For this *in vitro* assay, irradiated peripheral blood mononuclear cells (PBMCs) isolated from the blood of healthy donors matching for HLA-DQ2.5 or HLA-DQ8 were used as a source of APCs. These irradiated PBMCs were exposed to three polypeptide constructs (SEQ ID NO:23 wherein X is C [Cv6, i.e., SEQ ID NO:49], SEQ ID NO:23 wherein X is A [Av6], and SEQ ID NO:46 [4K]) (concentrations ranging from 0.5 to 10 micromolar) before being co-cultured with individual human T cell clones specific for DQ2.5-glia-α1a, DQ2.5-glia-α2, DQ2.5-glia-co1, DQ2.5-glia-co2, or DQ8-glia-α1 epitopes.

As a positive control for antigen-specific T cell stimulation, the cognate gliadin-derived immunogenic peptide known to activate each CD4⁺ T cell clone was used, as described in the literature (Sollid et al., Immunogenetics, 2020 Feb;72(1-2):85-88; Tye-Din et al., Sci Transl Med, 2010, Vol. 2, Issue 41, 41-51). Negative controls consisted of cells treated with a cocktail of four irrelevant peptides of the following sequence: PFPLPQP (SEQ ID NO:87), PQYQPEQ (SEQ ID NO:88), PFEPQPL (SEQ ID NO:89), and PQSYPEQ (SEQ ID NO:90).

### Results:

As seen in Figures 12A and 12B, both 3072 1.26 (DQ2.5-glia-α1a specific) and 3007.28 (DQ2.5-glia-α2 specific) clones responded strongly to their cognate peptide in a dose-specific manner. Similarly, all three of the polypeptide constructs induced strong responses by T-cell clones with specificity for DQ2.5-glia-α1a or DQ2.5-glia-α2 that titrated with peptide dose. Responses between the different polypeptides were comparable and similar to what was observed with the cognate peptide (mean spot forming units of 159, 183, and 177 for Cv6, Av6, and 4K, respectively, at the dose of 10 µM in 3072 1.26 clone, compared to 182 for cognate peptide; mean stop forming units of 117, 122, and 109 for Cv6, Av6, and 4K, respectively, at the dose of 10 µM in 3007.28 clone, compared to 94 for cognate peptide). Responses to irrelevant peptides were very low, confirming the specificity of the T-cell clone response to cognate peptide (mean spot forming units of 11 and 0 in 3072 1.26 and 3007.28 clones, respectively, at the dose of 10 µM).

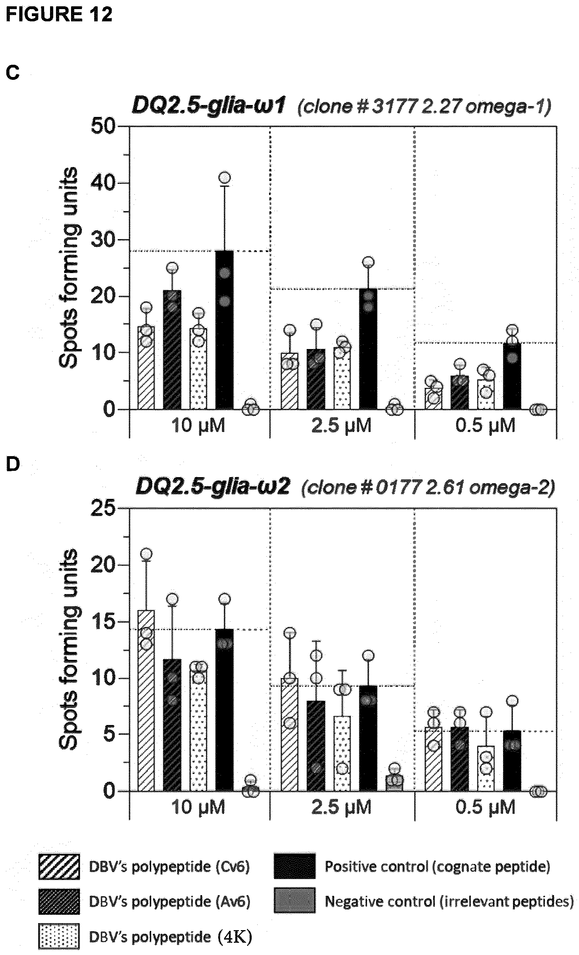

As seen in Figure 12C and 12D, IFN-γ levels induced in the ω-specific T cell clones were lower than those observed in α-specific clones. At 10 µM, Av6 induced a slightly higher IFN-γ response than Cv6 and 4K in the 3177 2.27 (DQ2.5-glia-ω1 specific) clone, whereas polypeptide Cv6 induced slightly stronger T cell responses than Av6 and 4K in the 0177 2.61 (DQ2.5-glia-ω2 specific) clone (mean spot forming units of 15, 21, and 14 for Cv6, Av6, and 4K, respectively, at the dose of 10 µM in 3177 2.27 clone, compared to 28 for cognate peptide; mean spot forming units of 16, 12, and 11 for Cv6, Av6, and 4K, respectively, at the dose of 10 µM in 0177 2.61 clone, compared to 14 for cognate peptide). All three polypeptides induced responses in a dose-specific manner. Responses to irrelevant peptides were very low, confirming the specificity of the T-cell clone response to cognate peptide (mean spot forming unit of 0 in both 3177 2.27 and 0177 2.61, at the dose of 10 µM).

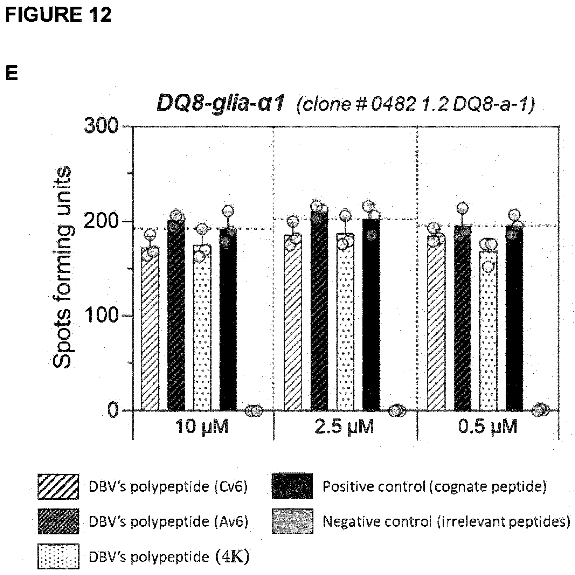

In line with the data obtained with the DQ2.5-glia-α-specific T cell clones, the three polypeptides induced very strong IFN-γ responses in the 0482 1.2 clone (DQ8-glia-α-specific) (Figure 12E). The magnitude of responses meant that a dose-response could not be observed for any antigen. The three polypeptides performed similarly to cognate peptide, with Av6 inducing slightly higher responses at all three doses tested compared to Cv6 and 4K (mean spot forming units of 173, 201, and 175 for Cv6, Av6, and 4K, respectively, at the dose of 10 µM in 0482 1.2 clone, compared to 193 for cognate peptide). Responses to irrelevant peptides were very low, confirming the specificity of the T cell responses to cognate antigen (mean spot forming units of 0 at the 10 µM dose).

### Conclusion on the reactivation of T cell clones:

Overall, these results showed that each of the three polypeptide constructs (i.e., Cv6, Av6 and 4K) have the capacity to efficiently reactivate T cell clones or lines specific to DQ2.5-glia-α1a/α2, DQ2.5-glia-ω1/ω2, and DQ8-glia-α1 in a standardized IFN-γ ELISpot assay. These data confirm the functionality of the key epitopes contained within the polypeptide constructs, as evidenced by a modulation of IFN-γ expression. These experiments demonstrate that the individual gliadin epitopes contained within the polypeptide constructs are efficiently presented by irradiated peripheral mononuclear cells and can consistently promote the activation of CD4⁺ T cell clones with matching specificity.

### References cited in the Background section.

1. Iversen R, Sollid LM. The Immunobiology and Pathogenesis of Celiac Disease. Annual Review of Pathology: Mechanisms of Disease (2023) 18: doi: 10.1146/annurev-pathmechdis-031521-032634
2. Singh P, Arora A, Strand TA, Leffler DA, Catassi C, Green PH, Kelly CP, Ahuja V, Makharia GK. Global Prevalence of Celiac Disease: Systematic Review and Meta-analysis. Clinical Gastroenterology and Hepatology (2018) 16:823-836.e2. doi: 10.1016/j.cgh.2017.06.037
3. Sahin Y. Celiac disease in children: A review of the literature. World J Clin Pediatr (2021) 10:53-71. doi: 10.5409/wjcp.v10.i4.53
4. Sharma N, Bhatia S, Chunduri V, Kaur S, Sharma S, Kapoor P, Kumari A, Garg M. Pathogenesis of Celiac Disease and Other Gluten Related Disorders in Wheat and Strategies for Mitigating Them. Front Nutr (2020) 7: doi: 10.3389/fnut.2020.00006
5. Caio G, Volta U, Sapone A, Leffler DA, de Giorgio R, Catassi C, Fasano A. Celiac disease: A comprehensive current review. BMC Med (2019) 17:1-20. doi: 10.1186/s12916-019-1380-z
6. Gianfrani C, Troncone R, Mugione P, Cosentini E, de Pascale M, Faruolo C, Senger S, Terrazzano G, Southwood S, Auricchio S, et al. Celiac Disease Association with CD8 + T Cell Responses: Identification of a Novel Gliadin-Derived HLA-A2-Restricted Epitope. The Journal of Immunology (2003) 170:2719-2726. doi: 10.4049/jimmunol. 170.5.2719
7. Sollid LM, Qiao S-W, Anderson RP, Gianfrani C, Koning F. Nomenclature and listing of celiac disease relevant gluten T-cell epitopes restricted by HLA-DQ molecules. Immunogenetics (2012) 64:455-460. doi: 10.1007/s00251-012-0599-z
8. Ciccocioppo R, di Sabatino A, Corazza GR. The immune recognition of gluten in coeliac disease. Clin Exp Immunol (2005) 140:408-416. doi: 10.1111/j.1365-2249.2005.02783.x
9. Sollid LM, Tye-Din JA, Qiao S-W, Anderson RP, Gianfrani C, Koning F. Update 2020: nomenclature and listing of celiac disease-relevant gluten epitopes recognized by CD4+ T cells. Immunogenetics (2020) 72:85-88. doi: 10.1007/s00251-019-01141-w
10. Hellman R. Gluten Free Diets - A Challenge for the Practicing Physician. Mo Med (2020) 117:119-123.
11. Daveson AJM, Ee HC, Andrews JM, King T, Goldstein KE, Dzuris JL, MacDougall JA, Williams LJ, Treohan A, Cooreman MP, et al. Epitope-Specific Immunotherapy Targeting CD4-Positive T Cells in Celiac Disease: Safety, Pharmacokinetics, and Effects on Intestinal Histology and Plasma Cytokines with Escalating Dose Regimens of Nexvax2 in a Randomized, Double-Blind, Placebo-Controlled Phase 1 Study. EBioMedicine (2017) 26:78-90. doi: 10.1016/j.ebiom.2017.11.018
12. Goel G, King T, Daveson AJ, Andrews JM, Krishnarajah J, Krause R, Brown GJE, Fogel R, Barish CF, Epstein R, et al. Epitope-specific immunotherapy targeting CD4-positive T cells in coeliac disease: two randomised, double-blind, placebo-controlled phase 1 studies. Lancet Gastroenterol Hepatol (2017) 2:479-493. doi: 10.1016/52468-1253(17)30110-3.

### List of sequences

| SEQ ID | **SEQUENCE** |
|---|---|
| NO:1 | PFPQPZLPY |
| NO:2 | PYPQPZLPY |
| NO:3 | PQPZLPYPQ |
| NO**:**4 | ZGSFQPSQZ |
| NO**:**5 | PQQSFPZQZ |
| NO:6 | ZQPZQPFPZ |
| NO:7 | QQPFPZQPQ |
| NO:8 | PFPQPZQPF |
| NO:9 | PQPZQPFPW |
| NO:10 | PQQSFPEQE |
| NO:11 | QPEQPFP |
| NO: 12 | EQPEQPFPE |
| NO: 13 | ZGSVQPQZL |
| NO: 14 | PFPQP**Z**LPYPQP**Z**LPYPQP**Z**LPYPQ |
| NO: 15 | QLQPFPQP**Z**LPYPQPZLPYPQPZLPYPQPQP |
| NO:16 | PQPZQPZQPFPZQPQ |
| NO: 17 | QQFPQPZQPZQPFPZQPQQQF |
| NO: 18 | PSG**Z**GSFQPSQZNPQ |
| NO:19 | QQYPSG**Z**GSFQPSQ**Z**NPQAQG |
| NO:20 | PQQ**PFPQPZQPFPW**QPQ |
| NO:21 | PQQPQQPFPQPZQPFPWQPQQPF |
| NO:22 | QLQPFPQPZLPYPQPZLPYPQPZLPYPQPQPGPRRKKPQPZQPZQPFPZQPQGXRRKKPQQPFP QPZQPFPWQPQGPKKPSGZGSFQPSQZNPQ |
| NO:23 | QLQPFPQPELPYPQPELPYPQPELPYPQPQPGPRRKKPQPEQPEQPFPEQPQGXRRKKPQQPFP QPEQPFPWQPQGPKKPSGEGSFQPSQENPQ |
| NO:24 | QLQPFPQPELPYPQPELPYPQPELPYPQPQPGPKKRKPQPEQPEQPFPEQPQGPKKRKPQQPFP QPEQPFPWQPQGPKKPSGEGSFQPSQENPQ |
| NO:25 | LQLQPFPQPELPYPQPELPYPQPELPYPQPQPGPKKRKPQPEQPEQPFPEQPQGPKKRKPQQPF PQPEQPFPWQPQGPKKPSGEGSFQPSQENPQ |
| NO:26 | LQLQPFPQPELPYPQPELPYPQPELPYPQPQPGPKKPQPEQPEQPFPEQPQGPKKPQQPFPQPE QPFPWQPQGPKKPSGEGSFQPSQENPQ |
| NO:27 | LQLQPFPQPELPYPQPELPYPQPELPYPQPQPKGKKPQPEQPEQPFPEQPQKGKKPQQPFPQPE QPFPWQPQGPKKPSGEGSFQPSQENPQ |
| NO:28 | PFPQPZQPFPW |
| NO:29 | EGSFQPSQE |
| NO: 30 | PFPQPEQPFPW |
| NO:31 | LQLQPFPQPZLPYPQP**Z**LPYPQPZLPYPQPQPF |
| NO:32 | KKKK |
| NO: 33 | GPRRKK |
| NO:34 | GPKK |
| NO: 35 | GARRKK |
| NO: 36 | GCRRKK |
| NO:37 | RVRR |
| NO: 38 | GAGA |
| NO: 39 | AGAG |
| NO: 40 | KGKG |
| NO:41 | GPGPG |
| NO: 42 | PQPEQPEQPFPEQPQKGKKLQLQPFPQPELPYPQPELPYPQPELPYPQPQPFKGKKPQQPFPQP EQPFPWQPQKGKKPSGEGSFQPSQENPQ |
| NO: 43 | PQQPFPQPEQPFPWQPQKGKKPQPEQPEQPFPEQPQKGKKLQLQPFPQPELPYPQPELPYPQPE LPYPQPQPFKGKKPSGEGSFQPSQENPQ |
| NO: 44 | QLQPFPQPZLPYPQPZLPYPQPZLPYPQPQPKKKKPQPZQPZQPFPZQPQKKKKPQQPFPQPZQ PFPWQPQKKKKPSGZGSFQPSQZNPQ |
| NO: 45 | KGKK |
| NO: 46 | QLQPFPQPELPYPQPELPYPQPELPYPQPQPKKKKPQPEQPEQPFPEQPQKKKKPQQPFPQPEQ PFPWQPQKKKKPSGEGSFQPSQENPQ |
| NO: 47 | LQPFPQPELPYPQPQ |
| NO: 48 | QPFPQPEQPFPWQP |
| NO: 49 | QLQPFPQPELPYPQPELPYPQPELPYPQPQPGPRRKKPQPEQPEQPFPEQPQGCRRKKPQQPFP QPEQPFPWQPQGPKKPSGEGSFQPSQENPQ |
| NO:50 | QLQPFPQPELPYPQPELPYPQPELPYPQPQPGPRRKKPQPEQPEQPFPEQPQGARRKKPQQPFP QPEQPFPWQPQGPKKPSGEGSFQPSQENPQ |
| NO:51 | PFPQPELPY |
| NO:52 | PFPQPQLPY |
| NO:53 | PYPQPELPY |
| NO: 54 | PYPQPQLPY |
| NO: 55 | PQPELPYPQ |
| NO: 56 | PQPQLPYPQ |
| NO:57 | EGSFQPSQE |
| NO:58 | QGSFQPSQQ |
| NO: 59 | EGSFQPSQQ |
| NO: 60 | QGSFQPSQE |
| NO: 61 | PQQSFPEQE |
| NO:62 | PQQSFPQQQ |
| NO: 63 | PQQSFPEQQ |
| NO: 64 | PQQSFPQQE |
| NO: 65 | EQPEQPFPE |
| NO: 66 | QQPQQPFPQ |
| NO: 67 | EQPEQPFPQ |
| NO: 68 | EQPQQPFPE |
| NO: 69 | QQPQQPFPE |
| NO: 70 | QQPEQPFPQ |
| NO:71 | EQPQQPFPQ |
| NO: 72 | QQPEQPFPE |
| No:73 | QQPFPEQPQ |
| No:74 | QQPFPQQPQ |
| NO: 75 | PFPQPEQPF |
| NO: 76 | PFPQPQQPF |
| NO: 77 | PQPEQPFPW |
| NO: 78 | PQPQQPFPW |
| NO: 79 | EGSVQPQEL |
| NO:80 | QGSVQPQQL |
| NO:81 | EGSVQPQQL |
| NO: 82 | QGSVQPQEL |
| NO: 83 | QLQPFPQPELPYPQPELPYPQPELPYPQPQP |
| NO: 84 | PQPEQPEQPFPEQPQ |
| NO: 85 | PQQPFPQPEQPFPWQPQ |
| NO: 86 | PSGEGSFQPSQENPQ |
| NO: 87 | PFPLPQP |
| NO:88 | PQYQPEQ |
| NO: 89 | PFEPQPL |
| NO:90 | PQSYPEQ |

## Claims

1. An immunogenic polypeptide construct of formula (Ic):
[BB1]-[CCw]-[BB2a]-[CCx]-[BB3a]-[CCy]-[BB4a] (Ic)
wherein:
- BB1 is a polypeptide of SEQ ID NO:15, BB2a is a polypeptide of SEQ ID NO:16, BB3a is a polypeptide of SEQ ID NO:20, and BB4a is a polypeptide of SEQ ID NO:18, wherein all Z residues are E residues in SEQ ID NOS:15, 16, 20, and 18, and
- each CCw, CCx, and CCy are independently selected from the group consisting of KKKK (SEQ ID NO:32), KK, GPRRKK (SEQ ID NO:33), GPKK (SEQ ID NO:34), GARRKK (SEQ ID NO:35), AYY, RR, and GCRRKK (SEQ ID NO:36)

2. The immunogenic polypeptide construct according to claim 1, wherein each CCw, CCx, and CCy are independently selected from the group consisting of GPRRKK, GPKK, GARRKK, and GCRRKK.

3. The immunogenic polypeptide construct according to claim 1 or 2, which consists of a polypeptide selected from SEQ ID NO: 46, 49, and 50.

4. The immunogenic polypeptide construct according to claim 3, which consists of a polypeptide of SEQ ID NO: 50.

5. The immunogenic polypeptide construct according to claim 3, which consists of a polypeptide of SEQ ID NO: 49.

6. The immunogenic polypeptide construct according to claim 3, which consists of a polypeptide of SEQ ID NO: 46.

7. The immunogenic polypeptide construct according to any one of claims 1 to 6, wherein the C-terminal end of the polypeptide construct is amidated and/or the N-terminal end of the polypeptide construct is acetylated.

8. A pharmaceutical composition comprising an immunogenic polypeptide construct as defined in any one of claims 1 to 7, and at least one pharmaceutically acceptable excipient.

9. A skin patch comprising an immunogenic polypeptide construct as defined in any one of claims 1 to 7.

10. The skin patch according to claim 9, wherein the skin patch comprises a backing having a periphery adapted to create a closed chamber when applied on a subject's skin, wherein the immunogenic polypeptide construct is present in dry form on the backing of the patch, and optionally wherein the closed chamber is a hermetically closed chamber.

11. The immunogenic polypeptide construct according to any one of claims 1 to 7 for use in the treatment or the prevention of celiac disease.

12. The immunogenic polypeptide construct according to any one of claims 1 to 7 for use in increasing tolerance to gliadin in a subject sensitive to gliadin.

13. The immunogenic polypeptide construct for use according to claim 11 or 12, wherein the immunogenic polypeptide construct is administered by epicutaneous route.

14. The immunogenic polypeptide construct for use according to any one of claims 11 to 13, wherein the immunogenic polypeptide construct is administered by means of a skin patch.

## Patentansprüche

1. Immunogenes Polypeptidkonstrukt der Formel (Ic):
[BB1]-[CCw]-[BB2a]-[CCx]-[BB3a]-[CCy]-[BB4a] (Ic)
wobei:
- BB1 ein Polypeptid der Sequenz-ID Nr. 15, BB2a ein Polypeptid der Sequenz-ID Nr. 16, BB3a ein Polypeptid der Sequenz-ID Nr. 20 und BB4a ein Polypeptid der Sequenz-ID Nr. 18 ist, wobei alle Z-Reste in den Sequenz-IDs 15, 16, 20 und 18 E-Reste sind, und
- CCw, CCx und CCy jeweils unabhängig aus der Gruppe bestehend aus KKKK (Sequenz-ID Nr. 32), KK, GPRRKK (Sequenz-ID Nr. 33), GPKK (Sequenz-ID Nr. 34), GARRKK (Sequenz-ID Nr. 35), AYY, RR und GCRRKK ausgewählt werden. (SEQ ID NO:36).

2. Das immunogene Polypeptidkonstrukt nach Anspruch 1, wobei CCw, CCx und CCy jeweils unabhängig voneinander aus der Gruppe bestehend aus GPRRKK, GPKK, GARRKK und GCRRKK ausgewählt sind.

3. Das immunogene Polypeptidkonstrukt nach Anspruch 1 oder 2, bestehend aus einem Polypeptid, ausgewählt aus den Sequenz-ID-Nr. 46, 49 und 50.

4. Das immunogene Polypeptidkonstrukt nach Anspruch 3, bestehend aus einem Polypeptid mit der Sequenz-ID-Nr. 50.

5. Das immunogene Polypeptidkonstrukt nach Anspruch 3, bestehend aus einem Polypeptid mit der Sequenz-ID Nr. 49.

6. Das immunogene Polypeptidkonstrukt nach Anspruch 3, bestehend aus einem Polypeptid mit der Sequenz-ID Nr. 46.

7. Das immunogene Polypeptidkonstrukt nach einem der Ansprüche 1 bis 6, wobei das C-terminale Ende des Polypeptidkonstrukts amidiert und/oder das N-terminale Ende des Polypeptidkonstrukts acetyliert ist.

8. Pharmazeutische Zusammensetzung, umfassend ein immunogenes Polypeptidkonstrukt, wie in einem der Ansprüche 1 bis 7 definiert, und mindestens einen pharmazeutisch verträglichen Hilfsstoff.

9. Hautpflaster, umfassend ein immunogenes Polypeptidkonstrukt, wie in einem der Ansprüche 1 bis 7 definiert.

10. Das Hautpflaster nach Anspruch 9, wobei das Hautpflaster eine Trägerschicht mit einem Rand aufweist, der angepasst ist, um beim Aufbringen auf die Haut eines Patienten eine geschlossene Kammer zu bilden, wobei das immunogene Polypeptidkonstrukt in trockener Form auf der Trägerschicht des Pflasters vorliegt, und die geschlossene Kammer gegebenenfalls eine hermetisch geschlossene Kammer ist.

11. Das immunogene Polypeptidkonstrukt nach einem der Ansprüche 1 bis 7 zur Verwendung bei der Behandlung oder Prävention von Zöliakie.

12. Das immunogene Polypeptidkonstrukt nach einem der Ansprüche 1 bis 7 zur Verwendung bei der Steigerung der Gliadin-Toleranz bei einer Person, die empfindlich auf Gliadin reagiert.

13. Das immunogene Polypeptidkonstrukt zur Verwendung nach Anspruch 11 oder 12, wobei das immunogene Polypeptidkonstrukt epikutan verabreicht wird.

14. Das immunogene Polypeptidkonstrukt zur Verwendung nach einem der Ansprüche 11 bis 13, wobei das immunogene Polypeptidkonstrukt mittels eines Hautpflasters verabreicht wird.

## Revendications

1. Construction polypeptidique immunogénique de formule (Ic) :
[BB1]-[CCw]-[BB2a]-[CCx]-[BB3a]-[CCy]-[BB4a] (Ic)
dans laquelle :
- BB1 est un polypeptide de SEQ ID NO : 15, BB2a est un polypeptide de SEQ ID NO : 16, BB3a est un polypeptide de SEQ ID NO : 20, et BB4a est un polypeptide de SEQ ID NO : 18, dans lesquels tous les résidus Z sont des résidus E dans SEQ ID NO : 15, 16, 20 et 18, et
- chaque CCw, CCx et CCy est indépendamment choisi dans le groupe constitué par KKKK (SEQ ID NO : 32), KK, GPRRKK (SEQ ID NO : 33), GPKK (SEQ ID NO : 34), GARRKK (SEQ ID NO : 35), AYY, RR, et GCRRKK (SEQ ID NO : 36).

2. Construction polypeptidique immunogénique selon la revendication 1, dans laquelle chaque CCw, CCx et CCy est indépendamment choisi dans le groupe constitué par GPRRKK, GPKK, GARRKK, et GCRRKK.

3. Construction polypeptidique immunogénique selon la revendication 1 ou 2, qui consiste en un polypeptide choisi parmi les SEQ ID NO : 46, 49 et 50.

4. Construction polypeptidique immunogénique selon la revendication 3, qui consiste en un polypeptide de la SEQ ID NO : 50.

5. Construction polypeptidique immunogénique selon la revendication 3, qui consiste en un polypeptide de la SEQ ID NO : 49.

6. Construction polypeptidique immunogénique selon la revendication 3, qui consiste en un polypeptide de la SEQ ID NO : 46.

7. Construction polypeptidique immunogénique selon l'une quelconque des revendications 1 à 6, dans laquelle l'extrémité C-terminale de la construction polypeptidique est amidée et/ou l'extrémité N-terminale de la construction polypeptidique est acétylée.

8. Composition pharmaceutique comprenant une construction polypeptidique immunogénique telle que définie dans l'une quelconque des revendications 1 à 7, et au moins un excipient pharmaceutiquement acceptable.

9. Patch cutané comprenant une construction polypeptidique immunogénique telle que définie dans l'une quelconque des revendications 1 à 7.

10. Patch cutané selon la revendication 9, lequel patch cutané comprend un envers ayant une périphérie adaptée pour créer une chambre fermée lors de son application sur la peau d'un sujet, dans lequel la construction polypeptidique immunogénique est présente sous forme sèche sur l'envers du patch, et éventuellement dans lequel la chambre fermée est une chambre hermétiquement fermée.

11. Construction polypeptidique immunogénique selon l'une quelconque des revendications 1 à 7, pour une utilisation dans le traitement ou la prévention de la maladie cœliaque.

12. Construction polypeptidique immunogénique selon l'une quelconque des revendications 1 à 7, pour une utilisation dans l'augmentation de la tolérance à la gliadine chez un sujet sensible à la gliadine.

13. Construction polypeptidique immunogénique pour une utilisation selon la revendication 11 ou 12, laquelle construction polypeptidique immunogénique est administrée par voie épicutanée.

14. Construction polypeptidique immunogénique pour une utilisation selon l'une quelconque des revendications 11 à 13, laquelle construction polypeptidique immunogénique est administrée au moyen d'un patch cutané.
